# EUROPEAN PATENT APPLICATION

(11) **EP 3 029 460 A1**
(43) Date of publication of application: **08.06.2016**
(21) Application number: 16152292.5
(22) Date of filing: 24.09.2010
(51) Int. Cl.: G01N 33/50

(54) **O-CADHERIN AS A BIOMARKER FOR CIRCULATING TUMOR CELLS**

(30) Priority: 27.01.2010 US 298845 P; 26.02.2010 US 308780 P; 01.03.2010 US 309131 P
(62) Divisional of application: 10844915.8
(71) Applicant: Duke University, Durham, NC 27705 (US)
(72) Inventor: GARCIA-BLANCO, Mariano A., Hillsborough, NC 27278 (US); ARMSTRONG, Andrew J., Chapel Hill, NC 27514 (US); GEORGE, Daniel J., Chapel Hill, NC 27514 (US); OLTEAN, Sebastian, Exeter EX4 4AY (GB)
(74) Representative: Grünecker Patent- und Rechtsanwälte PartG mbB

(57) **Abstract**

Provided are methods for detecting circulating tumor cells (CTCs) in a subject. The methods may include detecting the expression of at least one epithelial mesenchymal transition (EMT) biomarker. Further provided are kits for detecting CTCs. The kits may include antibodies to at least one EMT biomarker. Further provided are methods of predicting the responsiveness of a subject to a cancer drug, methods of targeting delivery of a cancer drug in a subject, methods of providing a cancer prognosis to a subject, and methods for following the progress of cancer in a subject. All of these methods and kits are based on the use of O-Cadherin as a biomarker for CTCs.

## Description

### CROSS-REFERENCE TO RELATED APPLICATIONS

This application claims the benefit of priority to United States Provisional Patent Application No. 61/298,845 filed January 27, 2010; United States Provisional Patent Application No. 61/308,780 filed February 26, 2010; and United States Provisional Patent Application No. 61/309,131 filed March 1, 2010, which are all incorporated herein by reference in their entireties.

### STATEMENT REGARDING FEDERALLY SPONSORED RESEARCH

This invention was made with government support under federal grant number 5R33CA097502 from the NIH (NCI), and federal grant number 5K12CA10063904 from the NIH (NCI). The U.S. Government has certain rights to this invention.

### SEQUENCE LISTING

The sequence listing is filed with the application in electronic format only and is incorporated by reference herein. The sequence listing text file "B2442027.txt" was created on September 24, 2010 and is 131,287 bytes in size.

### FIELD

The disclosure relates to methods for the detection and prognosis of cancer. Moreover, the disclosure provides methods for detecting circulating tumor cells (CTCs) that include the identification, detection, and optional enumeration of one or more biomarkers associated with CTCs that can be used in methods relating to a prognosis, diagnosis, or the treatment of cancer in a subject.

### BACKGROUND

Most metazoan cells can be classified as either epithelial or mesenchymal based on morphology, behavior and molecular signatures. Epithelial cells are generally polar in the apico-basal direction, adherent to adjacent cells in the plane perpendicular to the polarity, and non-motile in the polar direction. Mesenchymal cells, in contrast, lack polarity, do not form tight interactions with neighboring cells, and are motile. In adult animals epithelial and mesenchymal cells remain stably in one state or the other; that is, an epithelial cell does not change its properties and become mesenchymal. During development, however, epithelial cells of the early embryo give rise to all three embryonal layers (endoderm, mesoderm and ectoderm), which include mesenchymal cells (Hay, E.D., et al. Am. J. Kidney Dis. 1995, 26, 678-690). Therefore, these early embryonal cells have the ability to transition between epithelial and mesenchymal states, a property sometimes referred to as epithelial plasticity. Embryos have been shown to undergo epithelial-mesenchymal transitions (EMTs) as well as mesenchymal-epithelial transitions (METs) (Acloque, H., et al. J. Clin. Invest. 2009, 119, 1438-1449).

Circulating tumor cells (CTCs) are cells that have detached from a primary tumor and circulate in the bloodstream. CTCs may constitute seeds for subsequent growth of additional tumors (metastasis) in different tissues. Thus, detection of CTCs can provide for diagnosis and/or prognosis for overall survival and therapeutic implications in subjects with cancers such as metastatic prostate and breast cancer. The number of CTCs in any patient sample (e.g., a blood sample) can be very small, which can make detection difficult. Current methods for detecting CTCs are based on the detection of epithelial cell adhesion molecule (EpCAM) expression, which is a biomarker associated with epithelial cells. Such methods can under-detect CTCs under circumstances where cells undergo a decrease or loss of EpCAM expression, such as biologic processes including EMT. Because of the important role CTCs can play in the diagnosis, monitoring, and prognosis of disease in patients having cancer, any shortcoming in the detection technology needs to be addressed by the art.

Accordingly, there is a need for methods and systems for detecting CTCs that do not rely on existing capture technologies, and methods for correlating CTC detection to diagnosis, monitoring, and prognosis of disease in cancer patients.

### SUMMARY

In an aspect, the disclosure provides a method for detecting a circulating tumor cell (CTC) in a biological sample, the method comprising detecting at least one epithelial mesenchymal transition (EMT) biomarker in the biological sample.

In an aspect, the disclosure provides a kit for detecting a circulating tumor cell (CTC) in a biological sample, the kit comprising an antibody to at least one EMT biomarker and instructions for use.

In an aspect, the disclosure provides a method of predicting responsiveness of a subject having cancer to a course of cancer treatment, the method comprising: determining the level or presence of expression of at least one EMT biomarker to obtain an EMT biomarker profile and/or optionally a gene expression pattern for a CTC; and predicting the responsiveness of the subject to the cancer drug based on the EMT biomarker profile and/or optional gene expression pattern. In some embodiments the method includes: determining the level or presence of expression of at least one EMT biomarker in a sample from the subject to obtain a biomarker profile and optionally a gene expression pattern in a CTC for the subject; identifying the type of cancer from the biomarker profile and/or optional gene expression pattern, and optionally characterizing the stage of the cancer; and predicting responsiveness of the subject to the cancer drug based on any one of the biomarker pattern, the optional gene expression pattern, the type of cancer, or the stage of the cancer. Embodiments of this aspect can include detecting a number of cells captured and enumerated from a blood sample using at least one EMT biomarker applied to a sample from the subject. These cells that express the EMT biomarker are thereby captured using the EMT biomarker and could then be used to obtain a gene expression pattern in CTCs for the subject; to predict responsiveness of the subject to the cancer drug based on the obtained gene expression pattern, and for the detection of other biomarkers in these CTCs to assist in guiding therapy of that subject. These cells could also be used to measure the level of the specified EMT biomarker or other EMT biomarkers.

In an aspect, the disclosure provides a method of assessing the number of CTCs using both the traditional EpCAM based capture methodology and an EMT-marker based capture methodology. This EMT-based capture may replace or complement existing CTC capture technologies. The further capture, enumeration, and characterization of these CTCs using EMT antigen capture may further targeting delivery of a cancer drug in a subject having cancer comprising administering to the subject a cancer drug linked to an antibody specific for at least one EMT biomarker or specific drugs based on a gene expression profile or presence of this EMT biomarker.

In an aspect, the disclosure provides a method of estimating the prognosis of a subject with cancer as well as permitting a further characterization of CTCs that may predict for therapeutic responsiveness, the method comprising: determining the level of or presence of expression of at least one EMT biomarker in a sample from the subject to determine the number of CTCs in the subject and to obtain a gene expression pattern for the subject; and providing a prognosis to the subject based on the gene expression or biomarker profile pattern obtained.

In an aspect, the disclosure provides a method for monitoring progression of cancer in a subject undergoing therapeutic treatment, the method comprising detecting the level of expression or presence of expression of at least one EMT biomarker and the quantification of CTCs captured using this method in blood samples taken from the subject at a first and a second time; and comparing the first and second levels of expression; wherein a detected difference in the level of expression of the at least one EMT biomarker in the first and second samples over time indicates a change in the progression status of the cancer.

In an aspect, the disclosure provides a method for detecting cancer in a subject, the method comprising determining the presence of CTCs that express at least one EMT biomarker in a sample from the subject as compared to a normal or control sample, wherein an increased level of at least one EMT biomarker indicates presence of cancer progression or metastatic spread in the subject.

In an aspect, the disclosure provides a method of treating cancer in a subject comprising administering to the subject a cancer drug linked to an antibody that specifically binds at least one EMT biomarker.

Other aspects and embodiments of the disclosure will become apparent by consideration of the detailed description and accompanying drawings.

### BRIEF DESCRIPTION OF THE DRAWINGS

Figure 1. (**A**) depicts a schematic representation of the IIIb and IIIc alternatively spliced isoforms of FGFR2. **(B**) is a schematic of the pRIIIcl² minigene and the fluorescence read-out. (**C**) is an RT-PCR analysis of the reporter (upper panel) and endogenous FGFR2 (lower panel). (**D**) are epifluorescence and phase-contrast pictures of clones AT3-M and AT3-T.
**Figure 2****.** (**A**) depicts examples of clusters of DsRED positive cells formed by AT3-M cells upon treatment with conditioned media from clone AT3-T. (**B**) depicts flow cytometry analysis of the same experiment.
**Figure 3****.** (**A**) depicts growth curves for clones AT3-T and AT3-M. (**B**) is graph of growth of AT3-M, AT3-T, and DT cells in soft agar. (**C**) depicts a sacrifice curve for rats injected with AT3-M or AT3-T cells. (**D**) depicts a comparison of tumor volumes resulting from AT3-T and AT3-M injection.
**Figure 4****.** (**A**) a representative example of cells that express both RFP and GFP at the periphery of an AT3-M tumor stably transfected with Gint and pRIIIcl² reporters. (**B**) a representative example of a section from an AT3-T tumor stably transfected with GFP and pRIIIcl² reporters.
**Figure 5** a representative example of cells that express both RFP and GFP at the periphery of an AT3-M tumor stably transfected with Gint and pRIIIcl² reporters.
**Figure 6****.** (**A**) representative pictures of cells for the scratch-wound assay. (**B**) a quantification of migration. (**C**) an invasion assay using Matrigel coated membranes. (**D**) a quantification of invasion assay results.

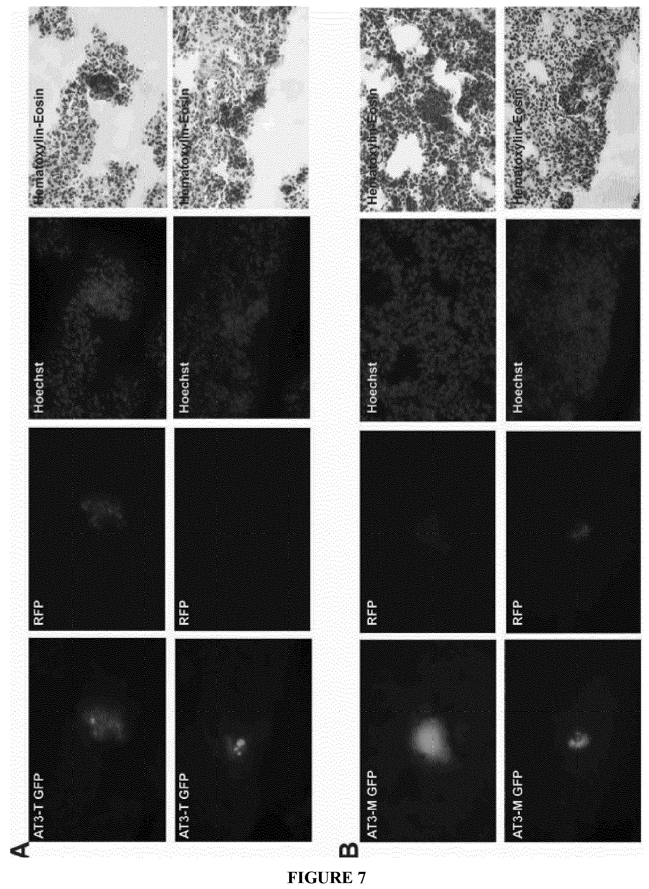

**Figure 7** are metastatic foci in lungs from animals with tumors from either AT3-T or AT3-M clones (stably transfected with GFP and pRIIIcI² reporters). (**A**) (upper panel) is an example of a section exhibiting the pattern for clone AT3-T (i.e. GFP+, DsRED+) in a metastatic focus and (lower panel) an example of a section exhibiting a plastic pattern for clone AT3-T (i.e. GFP+, DsRED-) in a metastatic focus. (**B**) (upper panel) is an example of a section exhibiting the pattern for clone AT3-M (i.e. GFP+, DsRED-) in a metastatic focus and (lower panel) an example of a section exhibiting a plastic pattern for clone AT3-M (i.e. GFP+, DsRED+) in a metastatic focus.
**Figure 8A** a membrane with serial two-fold dilutions of whole cell Iysates cut in half and immunoblotted for CD 133 (upper panel) or β-actin (lower panel). (**B**) a membrane with serial twofold dilutions of whole cell lysates cut in half and immunoblotted for CD44 (upper panel) or β-actin (lower panel).
**Figure 9** depicts a model comparing stem cell-like character and epithelial mesenchymal phenotype.

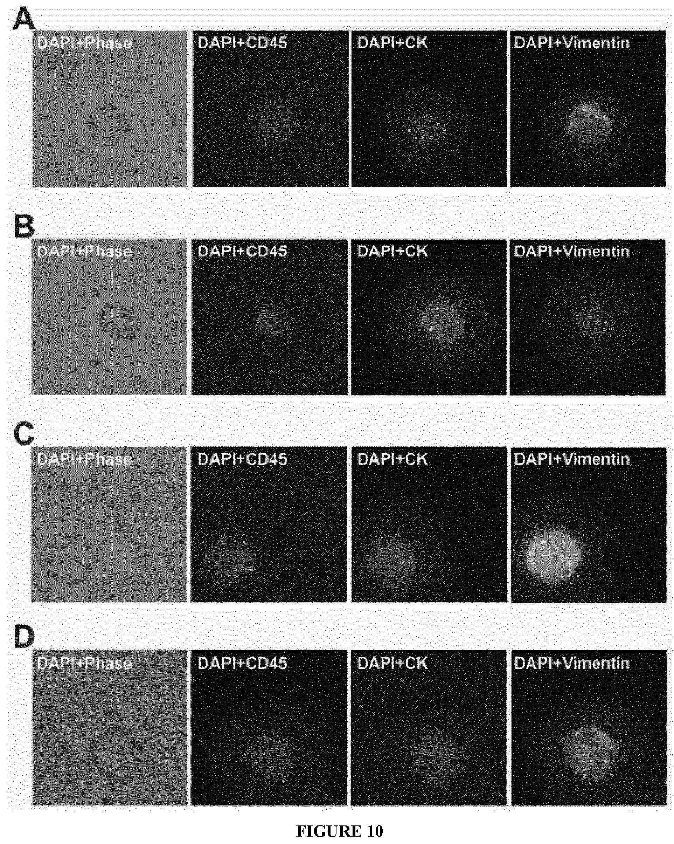

**Figure 10** depicts CTCs from patients with prostate adenocarcinoma. (**A**) illustrates an example of a leukocyte from a human peripheral blood mononuclear cell (PMBC) sample: CD45 (+), CK (-), and vimentin (+). (**B**) illustrates an example of a CD45 (-), CK (+), and vimentin (-) cell from a patient with metastatic breast cancer. (**C**) illustrates an example of a CD45 (-), CK (+), vimentin (+) from a patient with metastatic breast cancer (mBC). (**D**) illustrates an example of a CD45 (-), CK (+), vimentin (+) from a patient with metastatic progressive castrate-resistant prostate cancer (mCRPC).
**Figure 11** depicts immunofluorescent images of CTCs from patients with mCRPC and mBC.
**Figure 12** depicts immunofluorescent images of CTCs from patients with mCRPC and mBC.
**Figure 13** depicts immunofluorescent images of CTCs from patients with mCRPC and mBC.
**Figure 14** depicts immunofluorescent images of CTCs from patients with mCRPC and mBC.
**Figure 15** depicts immunofluorescent images of CTCs from patients with mCRPC and mBC.
**Figure 16** depicts immunofluorescent images of CTCs from patients with mCRPC and mBC.

### DETAILED DESCRIPTION

Before any embodiments are described in detail, it is to be understood that the claims are not limited to the details of construction and the arrangement of components set forth in the following description or illustrated in the included drawings.

In a general sense, the disclosure provides biomarkers that have been identified to be associated with circulating tumor cells (CTCs). As described herein, one or more biomarkers of epithelial mesenchymal transition (EMT) are detectable on CTCs of patients afflicted with common epithelial malignancies. These transitional cells often display stem cell-like characteristics (stemness) and/or plasticity. Further, the disclosure provides description that metastatic propensity and epithelial phenotypic changes correlate with alternative splicing of the FGFR2 gene. The disclosure also provides that, as illustrated in the non-limiting Examples, transitional cells are found in cancer patients where many CTCs co-expressed biomarkers associated with epithelial and mesenchymal cells.

Thus, as described below EMT biomarker expression can be used to detect and quantify CTCs in a biological sample. Accordingly, methods comprising detection of EMT biomarker expression, or detection of CTCs, or a combination thereof, can be used to assess cancer prognosis, tumor invasiveness, risk of metastasis, or to stage tumors. As one of skill in the art will appreciate, any suitable method for evaluating EMT biomarker expression can be used to evaluate EMT biomarker expression according to the methods described herein including, but not limited to, detection with antibodies, real time RT-PCR, Northern analysis, Western analysis, and flow cytometry.

As described herein the ability for a cell to transition easily between epithelial-like and mesenchymal-like states (phenotypic plasticity) is a relevant determinant of malignant fitness more so than the properties of the end states. While these epithelial transitions are phenotypic, the propensity to transition (plasticity) among carcinoma cells may be determined by genotype. The majority of plastic cells may inhabit transitional intermediate states with properties of both epithelium and mesenchyme, and that these transitional cells may be particularly malignant. Such cells may be detected in: (1) tumors where the cancer cells have mixed histology, which indeed have been observed and have been classified as highly aggressive (e.g., clonal sarcomatous carcinomas of epithelial origin, which exhibit an extremely aggressive behavior, such as sarcomatoid renal cell carcinoma and carcinosarcoma of the prostate); and (2) cancer cells co-expressing epithelial and mesenchymal markers, as described herein.

The disclosure, as illustrated by the non-limiting embodiments in the Examples, provides for identification of cells that possess an intermediate phenotype - expressing epithelial and mesenchymal isoforms of FGFR2, having epithelial-like morphology and gene expression patterns, while also displaying mesenchymal cell-like migration, tumor formation, and metastases. In embodiments, these cells are identified in patients with advanced cancer, metastatic adenocarcinoma, and metastatic breast and prostate carcinomas. In some embodiments, the cells comprise CTCs. In some embodiments the CTCs co-expresses biomarkers including, for example, EpCAM, cytokeratin, and vimentin, which identify cells as both epithelial- and mesenchymal-like. In some embodiments, these CTCs in intermediate phenotypic states are identified by detecting EMT biomarkers and provide a diagnosis and/or prognosis of the state and/or degree of malignancy of a cancer.

In an aspect the disclosure provides a method for detecting CTCs in a biological sample, the method comprising detecting at least one epithelial mesenchymal transition (EMT) biomarker in the biological sample. In some embodiments such as illustrated in the Examples, biomarkers of EMT are present on the CTCs of patients with common epithelial malignancies. In some embodiments methods that include detection and identification of alternative splice variants of the FGFR2 gene are used to correlate to metastatic propensity and epithelial phenotypic in a CTC.

Thus, EMT biomarker expression may be used to detect CTCs. EMT biomarker expression, or detection of CTCs, or a combination thereof, may be used to assess cancer prognosis, tumor invasiveness, risk of metastasis, or to stage tumors. As mentioned above, the methods described herein can include any suitable method for evaluating EMT biomarker expression including, but not limited to, detection with antibodies, real time RT-PCR, Northern analysis, magnetic particles (e.g., microparticles or nanoparticles), Western analysis, and any method or system involving flow cytometry. In some embodiments, the methods and EMT biomarkers can be used in a commercially available system such as a system that has been approved by a regulatory agency (e.g., FDA) including, for example, CellSearch® technology (Veridex LLC). Thus, the methods can incorporate standard protocols that are known in the art. For example, embodiments comprising CellSearch® technology can include detecting the presence of an EMT biomarker, and correlated to quantifying the number of circulating tumor cells (CTCs) a biological sample, (e.g., blood collected from women in need of a new treatment regimen for metastatic breast cancer, or men in need of treatment for mCRPC). Typical protocols can include drawing blood sample sizes of about 15 mL that can be collected at any particular time (suitably when the patient starts the new therapy, and then again at three to four week intervals). The number of CTCs can be correlated with disease response or progression as determined by standard radiology studies (e.g., CT scans) performed every nine to 12 weeks.

In an aspect, the disclosure relates to a method for detecting a circulating tumor cell (CTC) in a biological sample, wherein the method comprises detecting at least one epithelial mesenchymal transition (EMT) biomarker in the biological sample. As noted above, a biological sample can be from any tissue or fluid from an organism. In some embodiments the biological sample is from a bodily fluid or tissue that is part of, or associated with, the lymphatic system or the circulatory system of the organism. In some embodiments the biological sample is a blood sample.

The epithelial mesenchymal transition (EMT) and cellular plasticity biomarkers used in the methods described herein are associated with circulating tumor cells (CTCs). Accordingly, in various embodiments the methods include detecting the presence of one or more EMT biomarker and correlating that detection with the presence of a CTC, optionally quantifying the number of CTCs in the sample. As discussed herein, EMT biomarkers can include any detectable biomolecule that is associated with a transitional cell that exhibits characteristics (e.g., phenotype, or surface antigen or gene expression profiles, etc.) of plasticity, stem-like properties, invasiveness, and/or chemo-resistance of a cell. In some non-limiting embodiments, the EMT biomarker includes any of vimentin, N-cadherin, O-cadherin, E-cadherin, FGFR2 splice variant isoforms (such as, for example FGFR2 that includes or excludes either exon IIIc or exon IIIb), or CD133, or any combination of two or more thereof. In some embodiments, the EMT biomarker can include one or more of vimentin (polypeptide SEQ ID NO: 14 encoded by polynucleotide SEQ ID NO: 13), N-cadherin (polypeptide SEQ ID NO: 2 encoded by polynucleotide SEQ ID NO: 1; polypeptide SEQ ID NO: 16 encoded by polynucleotide SEQ ID NO: 15), O-cadherin (polypeptide SEQ ID NO: 4 encoded by polynucleotide SEQ ID NO: 3; polypeptide SEQ ID NO: 18 encoded by polynucleotide SEQ ID NO: 17), E-cadherin (polypeptide SEQ ID NO: 12 encoded by polynucleotide SEQ ID NO: 11; polypeptide SEQ ID NO: 24 encoded by polynucleotide SEQ ID NO: 23), FGFR2 (polypeptide SEQ ID NO: 8 encoded by polynucleotide SEQ ID NO: 7; polypeptide SEQ ID NO: 10 encoded by polynucleotide SEQ ID NO: 9; polypeptide SEQ ID NO: 22 encoded by polynucleotide SEQ ID NO: 21), and CD133 (polypeptide SEQ ID NO: 6 encoded by polynucleotide SEQ ID NO: 5; polypeptide SEQ ID NO: 20 encoded by polynucleotide SEQ ID NO: 19). In some embodiments, the EMT biomarker can include one or more of N-cadherin, for example human N-cadherin (for example SEQ ID NO: 16, CCDS ID No: CCDS11891.1); O-cadherin, for example human O-cadherin (for example SEQ ID NO: 18, CCDS ID No: CCDS10803.0); E-cadherin, for example human E-cadherin (for example SEQ ID NO: 24, CCDS ID No: CCDS10869.1); CD133, for example human CD133 (for example SEQ ID NO: 20, CCDS ID No: CCDS47029.1); FGFR2, for example human FGFR2 (for example SEQ ID NO: 22, CCDS ID No: CCDS31298.1); and vimentin, for example human vimentin (for example SEQ ID NO: 14, Accession No. BC000163). It will be understood by one of skill in the art that when reference is made to polynucleotides that encode polypeptides in the above embodiments as well as embodiments throughtout, the polynucleotide can be disclosed as either an RNA (e.g., mRNA) or a DNA (e.g., cDNA).

The EMT biomarkers can be associated with any organism (ortholog) and in certain embodiments are EMT biomarkers associated with a human. Any portion or the entirety of an EMT biomarker can be used for detecting in the methods described herein such as, for example, an epitope of an EMT biomarker protein that binds to an antibody, or a nucleic acid sequence of an EMT biomarker an expressed or transcribed mRNA molecule that is complementary to a reporter nucleic acid probe or primer. In some embodiments, the methods provide for detecting expression of at least two EMT biomarkers. In certain embodiments, expression of vimentin and E-cadherin are detected. In certain embodiments, expression of N-cadherin and O-cadherin are detected. This measure may be used alone or in combination with another method to detect CTCs. In certain embodiments, the methods described herein may be used as a supplemental method in conjunction with CellSearch® Circulating Tumor Cell Test (noted above). Thus, embodiments provide for a method as part of a dual or complementary detection system that can be used to detect and optionally quantify CTCs in a sample (e.g., comprising the detection of EpCAM and at least one EMT biomarker). The expression of at least one EMT biomarker may be used to isolate CTCs. The expression of at least one EMT biomarker may be used to count or provide a relative number or amount of CTCs, using any known method for correlating detection of a biomarker to a cell, such as a CTC. CTCs may be detected at the time of, prior to, or after metastasis.

Cancers may include, but are not limited to, breast cancer, colon cancer, lung cancer, prostate cancer, testicular cancer, brain cancer, skin cancer, rectal cancer, gastric cancer, esophageal cancer, sarcomas, tracheal cancer, head and neck cancer, pancreatic cancer, liver cancer, ovarian cancer, lymphoid cancer, cervical cancer, vulvar cancer, melanoma, mesothelioma, renal cancer, bladder cancer, thyroid cancer, bone cancers, carcinomas, sarcomas, and soft tissue cancers. Thus, the disclosure is generally applicable to any type of cancer in which expression of an EMT biomarker occurs. In certain embodiments, the cancer is a solid tumor malignancy. In certain embodiments, the cancer is breast, colon, or prostate cancer.

Expression of at least one EMT biomarker may be detected using any suitable method known in the art, including but not limited to, binding with antibodies or fragment thereof, antibodies tethered to or associated with an imaging agent, expression reporter plasmids, flow cytometry, and any suitable array scanner technology. The antibody or fragment thereof may suitably recognize a particular intracellular protein, protein isoform, or protein configuration.

As used herein, an "imaging agent" or "reporter molecule" is any entity which enhances visualization or detection of the cell to which it is delivered. Any type of detectable reporter molecule/imaging agent can be used in the methods disclosed herein for the detection of one or more EMT biomarker. Such detectable molecules are known in the art and include, for example, magnetic beads, fluorophores, radionuclides, nuclear stains (e.g., DAPI). For example, an imaging agent can include a compound that comprises an unstable isotope (i.e., a radionuclide) or a fluorescent moiety, such as Cy-5, Alexa 647, Alexa 555, Alexa 488, fluorescein, rhodamine, and the like. Suitable radionuclides include both alpha- and beta-emitters. In some embodiments, the targeting vehicle is labeled. In other embodiments, suitable radioactive moieties include labeled polynucleotides and polypeptides which can be coupled to the targeting vehicle. In some embodiments, the imaging agent comprises a radionuclide such as, for example, a radionuclide that emits low-energy electrons (e.g., those that emit photons with energies as low as 20 keV). Such nuclides can irradiate the cell to which they are delivered without irradiating surrounding cells or tissues. Non-limiting examples of radionuclides that are can be delivered to cells include ¹³⁷Cs, ¹⁰³Pd, ¹¹¹In, ¹²⁵I, ²¹¹At, ²¹² Bi and ²¹³Bi, among others known in the art. Further imaging agents suitable for delivery to a cell in accordance with some embodiments include paramagnetic species for use in MRI imaging, echogenic entities for use in ultrasound imaging, fluorescent entities for use in fluorescence imaging (including quantum dots), and light-active entities for use in optical imaging. A suitable species for MRI imaging is a gadolinium complex of diethylenetriamine pentacetic acid (DTPA). For positron emission tomography (PET), ¹⁸F or ¹¹C may be delivered. Other non-limiting examples of reporter molecules are discussed throughout the disclosure.

In an aspect, the disclosure provides a kit for detecting CTCs in a sample. In embodiments, the kit comprises an antibody to at least one EMT biomarker. The antibody in the kit can be connected to or associated with an imaging agent. In embodiments, the kit can comprise an antibody to at least one EMT biomarker, wherein the antibody is associated a magnetic bead. The magnetic bead may be used for ferromagnetic separation and enrichment of CTCs.

Aspects also relate to methods of predicting responsiveness of a subject to a cancer drug. The methods may comprise determining the level of expression of at least one EMT biomarker in a sample from the subject. The level of expression of at least one EMT biomarker may be used to obtain a gene expression pattern in CTCs for the subject. The methods may further comprise predicting responsiveness of the subject to the cancer drug based on the gene expression pattern obtained. Genome variation in CTCs from the subject may also be determined.

Also provided are methods of providing a cancer prognosis to a subject. The methods may comprise determining the level of expression of at least one EMT biomarker in a sample from the subject. The level of expression of at least one EMT biomarker may be used to determine the number of CTCs in the sample. The CTCs may be captured using at least one EMT biomarker. The level of expression of at least one EMT biomarker may be used to determine a gene expression pattern in the CTCs for the subject. A prognosis may be provided to the subject based on the gene expression pattern obtained.

Also provided are methods for following the progress of cancer in a subject. The methods may comprise determining the level of expression of at least one EMT biomarker in samples from the subject at a first and a second time, and comparing the first and second levels of expression. The level of expression of at least one EMT biomarker in the sample may be determined over time, such as following initiation of a new cancer therapy. The level of expression of at least one EMT biomarker in the sample may be used to determine the number or amount of CTCs. An increase between the first and second levels may indicate progression of the cancer. A decrease between the first and second levels may indicate remission or response of the cancer to the therapy. No difference between the first and second levels may indicate arrest or stability in the progression of the cancer.

Also provided are methods of screening for cancer in a subject. The methods may comprise determining the level of expression of at least one EMT biomarker in a sample from the subject. The level of expression of at least one EMT biomarker may be used to determine the amount or number of CTCs in the subject. The level of expression of at least one EMT biomarker may be compared to a normal or control sample. An increased level of at least one EMT biomarker may indicate presence of cancer in the subject.

Also provided are methods of arresting cell growth or inducing cell death of a cancer cell expressing an EMT biomarker. The methods include contacting the cancer cell with a conjugate capable of mediating intracellular delivery of an agent, such as the antibodies to EMT markers described herein. The agent is capable of arresting or attenuating the growth of the cell or inducing cell death through any mechanism after agent internalization. The cancer cell may be contacted with the conjugate *in vitro*, *in vivo,* or *ex vivo.* These methods may be useful in treating cancer by directly targeting cancer cells expressing an EMT biomarker for delivery of agents capable of decreasing or arresting cell growth or inducing cell death.

The disclosure also provides for targeted therapeutic methods and molecules that comprise an anti-cancer agent linked to a binding agent that targets at least one EMT as described herein. In some embodiments the link between the anti-cancer agent and the binding agent is a covalent bond. In some embodiments the link is formed by strong electrostatic interactions (hydrogen bonds, hydrophilic/hydrophobic interaction, or oppositely charged moieties, and the like). Any anti-cancer agent can be used in such molecules and therapeutic methods, and can be selected by one of skill in the art based on the type of cancer to be treated, the progress/stage of the cancer, potential adverse drug interactions, dosage requirements, administration schedule, and the like.

### EXAMPLES

### Example 1. Materials and Methods

*Plasmids and cell cultures.* The minigene used (pRIIIcl²) was previously described (S. Oltean et al., Pro Natl Acad Sci USA 2006, 103, 14116, incorporated herein by reference in its entirety). All cell lines were cultured in low glucose DMEM (Invitrogen) with 10% FBS and 15 IJg/mL blasticidin. Single cell progenies were isolated from a population of AT3 cells stably transfected with pRIIIcl² minigene by limiting dilution to produce a concentration of 1 cell/10 wells and plated on 96-well plates. Cells were counted using a hemocytometer to obtain an initial concentration of 1 x 10⁵ cells/mL. Through a series of progressive dilutions a final concentration of 1 cell/mL was obtained and 100 IJI were pipetted in each well of three 96-well plates. All wells were monitored through bright field microscopy, those appearing to contain more than one cell were excluded, and those containing single cells were further cultured into 25 mL flasks. 16 of an expected 27 clones were obtained using this procedure in a first round.

To measure cell population growth rate *in vitro*, cells were plated at 50,000/well in 6-well dishes. Viable cells were counted using Trypan Blue staining at 24, 48, 72, and 96h.

*Animals and tumor cell implantation.* Cells were trypsinized, washed, and resuspended in PBS at a final concentration of 3 x 10⁵ cells/mL, and kept on ice for less than 30 minutes before implantation. Cells (3 x 10⁵) were injected subcutis in both flanks of Copenhagen 2331 rats (Harlan Labs, Indianapolis, IN; 75-90 g, 2 months of age). Animals were continuously monitored for tumor growth. All animal procedures were approved by the Duke University Institutional and Animal Care and Use committee and followed NIH guidelines. Sacrifice curves were compared using a Mantel-Haenszel logrank test. Tumor volume was compared using an unpaired t test. Prism 4.0c for the Macintosh (Graphpad, La Jolla, CA) was used for statistical analyses.

*Histological sections and analysis.* Excised tumors and lungs were washed in PBS at room temperature. Depending on the size of the lungs, they were frozen either together or separately. The tumor sections and the lungs were placed in cryomolds, embedded in optimal-cutting-temperature tissue sectioning medium (Sakura Finetek, Torrance, CA), snap-frozen in liquid nitrogen, and stored at 80°C. Slides for fluorescence imaging were prepared as follows: the tissue was incubated for 2-3 h at -20°C to equilibrate the temperature and then sectioned with a microtome. The sections (15 µm) were placed on glass slides, fixed in 4% (wt/vol) paraformaldehyde for 30 min at room temperature, and rinsed in PBS at room temperature. The slides were mounted with gel/mount media (Biomeda, Foster City, CA). The sections were analyzed by using an Olympus (Melville, NY) IX 71 epifluorescence microscope, and images were acquired by using an Olympus DP70 digital camera. Image processing was done with DP Controller software (Olympus). For hematoxylin-eosin staining after fluorescence imaging, the slides were incubated in warm water for 15-20 minutes for the cover slip to come off, slides were dried, and staining was performed according to standard procedure.

*RNA extraction from tumor sections.* Sections were fixed in 4% (wt/vol) paraformaldehyde for 5 minutes, rinsed in PBS, and imaged. DsRED+ and DsRED- regions of the sections were marked on the slide. The slide was immersed in warm water for 5 minutes to remove the coverslip and the DsRED+ and DsRED- regions scraped off. RNA isolation was further performed as described before (N. Masuda, T. Ohnishi, S. Kawamoto, M. Monden, K. Okubo, Nucleic Acids Res 1999, 27, 4436, incorporated herein by reference in its entirety). Briefly, samples were treated with proteinase K in digestion buffer containing SDS, and further isolation of RNA was performed using the RNeasy kit (QIAGEN, Valencia, CA).

*Immunoblots.* Cells were collected from confluent 25 cm² tissues flasks by scraping, washed in PBS, and lysed in sample buffer. Whole cell lysates were serially diluted in sample buffer, fractionated via 7.5% SDS-PAGE, and transferred to PVDF. Membranes were cut in half. The bottom half was probed with anti-β-actin at 1:1000 or 1:5000 (Santa Cruz Biotechnology, CA, 47778) as an internal loading control, while the top half was probed with anti-CD 133 (Santa Cruz Biotechnology, CA, 30219) at 1:200 or anti-CD44 (Santa Cruz Biotechnology, CA, 7946) at 1:200.

*Gene expression Analysis.* Triplicate cultures of AT3-M and AT3-T cells were grown to -60% confluency. Total RNA was isolated using the RNeasy kit (Qiagen, Valencia, CA), and triplicate samples were submitted to the Duke Microarray Facility. Gene expression analysis was performed using the R027K rat spotted arrays 3.0 (Operon, Huntsville, AL). Bioinformatical analysis of expression differences between AT3-M and AT3-T cells was done using the GeneSpring GX software version 7.3.1 (Agilent Technologies, Durham, NC). The data files (representing signals for 26,986 gene probes in all six data points, three for AT3-M and three for AT3-T) were normalized using the feature: per Spot and per Chip - intensity dependent (Iowess) normalization. The resulting gene list was used to determine the significantly differentially expressed genes between AT3-M and AT3-T using the "Filtering on Volcano plot" feature with the following characteristics: (1) Test type: Parametric test, don't assume variances equal; (2) Multiple testing correction: None; (3) Fold Difference: Twofold or greater and a P-value cutoff of 0.05.

*Analysis of human circulating tumor cells.* Patients eligible for the CTC biomarker protocols included (1) men with progressive CRPC, with metastatic progression by PSA (two consecutive rises over nadir separated by >1 week) or radiologic criteria (RECIST or new bone scan lesions), a PSA ≥5, age ≥18 years; or (2) women with mBC with disease progression or with initiation of a new systemic therapy, who were > 18 years of age, and who were at least 7 days from treatment with an anthracycline-containing regimen. Blood (15 mL) was collected from patients and processed within 48 hours at the Duke University CTC lab using the Cell Search System (Veridex, Raritan, NJ). Veridex profile kits were used, which isolate EpCAM positive cells without additional staining. The isolated cells were either processed immediately or stored overnight in 4% paraformaldehyde and processed the next day. Immunostaining was done on teflon coated slides. Briefly, cells were pipetted into the wells of the slides and left to settle for ~30 minutes followed by standard immunostaining procedures with careful aspiration to minimize cell loss. An initial ferromagnetic wash using a benchtop magnet was performed to further isolate CTCs, with resuspension of the cell pellet after magnet release 100 uL PBS. Following 4% PFA fixation and permeabilization with PBT (PBS with 2% Triton) and blocking with 10% goat serum for 30 minutes, triple immunostaining was performed using CD45 antibody (AbCam #33533-50) labeled with Alexa 647, cytokeratin (AbD Serotec #MCA 1907HT) labeled with Alexa 555, and Vimentin (BD Biosciences, San Jose, CA #550513) labeled with Alexa 488. Nuclear staining with 4',6-diamidino-2phenylindole (DAPI) was then performed. A CTC was defined as an intact cell by microscopic examination, containing an intact nucleus and expressing cytokeratin but lacking CD45 staining, using appropriate controls (see Table 1 for antibodies and controls). Human peripheral blood mononuclear cells (PBMCs), obtained by Ficoll purification of buffy coats from normal donors, were kindly provided by Micah Luftig (Duke University, Durham NC) and used as control cells for CD45 expression. Linear regression analysis was performed to compare CTC count (standard Cellsearch method) against the proportion of CTCs that co-express vimentin. Goodness of fit was tested by analysis of variance.

**Table 1. EMT/Stemness Antigens to be assessed in CTCs.**

| **Antigen** | **Product** | **Positive Control** | **Negative Control** | **Leukocyte Expression** | **Dilution** |
|---|---|---|---|---|---|
| Vimentin | BD Biosciences, mouse monoclonal IgG1 | PBMCs, PC-3, DU145 | T47D, LnCAP | Yes | 2:225 |
| N-cadherin | DAKO, mouse monoclonal IgG1, 6G11 | Sarcoma, rat brain, PC-3 | DU145, T47D, mock | No | 4:225 |
| Cytokeratin (pan) | AbD Serotec, mouse monoclonal IgG1, MCAI907HT, clone AEI/AE3 | T47D, DU145 | PC-3, PBMCs | No | 2:45 |
| CD45 | Invitrogen, mouse IgG1, HI30, MHCD4500 | PBMC | PC-3, DU145 | Yes | 1:45 |
| CD 133 | Santa Cruz mouse monoclonal IgG, sc-130127 | CaCo-2 colon cancer cells | Mock | Variable | 4:225 |

The slides were mounted with gel/mount media (Biomeda, Foster City, CA). The slides were analyzed with an Olympus (Melville, NY) IX 71 epifluorescence microscope, and images were acquired using an Olympus DP70 digital camera. Image processing was done with DP controller software (Olympus). All fields were analysed, with each cytokeratin positive nucleated cell that was CD45 negative being counted as a CTC. Positive control cells for each antibody included PC-3 cells for vimentin, peripheral blood mononuclear cells (PBMCs) for CD45, and T47D breast cancer cell lines for cytokeratin. A similar volume of reaction mix without antibody was used for negative controls.

Media exchange experiments. The cells of AT3-T or AT3-M clones were plated at a concentration of 150,000 cells/2 mL of media in 6-well plates and allowed to incubate for 24 h. The conditioned media was then filtered using a 0.22 µm filter, and then immediately allowed to incubate with cells of the other clone, which was plated at the same concentration and had its media aspirated and cells washed with 2 mL of PBS. All cells with media replaced were incubated for 72 h, and phase and epifluorescent microscopy was used to monitor cell phenotypes 24, 48, and 72 h after treatment. Control plates, in which media was conditioned, cells washed with PBS and media added back to the same cells, were also used.

*Scratch-wound assay.* Cells were plated and left to grow to nearly 100% confluency in 6-well dishes. A wound was simulated by scratching the cells with a sterile 200 IJI pipette tip. The wells were washed twice with PBS and fresh media added. Pictures were taken in the same marked spot at 0, 24, and 48 h. Percent migration was calculated as (width at 0 h - width at 24 or 48 h) 1 width at 0 h x 100. Relative migration was compared using two-way analysis of variance via Prism 4.0c for the Macintosh (Graphpad, La Jolla, CA).

*Matrigel assay.* Matrigel assay was performed per manufacturer's indications (BO Biosciences). Briefly, after rehydration, 2 x 10⁵ cells were plated either in the control or in the matrigel-coated inserts and incubated for 22 h. Following incubation, the non-invading cells from the upper-part of the inserts were removed using cotton-tipped swabs. The cells from the lower part of the membrane were stained with hematoxylin-eosin, membranes were removed, placed on a slide and observed under the microscope.

*Immunohistochemical (IHC) analysis of metastases.* Under the same informed consent protocol as the analysis of human circulating tumor cells described above, men undergoing CTC collection additionally consented to have a radiologic-guided metastatic biopsy for analysis of biomarker expression by IHC. Samples were obtained through core needle biopsies during light sedation, and immediately formalin-fixed and paraffin embedded. For analysis, slides were deparaffinized, rehydrated, and endogenous peroxidase was inactivated for 30 min. in 0.3% H₂O₂ (hydrogen-peroxide) in methanol. Specific antigen retrieval steps were performed for individual antigens. Three markers were evaluated by IHC: vimentin (M7020, Dako, 1:150; antigen retrieval with pepsin treatment at 37°C for 15 minutes), cytokeratin cocktail (18-0132, Invitrogen, 1:50 and 349205, BD Biosciences 1:50, antigen retrieval with pepsin treatment at 37°C for 15 minutes), and CD45 (M0701, Dako, 1:200; antigen retrieval with sodium citrate 10 mM, pH 6.0 at 100°C for 30 minutes). Primary antibody was incubated for 60 minutes at room temperature. Dako Envision horseradish peroxidase secondary antibody was used for 30 minutes at room temperature and the signal was detected with DAB reagent (Vector kit SK 4100). Slides were counter stained with hematoxylin and eosin and assessed by a trained pathologist for expression using appropriate positive (localized prostate tissue microarray sections) and negative controls (mock antibody) for each marker.

*Statistical analyses*. To determine the significantly differentially expressed genes between AT3-M and AT3-T the GeneSpring GX "Filtering on Volcano plot" feature was used with the following characteristics: (1) Test type: Parametric test, don't assume variances equal; (2) Multiple testing correction: None; (3) Fold Difference: Twofold or greater and a P-value cutoff of 0.05. To compare CTC count (standard Cellsearch® method) against the proportion of CTCs that co-express vimentin, N-cadherin, or CD133, linear regression analysis was performed. Goodness of fit was tested by analysis of variance.

### Example 2. Isolation of individual AT3 clones that inhabit an intermediate phenotypic state

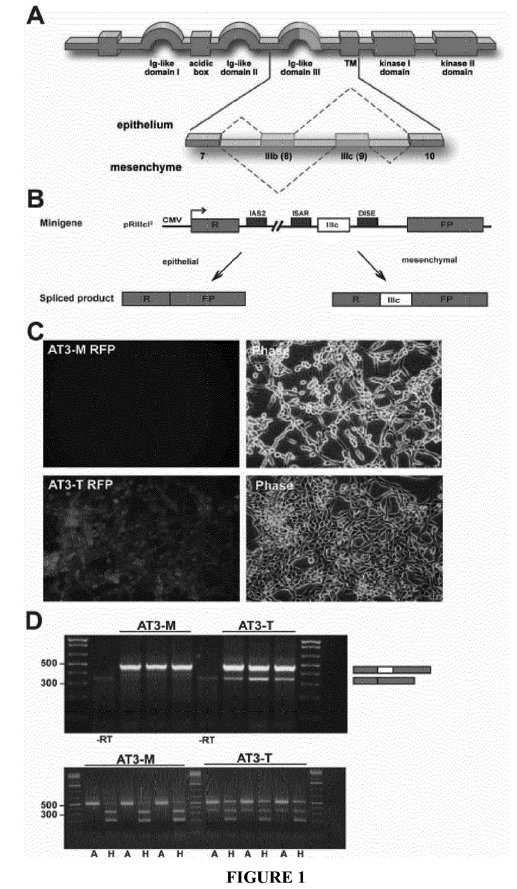

The alternative splicing of FGFR2 transcripts, which produces either FGFR2-IIIb or - IIIc variants in epithelial and mesenchymal cells respectively, is exquisitely regulated (Figure 1A). In Figure 1A is a schematic representation of the IIIb and IIIc alternatively spliced isoforms of FGFR2. FGFR2 contains an extracellular domain (with three IgG-like domains), a transmembrane domain (TM), and two intracellular tyrosine kinase domains. The IIIb isoform is found in epithelial cells while the IIIc isoform in mesenchymal cells. Exons IIIb and IIIc are regulated coordinately to provide mutually exclusive expression of the two isoforms and transcripts including both exons are destabilized by nonsense-mediated decay. We have previously used FGFR2 alternative splicing reporters, in particular constructs that measure the epithelial-specific silencing of exon IIIc (e.g., pRIIIcl² in Figure 1B), to report on the phenotypic state of cells *in vitro* and *in vivo.* In Figure 1B is a schematic of the pRIIIcl² minigene and the fluorescence read-out. The minigene contains the DsRED open reading frame interrupted by exon IIIc and flanking introns of the FGFR2 gene. In epithelial cells exon IIIc is skipped, DsRED open reading frame is formed and results in fluorescence signal. In mesenchymal cells, exon IIIc is included and the DsRED open reading frame is disrupted, resulting in low or close-to-background fluorescence signal. The pRIIIcl² splicing reporter, which produces a variant red fluorescence protein (DsRED) when exon IIIc is silenced, revealed MET in primary tumors derived from AT3 cells implanted in the flanks of Copenhagen white rats. While most tumors contained MET foci, each tumor had very few foci and these were not randomly distributed but rather were associated with collagenous stroma. In contrast to the low frequency of MET in primary tumors, a high incidence of MET among lung metastases in these animals was observed, suggesting an unexpected association between the more epithelial phenotype and aggressive behavior. These studies could not ascertain whether the epithelial-like AT3 cells found in the lungs had undergone MET in the primary tumors or during the process of metastasis.

In an attempt to find post-MET cells *in vitro*, limiting dilution was used to obtain clones from AT3 cells stably transfected with the pRIIIcl² reporter. A total of 16 clones of a maximum calculated recovery of 27 were obtained, which is ~ 60% cloning efficiency. Eleven of these sixteen clones expressed RIIIcl² transcripts (italicized in Table 2), and of these, eight expressed DsRED (Table 2). Some of the clones had an epithelial-like morphology (cells with cobblestone appearance and adherent to each other), while others had a mesenchymal-like morphology (spindle-shaped), as well as clones that displayed a mixed phenotype. It is important to note that given the high cloning efficiency and the high frequency of DsRED+ clones, it is highly unlikely for these epithelial-like clones to come from a very small population within the parental AT3 cells. Rather, the process of subcloning induced a phenotypic transition in a significant number of the AT3 cells.

**Table 2. Properties of AT3 clones.**

| **AT3 Clones** | **Cellular morphology³** | **DsRED expression²** | **Detection of exon IIIc skipping among RIIIcI3 transcripts¹** | **FGFR2 transcipts detected³** |
|---|---|---|---|---|
| *1* | *Epithelial* | *High* | + | *IIIc* |
| *2* | *Epithelial* | *High* | + | *IIIc* > *IIIb* |
| 3 | Epithelial | Low | ND | IIIc > IIIb |
| 4 | Epithelial | Low | ND | IIIc |
| 5 | *Epithelial* | *High* | + | *IIIc* > *IIIb* |
| 6 | Mesenchymal | Low | ND | IIIc |
| 7 | Mixed | Low | ND | IIIc |
| 8 | *Mixed* | *High* | + | *IIIc* |
| 9 | Mixed | Low | ND | IIIc |
| *10* | *Mixed* | *High* | + | *IIIc* |
| *11* | *Mesenchymal* | *Low* | - | *IIIc* |
| *12* | *Mesenchymal* | *Low* | - | *IIIc* |
| *13* | *Epithelial* | *High* | -*1* | *IIIc* > *IIIb* |
| 14 | *Epithelial* | *Low* | - | *IIIc* |
| *15* | *Epithelial* | *High* | + | *IIIc* |
| *16* | *Mixed* | *High* | - *2* | *IIIc* |

| | | | | |
|---|---|---|---|---|
| ¹See Figure 1C. A "+" indicates detection of RIIIcl² transcripts missing exon IIIc, a "-" all RIIIcI² transcripts include exon IIIc, ND means that no RIIIcl² transcripts were detected. ²Determined by epifluorescence microscopy (high is defined as fluorescence above background of naive AT3 cells and low undistinguishable from the same cells). ³Discussed further herein and illustrated in Figure 1C. | | | | |

All of the clones obtained by limiting dilution were analyzed to determine the splicing status of RIIIcl² and endogenous FGFR2 transcripts. We could not detect exon IIIc skipping among pRIIIcl² transcripts or any evidence of exon IIIb inclusion among endogenous FGFR2 transcripts in clones with a mesenchymal-like morphology (Figure 1C and Table 2). Figure 1C shows RT-PCR analysis of the reporter (upper panel) and endogenous FGFR2 (lower panel). Primers used for the reporter are designed in the DsRED regions flanking exon IIIc. RT-PCR shows a higher percentage of the skipped product in clone AT3-T compared to clone AT3M. Reactions that did not include RT (-RT) reveal a contaminating product that is out-competed by the presence of a bona fide cDNA template (AT3-M lanes). Since exons IIIb and IIIc differ in size by only 3 nucleotides, analysis of the presence of IIIb or IIIc exons in FGFR2 gene was done by using primers in the flanking exons and specific restriction digestion of the resulting RT-PCR products. Exon IIIb is digested by Aval (A) and IIIc by HincII (H). There is a higher percentage of exon IIIb in clone AT3-T. The RT-PCR are replicates from three different cultures of the two clones. These clones did not express detectable levels of DsRED (Figure 1D and Table 2). Figure 1D shows epifluorescence and phase-contrast pictures of clones AT3-M and AT3-T shows the difference in fluorescence intensity and morphology between the two clones. Epifluorescence pictures were taken at the same exposure. All pictures were acquired at 200X magnification. While the skipping of exon IIIc among pRIIIcl² transcripts from epithelial-like clones could be expected, the observation that all of these clones both skipped and included exon IIIc was unexpected (Figure 1C, Table 2 and data not shown). Analysis of endogenous FGFR2 transcripts revealed that four of the clones with epithelial morphology and DsRED expression had clear evidence of coexpression of both IIIb and IIIc isoforms (Table 2, and Figures 1C and 1D). As shown in Figure 1, AT3-T cells expressed epithelial and mesenchymal isoforms of FGFR2. The expression of DsRED in all the cells suggested that each cell in the culture was expressing both isoforms (Figure 1C).

We followed two clones with epithelial morphology, high DsRED levels and coexpression of FGFR2-IIIb and -IIIc transcripts (clone 2 and clone 5 (clone 5 herein AT3-T)) and noted that the phenotypic characteristics described above were stable for over six months. Equally, we followed clone 11 (clone 11 herein AT3-M) and clone 12 for six months, and noted that the mesenchymal morphology, undetectable DsRED expression and exclusive production of FGFR2-IIIc were also stable. We concluded from these observations that AT3 cells were plastic and were coaxed by sub-cloning to populate intermediate phenotypic states, with properties of epithelial and mesenchymal cells.

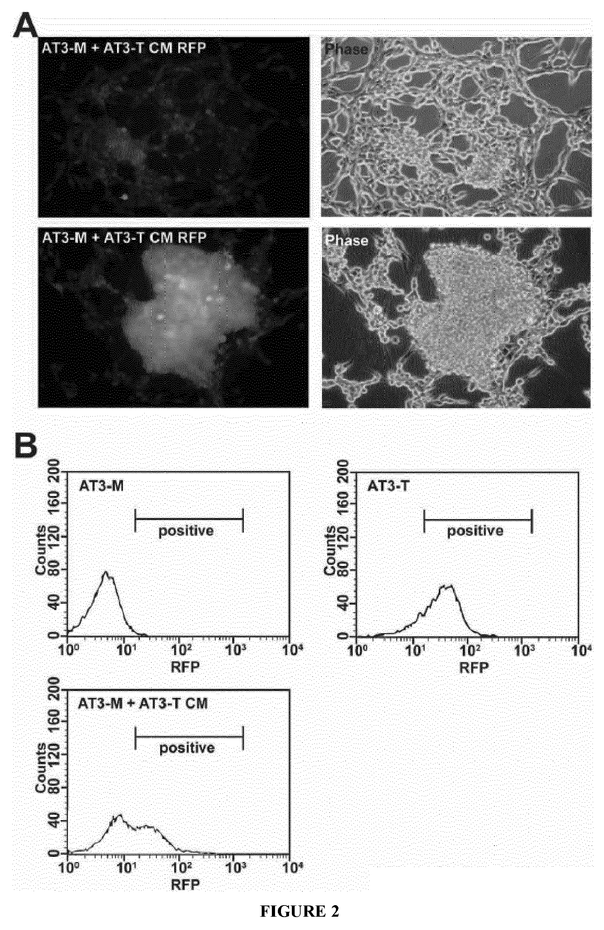

A media exchange experiment was used to investigate whether or not the splicing of RIIIcI² transcripts in the DsRED expressing clones was regulated by soluble factors.. Media conditioned by DsRED expressing clones (clone 5 in Table 2) was filtered and added to DsRED negative clones (clone 11 in Table 2). DsRED+ cells were observed among DsRED-cells incubated with DsRED+ conditioned media (Figure 2). Figure 2A shows examples of clusters of DsRED positive cells formed by AT3-M cells upon treatment with conditioned media from clone AT3-T. Media was conditioned for 24 h, filtered and added on AT3-M cells. Pictures (acquired at 200X) are taken 48 h following media exchange. Figure 2B shows results from flow cytometry analysis of the same experiment. Left upper panel represents clone AT3-M conditioned with media from the same clone, as a negative control. Right upper panel represents clone AT3-T, which is DsRED positive. The lower panel represents clone AT3-M 48 h after conditioned media from clone AT3-T was added. Different lots of fetal bovine serum caused variation in this effect. This effect was quantified by flow cytometry and these data suggested that about half of the DsRED- cells were induced to express DsRED at levels equivalent to those seen in DsRED+ cells (Figure 2). The changes observed were not due to prolonged culture of the cells in the same wells because conditioned media from a separate DsRED- culture did not induce DsRED expression. As shown in Figure 2, AT3-T conditioned media induced AT3-M cells to express DsRED. These observations suggest that soluble factors secreted by the DsRED+ clones or dilution of factors extant in the DsRED- conditioned media may contribute to plasticity.

### Example 3. AT3-M and AT3-T cells are tumorogenic

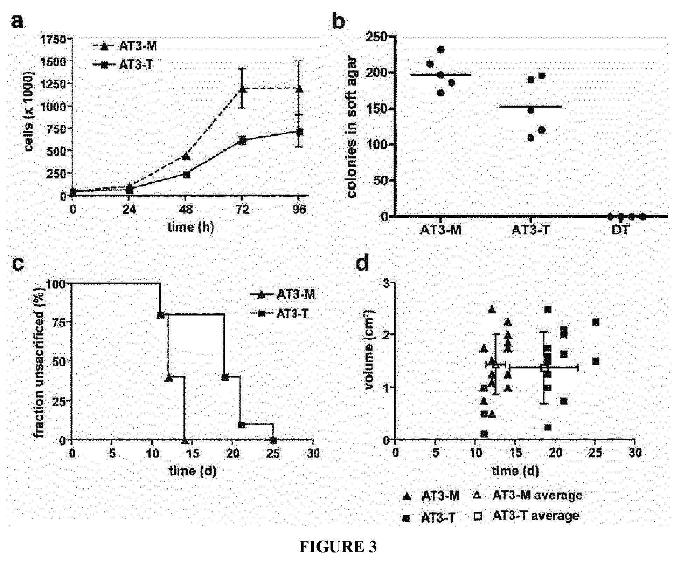

The initial characterization of the AT3-T revealed that these transitional cells grew slower and reached a lower confluent density than the AT3-M (Figure 3A). Figure 3A shows growth curves for clones AT3-T and AT3-M. Cells were plated at 0 h time-point, trypsinized, and counted at the indicated times. Data are the mean ± S.D. (n = 3). To investigate their growth *in vivo* AT3-M and AT3-T cells were co-transfected with pGint a plasmid that expresses EGFP (herein GFP) in both mesenchymal and epithelial cells, and sorted stable populations of each cell line using flow cytometry for uniform GFP intensity. The GFP expressing cells maintained the morphological characteristics, the differential DsRED expression, and the differences in the splicing of pRIIIcl² and FGFR2 transcripts first observed after sub-cloning.

We injected 3 x 10⁵ GFP-expressing AT3-T or AT3-M cells subcutis in both flanks of Copenhagen white 2331 male rats. All of the animals developed bilateral tumors, indicating that both AT3-M and AT3-T cells were highly tumorogenic in these syngeneic rats. As a humane endpoint, rats were sacrificed when tumor length estimated by palpation reached 1 cm. The *in vivo* growth curves for the AT3-M and AT3-T tumors were significantly different, as determined by a logrank test (p = 0.0020; Figure 3B). Figure 3B is a sacrifice curve for rats injected with AT3-M or AT3-T cells. Figure 3C shows comparison of tumor volumes resulting from AT3-T and AT3-M injection. The Y-axis represents tumor volumes at the time of sacrifice of the animals and the X-axis days from the time of implantation to the time of sacrifice. Average tumor volumes and average days until sacrifice are represented with S.D. bars. Some points represent more than one tumor with the same volume on the same day. Tumor volume was measured (Figure 3C) and although most AT3-T animals were sacrificed later, there was no significant difference in tumor size (p = 0.76). As shown in Figure 3, AT3-T cells grew more slowly than the mesenchymal-like AT3-M cells *in vitro* and *in vivo,* but both were equally tumorogenic. We concluded that whereas AT3-T cells grew more slowly *in vitro* and *in vivo* relative to their more mesenchymal siblings, these transitional cells were capable of forming tumors.

### Example 4. Both AT3 M and AT3-T areplastic

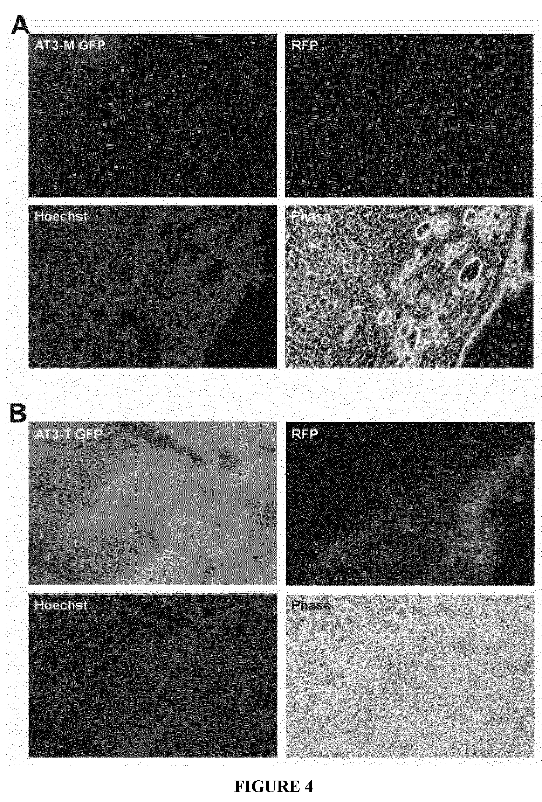

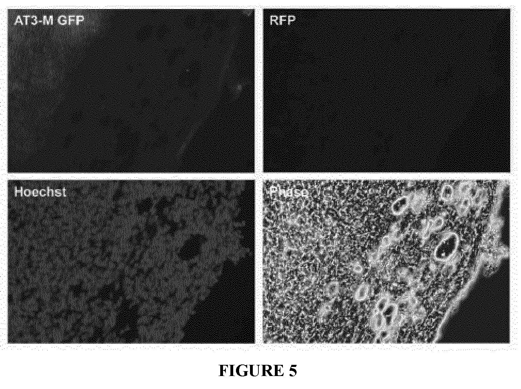

Since the implanted AT3-M and AT3-T cells could be tracked by GFP expression, and epithelial character could be interrogated by DsRED expression, the plasticity of the tumors were able to investigated. The overwhelming majority of cells in AT3-M tumors expressed GFP but not DsRED (Figure 4A). As shown in Figure 4, tumors from both AT3-T and AT3-M clones have evidence of plasticity. Figure 4A shows representative example of cells that express both RFP and GFP at the periphery of an AT3-M tumor stably transfected with Gint and pRIIIcl² reporters. Pictures were taken at 200X magnification. To compensate for a low RFP signal, the color curve of the entire picture was adjusted. Nonetheless, groups of cells were observed expressing both GFP and DsRED in many AT3-M tumor sections, especially near the tumor capsule, (Figure 4A; see also Figure 5). Figure 5 shows a representative example of cells that express both RFP and GFP at the periphery of an AT3-M tumor stably transfected with Gint and pRIIIcl² reporters. Pictures were taken at 200X magnification. In this version, overall RFP signal was not adjusted via color curve after the image was captured. RFP positive cells were clearly above background level.

Many sections from AT3-T tumors co-expressed GFP and DsRED; however, large areas were observed that expressed GFP but not DsRED in all 64 sections surveyed (Figure 4B). Figure 4B shows representative example of a section from an AT3-T tumor stably transfected with GFP and pRIIIcl² reporters. Pictures were taken at 200X magnification. RNA extracted from these regions of AT3-T tumors confirmed the presence of the pRIIIcl² transcripts. Both AT3-T and AT3-M cells were plastic and produced tumors with cells that displayed a range of epithelial-mesenchymal properties.

### Example 5. AT3-T cells are motile in vitro and metastatic in vivo

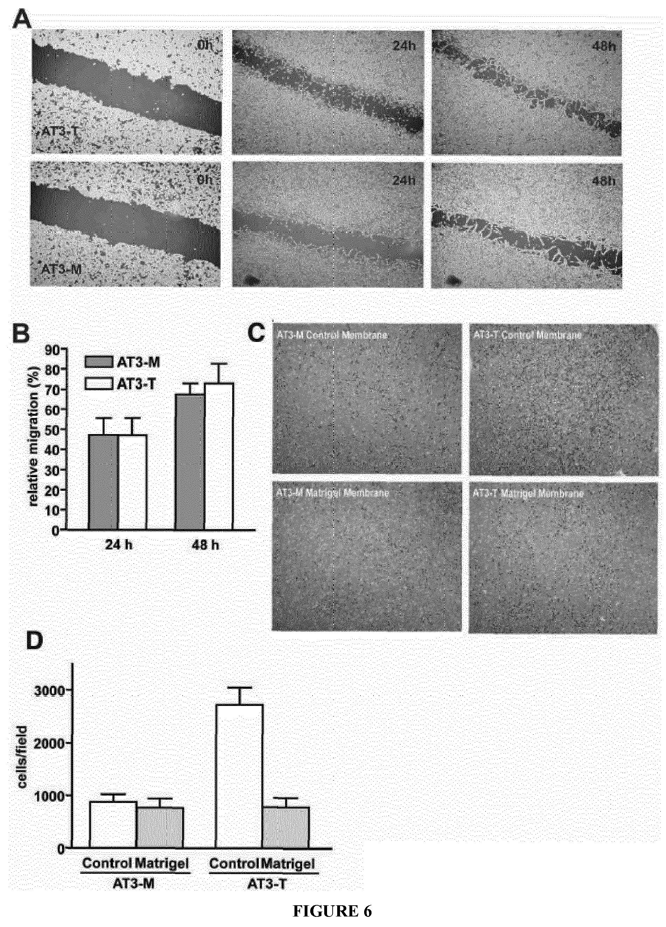

Comparison of AT3-T and AT3-M mobility and invasive potential was performed in culture. Motility was measured in culture by a "wound closure" assay, and no significant motility difference (p = 0.59) was found between cell lines 24 and 48 hours after a scratch-wound had been made in the cultures (Figure 6). Figure 6A shows representative pictures for the scratch-wound assay (experiment done in triplicate for each clone). Pictures were taken at 40X magnification. Figure 6B shows quantification of migration as explained in Methods. Mean and SO values were derived from triplicate experiments. Figure 6C shows invasion assay using Matrigel coated membranes. Representative pictures of each clone and for both control membranes and Matrigel-coated membranes (n = 5). Cells were stained with hematoxylin-eosin. Pictures were taken at 40X magnification. Figure 6D shows quantification of invasion assay results. Mean and SD values were derived from five individual experiments. To gauge invasive properties of the cells we measured the number of cells traversing through Matrigel membranes in a 22-hour period. The same number of AT3-T and AT3M cells was observed on the Matrigel membranes suggesting that the two cell lines were equally capable of invading this membrane (Figure 6). While a higher number of cells from clone AT3-T were observed on the control membrane compared to clone AT3-M, these studies nevertheless indicated that the more epithelial AT3-T cells had similar motility and invasive potential as the AT3-M cells. As shown in Figure 6, AT3-M and AT3-T cells exhibited similar migration *in vitro.*

In order to assess invasiveness *in vivo* lungs from the twenty animals harboring AT3-M and AT3-T tumors were examined for presence of metastatic foci. No macroscopic metastatic nodules were observed in any of the lungs, which was likely due to the sacrificing protocol used on the animals when the tumors reached a specified size instead of using survival as the end-point. The GFP expression from the Gint reporter was examined to evaluate the presence of micrometastases by epifluorescence microscopy. To assure a comprehensive evaluation, 7-8 equally spaced sections from each lung were surveyed (total of 150 sections for each clone). The presence of metastatic foci was determined by GFP fluorescence, followed by counter-staining of the sections with hematoxylineosin (Figure 7). Figure 7A shows (upper panel) an example of a section exhibiting the expected pattern for clone AT3-T (i.e. GFP+, DsRED+) in a metastatic focus, and (lower panel) an example of a section exhibiting a plastic pattern for clone AT3-T (i.e. GFP+, DsRED-) in a metastatic focus. Figure 7B shows (upper panel) an example of a section exhibiting the expected pattern for clone AT3-M (i.e. GFP+, DsRED-) in a metastatic focus, and (lower panel) an example of a section exhibiting a plastic pattern for clone AT3-M (i.e. GFP+, DsRED+) in a metastatic focus. As shown in Figure 7, metastatic foci in lungs from animals with tumors from either AT3Tor AT3-M clones (stably transfected with GFP and pRIIIcl² reporters) had evidence of plasticity. Metastatic foci were found in 7 out of 10 lungs for clone AT3-M and 6 out of 10 lungs for clone AT3-T.

Evaluation of the plasticity of the metastatic foci using the combined output of the GFP and DsRED reporters revealed plastic foci (DsRED+ for AT3-M and DsRED- for AT3-T) in the case of both clones: 3 out of 12 for clone AT3-T and 13 out 16 for clone AT3-M (Figure 7). These studies indicated phenotypic plasticity for the AT3-M cells and suggested it for the AT3-T cells. Importantly, both cell lines were metastatic despite differences in the original epithelial vs. mesenchymal phenotype.

*Plasticity and metastatic behavior of cancer cells.* Both the mesenchymal AT3-M and the more epithelial AT3-T cells metastasized efficiently. The drivers of metastasis, however, may be different in these two cells. The gene expression comparison between the AT3-M and AT3-T clones revealed at least one intriguing possibility: microarray analysis showed a 12-fold increase in the expression of junctional adhesion molecule C (JAM-C) in AT3-T compared to AT3-M, and this was confirmed by RT-PCR and immunoblot analysis. JAMs were present in leukocytes and at the tight junctions of epithelial and endothelial cells and have been shown to be involved in transendothelial migration of monocytes. JAM-C is expressed in several cell lines with high metastatic potential and knock-down of this molecule in the HT1080 human fibrosarcoma line significantly decreases its metastatic properties *in vivo.* Moreover, JAM-C is also present in the gene sets associated with stemness that had significant overlaps with genes that define clone AT3-T. Therefore clone AT3-T, by over-expression of different adhesion molecules may acquire metastatic capabilities. In addition, the overexpression of the downstream Hedgehog pathway effector GLI3 may be significantly upregulated in the more epithelial and stem cell-like AT3-T cells as compared to the more mesenchymal AT3-M cells. Hedgehog signaling has been linked to EMT, stemness, and metastasis/aggressiveness in several tumor types, and thus differential expression or regulation of developmental programs may underly these phenotypical differences across these cell lines. Increased expression of Patched, a Hedghog pathway component, has been linked to prostate tumors during progression to androgen independence and in circulating tumor cells of men with metastatic castration-resistant prostate cancer.

### Example 6. AT3-T cells display a stem cell-like gene expression signature

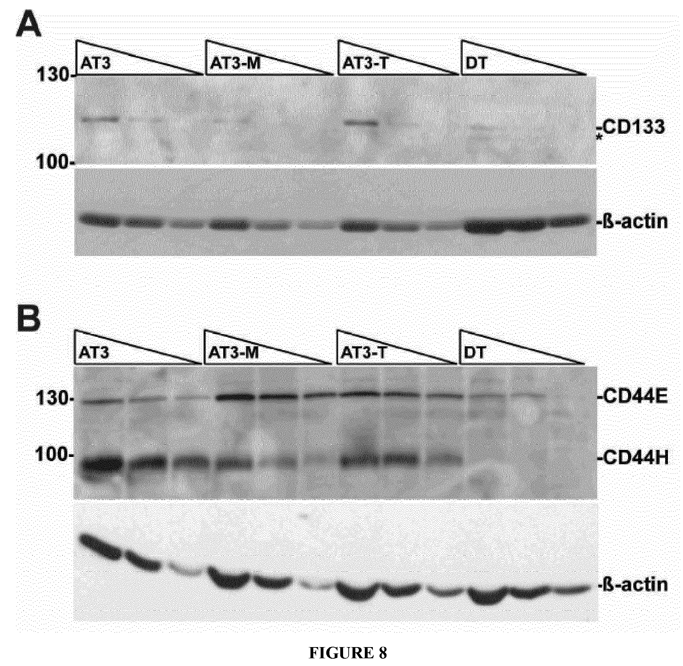

AT3-T cells sometimes formed tight clusters resembling protospheres. While sphere formation is not an exclusive property of stem cells, it has been associated with stemness in many different systems. Given these observations and the high tumorogenicity of AT3-T and AT3-M cells, they were tested for the expression of markers associated with cancer stem-like cells. Also included were the parental AT3 cells and another Dunning tumor cell line, DT cells, which display epithelial markers and are only weakly tumorogenic in Copenhagen white rats. The DT cells expressed very low levels of CD44 and CD133, which are associated with highly malignant cancer stem-like cells (Figure 8). CD 133 was detectable in DT lysates only when four fold more lysate was loaded. The mesenchymal-like AT3 cells expressed much higher levels of both CD44 and CD133 than the DT cells (note that the lanes for the DT samples are overloaded in Figure 8A), which is consistent with recent reports that EMT induces stemness in mammary epithelial carcinoma cells. Figure 8A shows a membrane with serial twofold dilutions of whole cell lysates was cut in half and immunoblotted for CD133 (upper panel) or β-actin (lower panel). Size markers are in kDa. A faster migrating CD 133 band repeatably detected only in DT lysates is marked (*), suggesting possible post-translational regulation. Figure 8B shows a membrane with serial twofold dilutions of whole cell lysates was cut in half and immunoblotted for CD44 (upper panel) or β-actin (lower panel). Representative blots from two independent sets of lysates are shown. AT3-T expressed CD44 and CD133. Interestingly, the AT3-T cells expressed overall higher levels of CD44 and CD133 than the more mesenchymal AT3-M. Moreover, AT3-T cells expressed a higher ratio of CD44H to CD44E when compared to AT3-M cells. The CD44H isoform has been associated with malignancy while CD44E is not. This suggests a more complex relationship between epithelial transitions and acquisition of stem cell-like properties. Consistent with expression of stem-like markers, both AT3-M and AT3-T cells formed colonies in soft agar and tumors when injected into Copenhagen white rats, and these tumors led to extensive metastases similar to parental AT3 cells (Figure 3B).

To further explore these connections between transitions and stemness, global gene expression in AT3-M and AT3-T cells was compared. This analysis showed that 422 genes were differentially expressed (≥ 2-fold; p-value < 0.05) in these two cells (Table 3). Many of the genes that were upregulated in AT3-T relative to AT3-M were preferentially expressed in epithelial cells and vice versa for those preferentially expressed in mesenchymal cells (Table 4). There were exceptions to this, however. Expression of the gene disintegrin-like and metalloprotease was consistent with a mesenchymal phenotype, but this mRNA level was 4-fold higher in AT3-T compared to AT3-M. Integrin β-4, normally associated with epithelial-like cells, was expressed 3-fold lower in AT3-T compared to AT3-M. These observations were consistent with the characterization of AT3-T cells as displaying more epithelial features than AT3-M cells and as populating an intermediate phenotypic state.

**Table 3.**

| **x Fold change (AT3-T/AT3-M)** | **Gene Symbol (Human)** | **Gene Symbol (Rat)** |
|---|---|---|
| 0.00771 | P2RX5 | P2rx5 |
| 0.011 | CCNB11P1 | #N/A |
| 0.0296 | STRA6 | Stra6 |
| 0.0327 | G0S2 | G0s2 |
| 0.0835 | SERPINF1 | Serpinf1 |
| 0.101 | GSTA1 | #N/A |
| 0.107 | RSNL2 | Clip4 |
| 0.115 | ADAMTS7 | #N/A |
| 0.134 | GZMB | #N/A |
| 0.137 | SPON2 | #N/A |
| 0.156 | MMP3 | #N/A |
| 0.191 | ATP8A1 | #N/A |
| 0.197 | EVPL | Evpl |
| 0.21 | LGALS3BP | Lgals3bp |
| 0.216 | SERPINB2 | Serpinb2 |
| 0.219 | NETO2 | Neto2 |
| 0.223 | PTX3 | #N/A |
| 0.23 | SERPINB7 | Serpinb7 |
| 0.233 | RASIP1 | #N/A |
| 0.235 | OMD | #N/A |
| 0.239 | HLA-G | #N/A |
| 0.239 | HLA-A | #N/A |
| 0.247 | CD97 | Cd97 |
| 0.251 | GJA4 | Gja4 |
| 0.254 | DSU | #N/A |
| 0.257 | MGLL | Mgll |
| 0.261 | SPHK1 | #N/A |
| 0.268 | HRBL | Zcwpw1 |
| 0.268 | ZCWPW1 | Zcwpw1 |
| 0.27 | ENPP3 | Enpp3 |
| 0.275 | PTGS1 | Ptgs1 |
| 0.278 | RAMP1 | Ramp1 |
| 0.281 | DHRS3 | Dhrs3 |
| 0.282 | FAM117A | Fam117a |
| 0.284 | TUBB2A///TUBB2B | Tubb2b |
| 0.284 | TUBB2B | Tubb2b |
| 0.285 | C10orf10 | LOC500300 |
| 0.289 | SYTL2 | #N/A |
| 0.291 | SLC39A4 | Slc39a4 |
| 0.292 | CHRD | Chrd |
| 0.292 | GIP | Gip |
| 0.293 | CKLF | Cklf |
| 0.294 | PLAU | Plau |
| 0.295 | GUF1 | #N/A |
| 0.307 | CGI-38 | Tppp3 |
| 0.311 | LECT2 | Lect2 |
| 0.318 | NQO2 | #N/A |
| 0.32 | C11orf75 | RGD1309410 |
| 0.324 | DOCK2 | #N/A |
| 0.325 | LGALS2 | #N/A |
| 0.326 | CASP4 | Casp1 |
| 0.326 | LTBP4 | Ltbp4 |
| 0.334 | HSPB1 | Hspb1 |
| 0.335 | ITGB4 | Itgb4 |
| 0.34 | BPHL | Bphl |
| 0.341 | FOXF2 | #N/A |
| 0.345 | MYH1 | #N/A |
| 0.345 | SMAD6 | Smad6 |
| 0.348 | TGFB1 | Tgfb1 |
| 0.351 | MMP10 | #N/A |
| 0.363 | MMP9 | Mmp9 |
| 0.363 | COL18A1 | Col18a1 |
| 0.366 | HES1 | #N/A |
| 0.369 | SLC35D2 | #N/A |
| 0.377 | ADORA2B | Adora2b |
| 0.377 | COL3A1 | Col3a1 |
| 0.379 | DPEP2 | Dpep2 |
| 0.382 | GPR153 | Gpr153 predicted |
| 0.383 | LOC55908 | #N/A |
| 0.389 | SELPLG | #N/A |
| 0.394 | P2RX1 | Atp2a3 |
| 0.394 | ATP2A3 | Atp2a3 |
| 0.394 | ADD3 | Add3 |
| 0.395 | TSPAN9 | Tspan9 |
| 0.399 | LOC54103 | #N/A |
| 0.4 | BFSP2 | #N/A |
| 0.4 | FLJ14213 | RGD1309969 |
| 0.4 | PGGT1B | Pggt1b |
| 0.401 | HCN2 | Hcn2 |
| 0.403 | C2orf33 | RGD1310230 |
| 0.404 | TMEPAI | #N/A |
| 0.405 | INHA | Inha |
| 0.406 | HPSE | #N/A |
| 0.409 | CRY1 | Cry1 |
| 0.413 | IL3RA | 113ra |
| 0.413 | CDC42EP1 | #N/A |
| 0.416 | ARG1 | Arg1 |
| 0.417 | MAPK14 | Mapk14 |
| 0.419 | FLJ22028 | #N/A |
| 0.421 | GALR2 | Galr2 |
| 0.422 | TSPAN8 | Tspan8 |
| 0.422 | FAM77C | RGD1561205 |
| 0.422 | USP2 | Usp2 |
| 0.422 | LAMA3 | #N/A |
| 0.424 | CCNE1 | Ccne1 |
| 0.424 | NSF | Nsf |
| 0.428 | ST3GAL5 | St3gal5 |
| 0.429 | SYNJ2 | Synj2 |
| 0.43 | ADA | Ada |
| 0.43 | PCBP3 | Pcbp3 |
| 0.433 | ZNF43 | #N/A |
| 0.433 | C14orf130 | Ubr7 |
| 0.436 | SOS2 | #N/A |
| 0.436 | RASSF3 | #N/A |
| 0.436 | GLMN | Glmn |
| 0.438 | OSR2 | Osr2 |
| 0.44 | AGTPBP1 | Agtpbp1 |
| 0.444 | DBNDD2 | RGD1311642 |
| 0.445 | SGCB | #N/A |
| 0.446 | HBLD2 | Iscal |
| 0.448 | SCARB1 | Scarb1 |
| 0.448 | EVI2A | Evi2a |
| 0.448 | AP4M1 | #N/A |
| 0.451 | IGF2BP3 | #N/A |
| 0.452 | FLJ10404 | Ddx41 |
| 0.454 | TGFB2 | Tgfb2 |
| 0.459 | PASK | Pask |
| 0.461 | C19orf37 | Zfp428 |
| 0.462 | BMP1 | Bmp1 |
| 0.464 | PTPN13 | Ptpn13 |
| 0.47 | PTPRG | #N/A |
| 0.47 | EFNB1 | Efnb1 |
| 0.472 | PER2 | Per2 |
| 0.472 | IRS3L /// LOC442715 | Irs3 |
| 0.472 | HRBL | Irs3 |
| 0.472 | MAP3K3 | Kcnh6 |
| 0.472 | WDR68 | Kcnh6 |
| 0.472 | KCNH6 | Kcnh6 |
| 0.472 | CCDC44 | Kcnh6 |
| 0.473 | CIB2 | Cib2 |
| 0.475 | MPZL1 | Mpzl1 |
| 0.475 | FADS2 | #N/A |
| 0.48 | ZNF185 | #N/A |
| 0.482 | SLC29A1 | Slc29a1 |
| 0.487 | RUNX3 | Runx3 |
| 0.488 | NINJ1 | Ninj1 |
| 0.489 | RASL11B | Rasl11b |
| 0.49 | ECE2 | Ece2 |
| 0.49 | TNNC2 | Tnnc2 |
| 0.491 | WASPIP | Wipf1 |
| 0.492 | FN1 | Fn1 |
| 0.494 | NDE1 | Nde1 |
| 0.494 | CAMK2G | Camk2g |
| 0.495 | CUTL1 | Cux1 |
| 0.495 | ABHD6 | Abhd6 |
| 0.495 | PTPN14 | Ptpn14 |
| 0.497 | FLJ13946 | #N/A |
| 0.498 | BAIAP2 | Baiap2 |
| 0.499 | MSL3L1 | Msl3l1 |
| 0.499 | DYNLT1 | Dynlt1 |
| 0.499 | GSTM3 | Gstm5 |
| | | |
| 2 | CHES1 | Foxn3 |
| 2.004 | AQR | Agr /// Znf770 |
| 2.006 | EPN1 | Epn1 |
| 2.011 | PPBP | Ppbp |
| 2.019 | SLC35D1 | #N/A |
| 2.022 | PTPRC | #N/A |
| 2.031 | USP47 | Usp47 |
| 2.041 | DHX29 | #N/A |
| 2.047 | HMOX1 | #N/A |
| 2.05 | CAV1 | Cav1 |
| 2.053 | BUB1B | Bub1b |
| 2.069 | KCNIP4 | #N/A |
| 2.072 | -- | #N/A |
| 2.072 | ADAM10 | #N/A |
| 2.073 | KIAA1155 | #N/A |
| 2.074 | PSTPIP2 | #N/A |
| 2.083 | MAML1 | #N/A |
| 2.084 | RAB32 | #N/A |
| 2.089 | FAM111A | #N/A |
| 2.095 | ATRNL1 | #N/A |
| 2.101 | PPIC | Ppic |
| 2.101 | CHD4 | Chd4 |
| 2.109 | IDE | Ide |
| 2.117 | PITPNM3 | #N/A |
| 2.121 | NFE2L1 | Nfe211 |
| 2.121 | MFSD1 | #N/A |
| 2.133 | KITLG | Kitlg |
| 2.161 | ING3 | Ing3 |
| 2.167 | CD24 | #N/A |
| 2.169 | IDS | #N/A |
| 2.177 | MGC3196 | LOC686289 /// LOC690285 |
| 2.185 | FBXL11 | Fbxl11 |
| 2.185 | -- | Fbxl11 |
| 2.191 | ZC3H12A | #N/A |
| 2.195 | RKHD2 | #N/A |
| 2.201 | LAMC2 | Lamc2 |
| 2.217 | KIF11 | Kif11 |
| 2.242 | SNAPC5 | Snapc5 |
| 2.252 | THRAP3 | #N/A |
| 2.261 | HS6ST1 | #N/A |
| 2.264 | OXCT1 | #N/A |
| 2.266 | TEK | #N/A |
| | HIST2H4///H4/o/// | |
| 2.268 | LOC648164 | #N/A |
| 2.271 | TMF1 | Tmf1 |
| 2.273 | ZBTB7B | Zbtb7b |
| 2.274 | CAMSAP1L1 | RGD1310950 |
| 2.279 | CYP3A5 | Cyp3ai |
| 2.279 | CYP3A7 | Cyp3a9 |
| 2.279 | CYP3A4 | Cyp3a9 |
| 2.282 | PENK | Penk1 |
| 2.283 | KIAA2010 | Smek1 |
| 2.284 | CHRNA1 | #N/A |
| 2.299 | BAT3 | Bat3 |
| 2.302 | ROM1 | Rom1 |
| 2.306 | HOXB8 | #N/A |
| 2.309 | KLK14 | #N/A |
| 2.31 | SUV39H1 | #N/A |
| | LOC440354///BOLA2/// | |
| 2.315 | LOC595101 | RGD1564579 |
| 2.315 | UBN1 | Ubn1 |
| 2.323 | C1orf103 | #N/A |
| 2.333 | EYA2 | Eya2 |
| 2.347 | MT2A | #N/A |
| 2.353 | KIAA1815 | Ermp1 |
| 2.355 | SETD1B | #N/A |
| 2.369 | MPHOSPH1 | Kif20b |
| 2.38 | EFNA1 | Efna1 |
| 2.392 | ABCF2 | Abcf2 |
| 2.397 | LIMA1 | Lima1 |
| 2.418 | EXTL3 | Extl3 |
| 2.418 | ARL6IP2 | Arl6ip2 |
| 2.442 | GRAMD3 | Gramd3 |
| 2.456 | JARID1A | Jarid1a |
| 2.476 | ARHGEF9 | Arhgef9 |
| 2.485 | CAD | Cad |
| 2.493 | RAI17 | #N/A |
| 2.526 | KIAA0284 | #N/A |
| 2.529 | SGPP1 | Sgpp1 |
| 2.531 | ABCB1 | #N/A |
| 2.531 | ABCB1///ABCB4 | #N/A |
| 2.542 | KIF1C | #N/A |
| 2.553 | KIAA0020 | LOC499339 |
| 2.563 | ADAM 15 | Adam15 |
| 2.577 | UBE1 | Uba1 |
| 2.577 | INE1 | Uba1 |
| 2.58 | GRIP2 | Grip2 |
| 2.59 | PPEF1 | #N/A |
| 2.619 | SC65 | Sc65 |
| 2.62 | FER1L3 | #N/A |
| 2.62 | NOC3L | #N/A |
| 2.62 | RBP4 | #N/A |
| 2.645 | SPINK4 | Spink4 |
| 2.653 | ATXN2L | #N/A |
| 2.711 | AHCYL1 | Ahcyl1 |
| 2.723 | TUBB3 | Tubb3 |
| 2.723 | MC1R | Tubb3 |
| 2.729 | AGPAT7 | Lpcat4 |
| 2.749 | HOXC11 | #N/A |
| 2.766 | APH1A | Aph1a |
| 2.785 | CNOT1 | RGD1308009 |
| 2.785 | CSNK2A2 | RGD1308009 |
| 2.794 | STAC | #N/A |
| 2.904 | STAG1 | #N/A |
| 2.942 | MBNL1 | #N/A |
| 2.982 | MNT | Mnt |
| 3.007 | RANBP5 | Ipo5 |
| 3.014 | HERC1 | Herc1 |
| 3.065 | ALDOC | Aldoc |
| 3.122 | KIAA0460 | - |
| 3.174 | FLT3 | #N/A |
| 3.278 | CXCL6 | Cxcl6 |
| 3.366 | GLI3 | #N/A |
| 3.489 | SSR3 | #N/A |
| 3.585 | BCAN | Bcan |
| 3.824 | FKBP10 | Fkbp10 |
| 3.903 | GSTK1 | Gstk1 |
| 3.931 | PSCDBP | #N/A |
| 3.974 | ALCAM | Alcam |
| 4.056 | ADAMTS13 | |
| 4.203 | SPRR2B | #N/A |
| 4.276 | GPR126 | #N/A |
| 5.169 | SULF1 | Sulf1 |
| 5.529 | TFF1 | Tff1 |
| 6.52 | PTN | Ptn |
| 8.591 | MLF1 | Mlf1 |
| 9.012 | THBS2 | Thbs2 |
| 10.79 | HEPH | Heph |
| 12.53 | JAM3 | Jam3 |

**Table 4. Examples of epithelial or mesenchymal genes in the expression data analysis of clones AT3-T and AT3-M.**

| **Gene name** | **x Fold change in AT3-T vs. AT3-M** |
|---|---|
| Junctional adhesion molecule C | 12.53 |
| Disintegrin-like and metalloprotease | 4.05 |
| Activated leukocyte cell adhesion molecule | 3.97 |
| Tubulin | 2.73 |
| Epithelial protein lost in neoplasm | 2.39 |
| Laminin | 2.20 |
| TGFβ2 | 0.45 |
| MMP9 | 0.36 |
| Collagen, type XVIII | 0.36 |
| MMP10 | 0.35 |
| Integrin β4 | 0.33 |
| TGFβ1 | 0.31 |
| Urokinase plasminogen activator | 0.29 |
| MMP3 | 0.15 |

Two gene sets were assembled: one composed of gene products upregulated in AT3-T (relative to AT3-M) and the second of those downregulated in AT3-T (relative to AT3-M). The two gene sets were compared for overlap with 5,452 gene sets from the Molecular Signature Database collections (Gene Set Enrichment Analysis (GSEA) http://www.broad.mit.edu/gsea/). Analysis of genes over-expressed in AT3-T relative to AT3-M for overlap with 5,452 gene sets from the Molecular Signature Database collections via Gene Set Enrichment Analysis (GSEA) did not show any significant enrichment of sets associated with EMT or MET. In this regard, both AT3-M and AT3-T resembled the mesenchymal-like, parental AT3 line. Among the 15 most significant overlaps for the genes overexpressed in AT3-T there were three sets of genes activated in hematopoetic stem cells (p = 3.24 x 10⁻⁸), neural stem cells (p = 3.07 x 10⁻⁷) and embryonal murine stem cells (p = 5.14 x 10⁻⁶), (Table 5) while among the 20 most significant overlaps for the genes that are relatively downregulated in AT3-T cells were two gene sets associated with development of mature cell types. Expression of the downstream hedgehog pathway effector GL13 was found to be 3.4-fold overexpressed in AT3-T cells compared to AT3-M cells, indicating that regulation of this developmental/stemness pathway in prostate cancer may be tied to the underlying phenotypic state during EMT/MET, similar to what has been reported in other tumors. These data indicated that AT3-T cells have gene expression profiles similar to stem cells, and, in concordance with the analysis of CD44 and CD133 protein expression, suggested that AT3-T cells exist in a more stem cell-like state than the more mesenchymal AT3-M cells.

**Table 5.**

| GSEA Collections: | C1, C3, C2, C5, C4 | | | | |
|---|---|---|---|---|---|
| # overlaps shown: | 20 | | | | |
| # gene sets in collections: | 5452 | | | | |
| # genes in comparison | 127 | | | | |
| (N) | | | | | |
| # genes in collections (N) | 39655 | | | | |
| | | | | | |
| gene set name | # genes in gene set (k) | Description | # genes in overlap (k) | k/K | p value |
| TATAAA_V$TATA_O1 | 1333 | Genes with promoter regions [- 2kb,2kb] around transcription start site containing the motif TATAAA which matches annotation for TAF<br> TATA | 20 | 0.015 | 8.07E-09 |
| STEMCELL_HEMAT OPOIETIC_UP | 1452 | Enriched in mouse hematopoietic stem cells, compared to differentiated brain and bone marrow cells | 20 | 0.0138 | 3.24E-08 |
| GNF2_RAP1B | 37 | Neighborhood of RAP1B | 5 | 0.1351 | 1.23E-07 |
| STEMCELL_NEURA L_UP | 1838 | Enriched in mouse neural stem cells, compared to differentiated brain and bone marrow cells | 21 | 0.0114 | 3.07E-07 |
| module 2 | 383 | Genes in Module 2 | 10 | 0.0261 | 4.34E-07 |
| CTTTGA_V$LEF1_Q2 | 1270 | Genes with promoter regions [-2kb,2kb] around transcription start site containing the motif CTTTGA which matches annotation for LEF1: lymphoid enhancer-binding factor 1 | 17 | 0.0134 | 5.48E-07 |
| SIGNAL_TRANSDUCTION | 1637 | Genes annotated by the GO term GO:0007165. The cascade of processes by which a signal interacts with a receptor, | 19 | 0.0116 | 9.33E-07 |
| | | causing a change in the level or activity of a second messenger or other downstream target, and ultimately effecting a change in the functioning of the cell. | | | |
| module_385 | 28 | Genes in module 385 | 4 | 0.1429 | 1.91E06 |
| V$MYCMAX_O1 | 261 | Genes with promoter regions [-2kb,2kb] around transcription start site containing the motif NNACCACGTGG TNN which matches annotation for MYC: v-myc myelocytomatosis viral oncogene homolog (avian)<br> MAX: MYC associated factor X | 8 | 0.0307 | 1.98E06 |
| GGGCGGR_V$SP1_Q6 | 3053 | Genes with promoter regions [-2kb,2kb] around transcription start site containing the motif GGGCGGR which matches annotation for SP1: Sp1 transcription factor | 26 | 0.0085 | 2.59E-06 |
| AACTTT_UNKNOWN | 1963 | Genes with promoter regions [-2kb,2kb] around transcription start site containing motif AACTTT. Motif does not match any known transcription factor | 20 | 0.0102 | 3.29E-06 |
| V$AP1_C | 281 | Genes with | 8 | 0.0285 | 3.38E-06 |
| | | promoter regions [-2kb,2kb] around transcription start site containing the motif NTGASTCAG which matches annotation for JUN: jun oncogene | | | |
| MEMBRANE_PART | 1673 | Genes annotated by the GO term GO:0044425. Any constituent part of a membrane, a double layer of lipid molecules that encloses all cells, and, in eukaryotes, many organelles; may be a single or double lipid bilayer; also includes associated proteins. | 18 | 0.0108 | 5.09E-06 |
| STEMCELL_EMBRYONIC_UP | 1344 | Enriched in mouse embryonic stem cells, compared to differentiated brain and bone marrow cells | 16 | 0.0119 | 5.14E-06 |
| INTRINSIC_TO_MEMBRANE | 1350 | Genes annotated by the GO term GO:0031224. Located in a membrane such that some covalently attached portion of the gene product, for example part of a peptide sequence or some other covalently attached moiety such as a GPI anchor, spans or is embedded in one or | 16 | 0.0119 | 5.43E-06 |
| | | both leaflets of the membrane. | | | |
| CELL_SURFACE | 79 | Genes annotated by the GO term GO:0009986. The external part of the cell wall and/or plasma membrane. | 5 | 0.0633 | 5.58E-06 |
| UVC_XPCS_8HR_DN | 408 | Down-regulated at 8 hours following treatment of XPB/CS fibroblasts with 3 J/m^2 UVC | 9 | 0.0221 | 6.35E-06 |
| NOTCH_SIGNALING_PATHWAY | 12 | Genes annotated by the GO term GO:0007219. The series of molecular signals initiated by binding of an extracellular ligand to a Notch receptor on the surface of the target cell. | 3 | 0.25 | 6.86E-06 |
| LEI_MYB_REGULATED_GENES | 325 | Myb-regulated genes | 8 | 0.0246 | 9.62E-06 |
| MORF_DDB1 | 246 | Neighborhood of DDB1 | 7 | 0.0285 | 1.40E-05 |

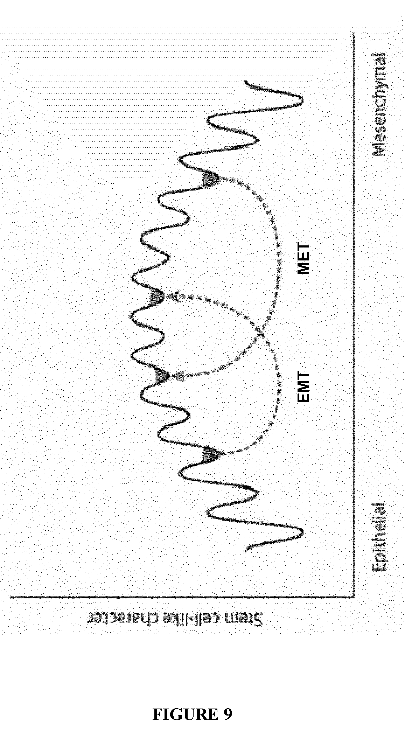

*Epithelial plasticity and stem cell-like behavior*. It is well appreciated that cells induced to undergo EMT activate stem cell pathways. Work presented here shows that AT3 cells that transitioned towards a more epithelial state, i.e. were involved in MET, also activated expression of stem cell-like markers. This finding suggested a broader relationship between plasticity and stem cell-like character or stemness, which was modeled using a Gibbs free energy diagram (Figure 9). Figure 9 shows a model comparing stem cell-like character and epithelial-mesenchymal phenotype. The x-axis represents the spectrum of epithelial to mesenchymal phenotypes and the y-axis represents the stem cell-like character of the cells. The left arrow represents an EMT and the right arrow represents an MET. The model posits that as cells transition back and forth along the epithelial and mesenchymal x-axis they course through states of varying stemness, and this property peaks at intermediate states between epithelial and mesenchymal phenotypes. The number of different states and the exact height of the barriers between states are speculative and are not meant to be taken as proportional. Two phenotypic transitions are shown, the first is a partial EMT (left arrow) and the second is a partial MET (right arrow). Both of these transitions result in states with higher stem cell-like character. It should be noted that the model also predicts that some EMTs, and equally some METs, will result in a decrease in stemness and indeed this has been observed when the highly aggressive human DKAT basal-type breast cancer cell line is induced to undergo EMT (N. D'Amato and V. Seewaldt, personal communication). The model also suggests a link between stemness, plasticity, and metastatic propensity, perhaps explained by activation of certain oncogenic pathways (e.g., PI3 kinase/Akt) and developmental pathways.

The model also predicts that cells with maximal stem-cell character, which by definition will be highly malignant, should display both epithelial and mesenchymal traits, because they inhabit intermediate states in the epithelialmesenchymal axis. The highly malignant rat adenocarcinoma AT3-T cells are in this type of state. Importantly, in humans with metastatic breast and prostate carcinomas many CTCs also exist in these intermediate states. These cells correlate with disease progression and are believed to be highly aggressive. A population of cells enriched in CTCs expressed RNAs encoding mesenchymal markers; however, the data did not indicate whether or not epithelial and mesenchymal markers were co-expressed in the same cell. Another clinical example of cells in intermediate states is found in sarcomatoid renal cell carcinomas, which have been shown to co-express epithelial markers, such as epithelial membrane antigen, and mesenchymal ones, like vimentin. These tumors, though rare (1-8% of renal tumors) are highly aggressive and difficult to treat. A similar situation may be found in carcinosarcomas of both the prostate and breast, highly aggressive, rare tumors with mixed epithelial and mesenchymal components but of clonal origin. It is not completely clear whether or not single cells in these tumor co-express epithelial and mesenchymal markers and are thus truly in intermediate states.

Finally, the model suggests that as sarcomas undergo MET they will activate stem cell-like pathways and become more aggressive. Indeed, there are many descriptions of sarcomas with mixed epithelial and mesenchymal components in close proximity as seen in some synovial- and osteo- sarcomas. New genetically-defined mouse models of soft tissue sarcoma should shed light on the existence and importance of cells intermediate cell states in progression of these tumors.

### Example 7. Phenotypic plasticity among human circulating tumor cells

The experiments described above indicated that Dunning rat prostate adenocarcinoma cells that inhabit an intermediate phenotypic state are tumorogenic, metastatic, and possess stem cell-like antigens and cellular programs. To investigate whether or not similar transitional cells could play a role in human cancer, cancer cells isolated from blood of men with metastatic castrate resistant progressive prostate cancer (CRPC) or women with progressive metastatic breast cancer (mBC) were examined. Circulating tumor cells (CTCs) represent an ideal source of tissue to investigate evidence of this plasticity *in vivo,* given that these cells are likely to be in circulation prior to and during metastatic colonization. CTCs have both independent prognostic and predictive significance in multiple epithelial malignancies, including breast and prostate cancer. These cells can be collected, isolated, and analyzed for a variety of biomarkers relevant to cancer biology.

It was tested whether there was a high likelihood of finding transitional cells within a population of CTCs captured by FDA-approved EpCAM (Epithelial Cell Adhesion Molecule)-targeted ferromagnetic antibodies. These cells were interrogated for expression of CD45 (expressed in many leukocytes; Figure 10A), cytokeratin (CK; an epithelial marker), and vimentin (a mesenchymal marker) by immunofluorescence. CTCs were defined as CD45-negative and CK-positive nucleated intact cells (Figure 10B) and transitional CTCs were so defined if they additionally co-expressed vimentin (Figure 10C-D). Figure 10 shows that CTCs from patients with prostate adenocarcinoma stained positive for epithelial and mesenchymal markers. Triple staining was performed using anti-CD45 antibody labeled with Alexa 647, anti-cytokeratin (CK) antibody labeled with Alexa 555, and anti-vimentin antibody labeled with Alexa 488. Nuclei were labeled with DAPI. Figure 10A shows an example of a leukocyte from a human peripheral blood mononuclear cell sample: CD45 (+), CK (-), and vimentin (+). Additionally, CD45 (+), CK (-), and vimentin (-) cells were observed. Figure 10B shows an example of a CD45 (-), CK (+), and vimentin (-) cell from a patient with metastatic breast cancer. Such cells were counted as vimentin (-) CTCs in Table 6. Figure 10C shows an example of a CD45 (-), CK (+), vimentin (+) from a patient with metastatic breast cancer. Such cells were counted as vimentin (+) CTCs in Table 6. Figure 10D shows an example of a CD45 (-), CK (+), vimentin (+) from a patient with metastatic progressive castrate-resistant prostate cancer. Such cells were counted as vimentin (+) CTCs in Table 6.

Transitional CTCs co-expressed vimentin and CK in many of the patients with elevated CTC counts (≥5 CTCs/7.5 mL by standard testing) (Table 6, Figure 10). In fact, among nine patients with progressive metastatic CRPC and eight patients with progressive mBC, it was found that approximately 75% (range 0-100%, 85.5% in CRPC, 54% in mBC) of the CTCs stained for both CK and vimentin (Figure 10C-D), indicating a transitional phenotype. These data indicated that circulating tumor cells in patients with metastatic breast and prostate cancer co-express epithelial (EpCAM and cytokeratin) and mesenchymal (vimentin) markers, and thus exist in a transitional phenotypic state, similar to that observed in our preclinical models.

**Table 6. Circulating tumor cell (CTC) counts and vimentin expression in patients with metastatic castration resistant prostate or metastatic breast cancer.**

| Subject Number | CTC Count (Cellsearch)* | Ratio: vimentin (+) CTCs/Total CTC Count |
|---|---|---|
| Castrate-Resistance Metastatic Prostates Cancer | | |
| 1 | 5 | 4/6 |
| 2 | 41 | 11/11 |
| 3 | 45 | 6/10 |
| 4 | 626 | 5/8 |
| 5 | 110 | 17/21 |
| 6 | 182 | 5/6 |
| 7 | 17 | 13/16 |
| 8 | 19 | 33/34 |
| 9 | 34 | 12/12 |
| Total | | 106/124 (85.5%) |
| Metastatic Breast Cancer | | |
| 1 | 21 | 0/6 |
| 2 | 7 | 2/2 |
| 3 | 8 | 4/4 |
| 4 | 21 | 1/2 |
| 5 | 12 | 2/2 |
| 6 | 188 | 21/22 |
| 7 | 138 | 8/20 |
| 8 | 377 | 6/23 |
| Total | | 44/81 (54.3%) |
| Overall Total | -- | 150/205 (73.1%) |

| | | |
|---|---|---|
| *Column 2 represents the CTC count as determined by the standard Cellsearch EpCAM based method for each subject, while column 3 represents the number and proportion of CTCs counted manually that were found to express cytokeratin and co-express vimentin, expressed as a ratio and percentage. | | |

*Plasticity and CTCs*. The identification of plasticity among CTCs in a significant subset of patient samples offers several important clinical opportunities. Expression of plasticity may have prognostic or predictive value in patients with metastatic cancers, especially mBC where a significant range of values were shown for plasticity. Thus, the subset of patients with very high plasticity may have a more aggressive natural history and exhibit greater resistance to systemic treatments. In terms of diagnosis and utility as predictive biomarkers the data suggested that in addition to cells expressing both epithelial and mesenchymal markers there may be an unknown number of CTCs that have moved further towards the mesenchymal pole and are EpCAM negative. These cells will be missed by the FDA approved CellSearch® System and also by the Adna Test (AdnaGen AG) system and current microfluidic technologies, which enrich for CTCs by immunoabsorbtion of cells expressing MUC1 or EpCAM. Indeed, recent studies in breast cancer have suggested that "normal" type breast cancer cell lines that overexpress both EMT and stem cell antigens (CD44+, CD24-) may lack EpCAM and are thus not detectable by currently approved CTC detection systems. Therefore it is possible that the number of CTCs in patients with metastatic cancer is much higher than currently appreciated. Identification of this additional subset of CTC can provide greater prognostic value than CTC counts as currently determined, as well as earlier detection of CTCs and the metastatic potential in patients with earlier stage disease.

Furthermore, CTCs in intermediate states, which comprise the 50-75% of cells isolated herein from patients with metastatic breast and prostate cancer as well as those cells that may go undetected because they have undergone a more complete EMT, represent a therapeutic problem. It has been well documented that EMT alters drug sensitivity of lung cancer cells and it has been challenging to direct therapy to cancer cells with stem cell-like properties, perhaps because of their recalcitrance to undergo apoptosis.

While recent studies suggest both a screening method and actual compounds (e.g., salinomycin) that can selectively target cancer stem cells, these aggressive cells still represent a formidable therapeutic challenge. Thus, molecules comprising a binding agent that has binding specificity to an EMT biomarker described herein and linked to an anti-cancer agent provide additional therapeutic options.

### Example 8. CTCs from patients with metastatic breast and prostate cancer express vimentin and N-cadherin

Eligible men had progressive metastatic CRPC (progression despite testosterone < 50 ng/dL) and were about to begin a new systemic therapy. Eligible women had progressive metastatic breast cancer (mBC) and were about to begin a new systemic therapy. Baseline characteristics of patients (n = 29) are presented in Table 7.

**Table 7. Baseline characteristics of patients (n = 29)**

| | Metastatic Prostate (n = 17) | Metastatic Breast (n=12) |
|---|---|---|
| DEMOGRAPHICS | | |
| Age, median | 69 (59-82) | 61.5 (48-81) |
| Race, Ethnicity | | |
| White, non-hispanic | 76% | 58% |
| Other, non-hispanic | 23 % | 42% |

| BASELINE DISEASE HISTORY | | |
|---|---|---|
| Gleason Score, median | 7 (7-9) | --- |
| ER/PR, % | --- | 75% / 67% |
| Baseline median PSA, Range | 396.4 (14-13,419.5) | --- |
| Baseline Pain Score (0-10), median | 1 (0-7) | 0 (0-6) |
| Karnofsky Performance Status, median | 90 (70-100) | 90 (70-100) (n=6) |
| # of Prior Hormonal Therapies | 2 (0-5) | 2 (0-4) |
| Prior Chemotherapy | 47% | 83% |
| Baseline CTC Count, median | 40 (4-828) | 13 (0-1062) |

| METASTATIC SITES | | |
|---|---|---|
| Lymph Node | 65% | 50% |
| Liver | 24% | 50% |
| Lung | 47% | 42% |
| Bone | 94% | 75% |

CTCs were drawn into standard FDA-approved Cellsave tubes and processed within 48 hours using the CellSearch® methodology using EpCAM-based ferromagnetic capture. A CTC was defined as an intact nucleated (DAPI+) cell that expressed pan-CK and lacked expression of the leukocyte antigen CD45, and was enumerated using standard methods. A second Cellsearch® tube was collected and processed using EpCAM capture, and isolated cells were stained for CK (IgG1, AbD Serotec) labeled with Alexa 555, CD45 (IgG1, AbCam) labeled with Alexa 647, and either vimentin (IgG1, BD Biosciences) or N-Cadherin (IgG1, DAKO) using immunoflouresent labeling with Alexa 488. The proportion of CTCs staining positive for an EMT antigen was calculated from the total number of CTCs manually scored from the second tube. Positive controls using American Red Cross-derived PBMCs (CD45), PC3 prostate cancer cells (vimentin, N-cadherin), and T47D breast cancer cells (CK) were used for each marker. Negative controls using mock antibody were used to optimize the staining/scoring of each antigen.

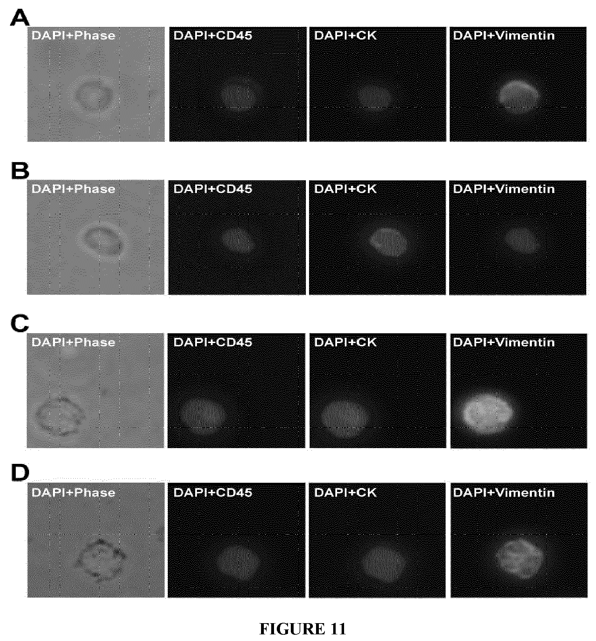

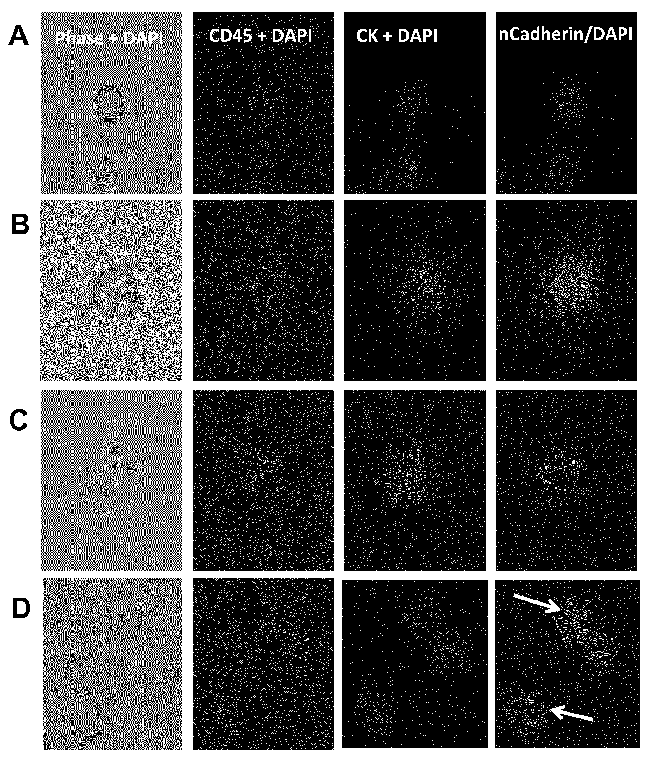

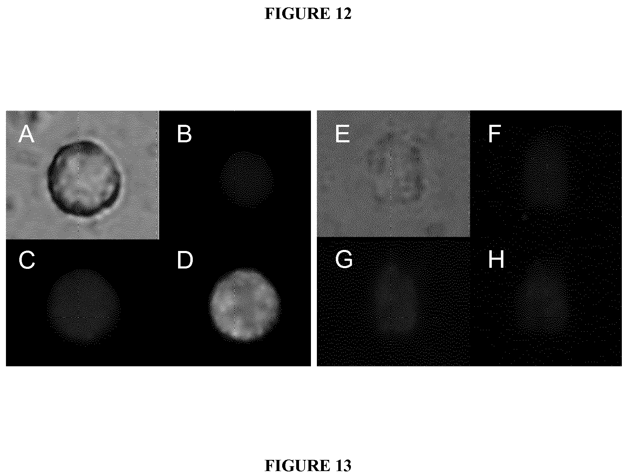

Prevalence of vimentin and CK co-expression in CTCs, and prevalence of N-cadherin and CK co-expression in CTCs are presented in Tables 8 and 9, respectively. Vimentin co-expression was detected in 17/20 (85%) patients with mCRPC or mBC and 78% of all CTCs. N-Cadherin co-expression was detected in 8/9 (89%) patients and 81% of CTCs. Immunofluorescent images of CTCs from patients with mCRPC and mBC are shown in Figure 11 (A, a leukocyte; B, vimentin negative CTC (CRPC); C, vimentin positive CTC (BC); and D) vimentin positive CTC (CRPC)). Immunofluorescent images of CTCs from patients with mCRPC and mBC are shown in Figure 12 (A, leukocyte; B, Ncad positive CTC (BC); C, Ncad negative CTC (BC); and D, two NCad positive CTCs (arrows) and 1 Ncad negative CTC (CRPC)). Immunofluorescent images of CTCs from patients with mCRPC and mBC are shown in Figure 13 (A, Phase/DAPI; B, CD45/DAPI; C, CK/DAPI; D, Vimentin/DAPI positivity in a man with mCRPC; E, Phase/DAPI; F, CD45/DAPI; G, CK/DAPI; and H, Vimentin/DAPI negativity in a second man with mCRPC).

The data showed the co-expression of cytokeratin with the EMT antigens vimentin and N-cadherin in CTCs from men with metatastic CRPC and women with metastatic breast cancer. A majority of CTCs examined co-expressed CK and EMT proteins by immunofluorescent labeling. The majority of patients in this study had CTCs that co-expressed vimentin or N-cadherin suggesting potential epithelial plasticity during metastasis. The data suggests that CTCs can lack epithelial markers and provide methods for assessing patients with breast and prostate cancer as well as for the optimal detection of circulating tumor cells in other common malignancies.

**Table 8.**

| **Subject Number** | | **CTC Count (Cellsearch)** | **Ratio of: Vimentin (+) CTCs / Total Manual CTC Count** |
|---|---|---|---|
| | 1 | 5 | 4/6 |
| | 2 | 4 | 2/2 |
| | 3 | 54 | 11/11 |
| castrate-resistant metastatic prostate cancer | 4 | 45 | 6/10 |
| | 5 | 626 | 5/8 |
| | 6 | 110 | 17/21 |
| | 7 | 182 | 5/6 |
| | 8 | 17* | 13/16 |
| | 9 | 19 | 33/34 |
| | 10 | 34 | 12/12 |
| Total | | 1127 | 108/126 (86%) |
| | 1 | 13 | 0/6 |
| | 2 | 85 | 2/2 |
| | 3 | 8 | 4/4 |
| | 4 | 21 | 1/2 |
| metastatic breast cancer | 5 | 12 | 2/2 |
| | 6 | 188 | 21/22 |
| | 7 | 324** | 29/33 |
| | 8 | 377 | 6/23 |
| | 9 | 0 | 0/0 |
| | 10 | 3 | 0/3 |
| Total | | 884 | 65/97 (67%) |
| Overall Total | | -- | 173/223 (78%) |

**Table 9.**

| **Subject Number** | | **CTC Count (Cellsearch)** | **Ratio of: N-Cadherin (+) CTCs / Total Manual CTC Count** |
|---|---|---|---|
| | 1 | 45 | 13/19 |
| | 2 | 12 | 5/7 |
| castrate-resistant metastatic prostate cancer | 3 | 10 | 8/8 |
| | 4 | 5 | 8/9 |
| | 5 | 12 | 4/4 |
| | 6 | 221 | 11/13 |
| | 7 | 828 | 81/96 |
| Total | | 1132 | 130/156 (83%) |
| metastatic breast cancer | 1 | 1062 | 9/13 |
| | 2 | 2 | 0/3 |
| Total | | 1064 | 9/16 (56%) |
| Overall Total | | -- | 139/172 (81%) |

| | | | |
|---|---|---|---|
| *Count from 3 months prior to baseline (no intervening therapy) **Count from time point #2 | | | |

In a second trial to test for the existence of transitional CTCs, blood was collected from 31 men with mCRPC and 16 women with mBC (see baseline characteristics for the patients in Table 10 and Table 11). CTCs were processed using the CellSearch® EpCAM-based immunocapture method and profiled for expression of CD45 (PTPRC) (a leukocyte marker), cytokeratins (CK) (epithelial markers), vimentin (VIM) and N-cadherin (CDH2) (mesenchymal markers), and CD133 (a stem cell marker) by immunofluorescence (IF) (Table 2). Leukocytes were defined as nucleated (DAPI positive), CD45-positive and CK-negative cells, whereas CTCs were defined as nucleated (DAPI positive), CD45-negative and CK-positive cells. Among CTCs we identified transitional cells as those that additionally expressed vimentin or N-cadherin.

**Table 10. Baseline demographic and clinical characteristics of the men with metastatic CPRC.**

| **DEMOGRAPHICS** | n = 31 |
|---|---|
| Age, years (range) | 71 (59-89) |
| Race, Ethnicity | |
| White, non-Hispanic | 71 % |
| Black, non-Hispanic | 29 % |

| **BASELINE DISEASE HISTORY** | |
|---|---|
| Median Gleason Score (range) | 8 (5-10) |
| Median Baseline PSA¹ (ng/dl, range) | 267.5 (14.0-13,419.5) |
| Median Baseline Pain (range)² | 1 (0-7) |
| Median Karnofsky Performance Status (range) | 90 (60-100) |
| Median Number of Prior Hormonal Therapies (range) | 3 (0-5) |
| Prior Chemotherapy | 65 % |
| Prior Bisphosphonates | 71 % |

| **SITES OF METASTATIC DISEASE** | |
|---|---|
| Visceral (lung + liver) | 35% |
| Lymph Node Only | 0% |
| Bone metastatic: | |
| Bone Metastatic With Lymph Nodes (no visceral metastases) | 39% |
| Bone Metastatic Without Lymph Nodes (no visceral metastases) | 26% |

| | |
|---|---|
| ¹ PSA: prostate specific antigen. ² Pain is scored as a linear analog scale (0-10 range). | |

**Table 11. Baseline characteristics of mBC patients.**

| **DEMOGRAPHICS** | n = 16 |
|---|---|
| Median age (range) | 61(48-81) |
| Race, Ethnicity | |
| White, non-Hispanic | 44% |
| Black, non-Hispanic | 50% |
| Asian, non-hispanic | 6% |
| **BASELINE DISEASE HISTORY** | |
| ER and/or PR positive disease | 56% |
| HER2 positive disease (HER2 3+) | 0% |
| Median Karnofsky Performance Status (range) | 90 (70-90) |
| Median Number of Prior EndocrineTherapies (range) | 1 (0-4) |
| Median Number of Prior Chemotherapies | 2 (0-7) |

| **SITES OF METASTATIC DISEASE** | |
|---|---|
| Visceral (lung or liver) | 75% |
| Lymph Node Only | 0% |
| Lymph Node, soft tissue, or contralateral breast only | 13% |
| Bone metastases only: | |
| Bone Metastatic With Lymph Nodes (no visceral metastases) | 0% |
| Bone Metastatic Without Lymph Nodes (no visceral metastases) | 13% |

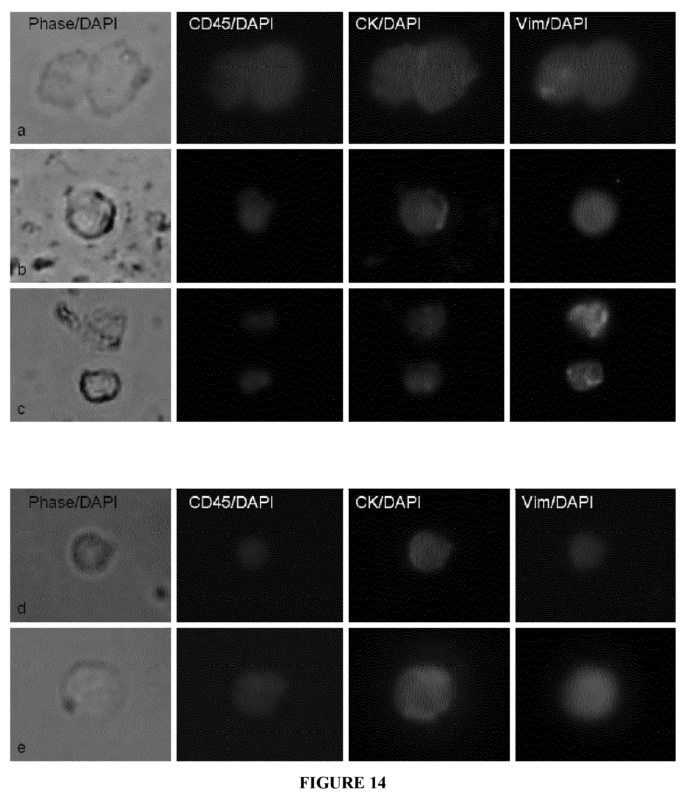

Among ten men with mCRPC, CTCs co-expressed vimentin and CK in 10/10 (100%) patients, and by this criterion 108/126 (86%) of enumerated CTCs were transitional (Table 12, Figure 14). Biopsies of bony metastases performed within one week of CTC collection in two of these patients revealed no vimentin expression in the CK positive tumor foci, but strong vimentin expression in the surrounding bone stroma, which lacks CK expression. These same patients had CTCs taken at the same time as the CT-guided tumor biopsy that commonly expressed co-expressed CK and vimentin. These findings are consistent with invasion and metastasis by transitional CTCs that subsequently undergo MET; alternatively, vimentin expression may be heterogeneously expressed in metastases, similar to CTC expression.

**Table 12. Circulating tumor cell (CTC) and transitional CTCs in patients with metastatic CRPC.**

| Subject Number | CTC Count (Cellsearch)ⁱ | Ratio: Vimentin (+) CTCs / Total Manual CTC Countⁱⁱ |
|---|---|---|
| 1 | 5 | 4/6 |
| 2 | 4 | 2/2 |
| 3 | 54 | 11/11 |
| 4 | 45 | 6/10 |
| 5 | 626 | 5/8 |
| 6 | 110 | 17/21 |
| 7 | 182 | 5/6 |
| 8 | 17 | 13/16 |
| 9 | 19 | 33/34 |
| 10 | 34 | 12/12 |
| Total | 1127 | 108/126 (86%) |

| Subject Number | CTC Count (Cellsearch) | Ratio: N-Cadherin (+) CTCs/ Total Manual CTC Count |
|---|---|---|
| 11 | 45 | 13/19 |
| 12 | 12 | 5/7 |
| 13 | 10 | 8/8 |
| 14 | 5 | 7/8 |
| 15 | 12 | 3/4 |
| 16 | 220 | 11/13 |
| 17 | 828 | 81/96 |
| 18 | 26 | 6/11 |
| 19 | 12 | 18/22 |
| 20 | 42 | 15/18 |
| Total | 1224 | 167/206 (81%) |

| Subject Number | CTC Count (Cellsearch) | Ratio: CD133 (+) CTCs/ Total Manual CTC Count |
|---|---|---|
| 21 | 485 | 38/38 |
| 22 | 16 | 6/11 |
| 23 | 91 | 15/21 |
| 24 | 6 | 0/0 |
| 25 | 36 | 29/29 |
| 26 | 27 | 9/9 |
| 27 | 43 | 10/15 |
| 28 | 2 | 0/0 |
| 29 | 23 | 12/14 |
| 30 | 38 | 23/26 |
| 31 | 30 | 12/17 |
| Total | 797 | 154/180 (86%) |

| | | |
|---|---|---|
| i The middle column represents the CTC Count from the FDA-approved Cellsearch® enumeration of CTCs for each subject. ii Right column represents the ratio of vimentin (co-expression of vimentin ranged from 60-100% of cells in a given individual and did not correlate with CTC count (R²=0.11)), N-cadherin (Co-expression of N-cadherin ranged from 55-100% of cells in a given individual, and did not correlate with CTC count (R²=-0.09)), or CD133 (CD133 co-expression ranged from 55-100% of evaluable cells in a given individual and did not correlate with CTC number (R²=0.04)) expressing CTCs among the total number of CTCs that were manually enumerated. A CTC was defined as an intact DAPI positive (nucleated) cell that lacked CD45 expression and expressed cytokeratin. | | |

**Table 13. CTCs and transitional CTCs in patients with mBC.**

| Subject Number | CTC Count (Cellsearch)i | Ratio: Vimentin (+) CTCs / Total Manual CTC Countii |
|---|---|---|
| 1 | 21 | 0/6 |
| 2 | 7 | 2/2 |
| 3 | 8 | 4/4 |
| 4 | 21 | 1/2 |
| 5 | 12 | 2/2 |
| 6 | 188 | 21/22 |
| 7 | 324 | 29/33 |
| 8 | 377 | 6/23 |
| 9 | 0 | 0/0 |
| 10 | 3 | 0/3 |
| Total | 961 | 65/97 (67%) |

| Subject Number | CTC Count (Cellsearch) | Ratio: N-Cadherin (+) CTCs/ Total Manual CTC Count |
|---|---|---|
| 11 | 1062 | 9/13 |
| 12 | 2 | 0/3 |
| 13 | 147 | 52/59 |
| 14 | 6 | 2/5 |
| 15 | 33 | 15/15 |
| 16 | 2 | 0/0 |
| Total | 1252 | 78/95 (82%) |

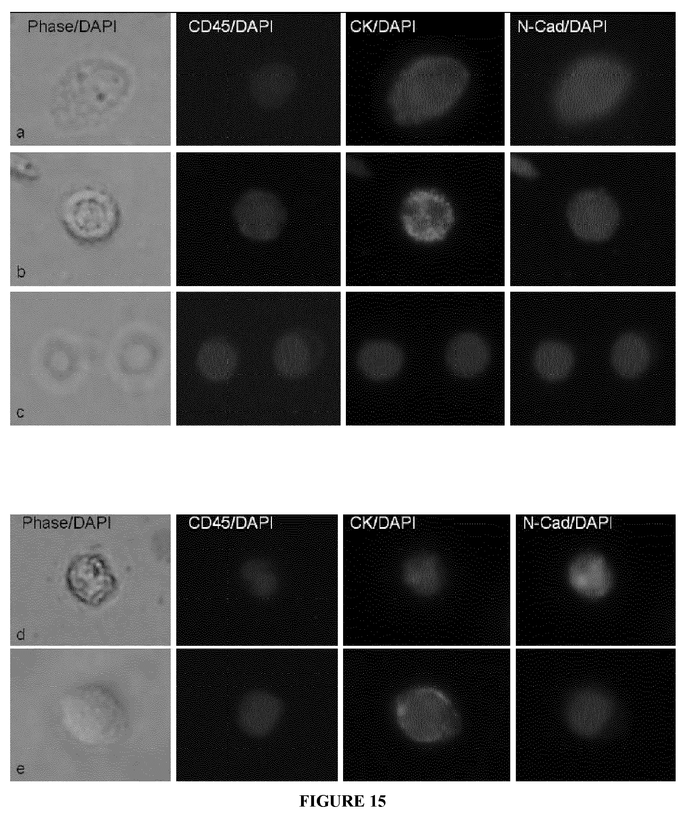

Among the next cohort of 10 men with mCRPC, CTCs co-expressed N-cadherin and CK in 10/10 (100%) patients, and by this criterion 167/206 (81%) of CTCs were identified as transitional (Table 12, Figure 15). Among 10 women with mBC, nine had detectable CTCs and of these, we found evidence of vimentin co-expression in seven (78%) patients, and 55/88 CTCs overall (63%) co-expressed vimentin (Table 13, Figure 14). Among another six women with detectable CTCs and mBC, four had evidence of CK and N-cadherin co-expression, and overall 78/95 CTCs (82%) had N-cadherin expression, with significant heterogeneity in expression in a given individual (Table 13, Figure 15). These data indicate that many CTCs in patients with mBC and mCRPC co-express epithelial (EpCAM and cytokeratin) and mesenchymal (vimentin, N-cadherin) markers, and thus exist in a transitional phenotypic state, similar to that observed in our preclinical models.

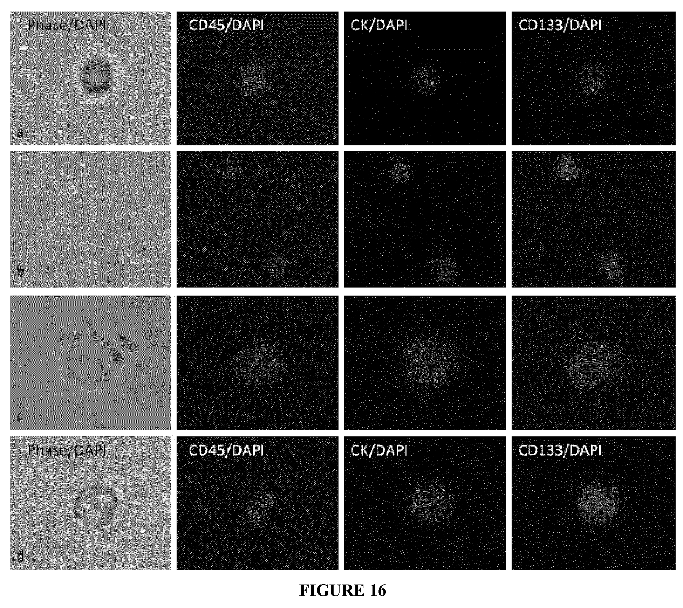

Given the expression of the stem cell associated antigen CD133 in transitional AT3-T cells, CD133 expression in CTCs from men with mCRPC was evaluated. CD133 was expressed in 11/11 (100%) men with CTCs, and in 154/180 (86%) of CTCs from these men (Table 12, Figure 16). These data suggest that CTCs from patients with common epithelial malignancies inhabit transitional states characterized by co-expression of epithelial and mesenchymal markers as well as CD133, biomarkers that have been associated with stem-like properties, invasiveness, and chemoresistance.

SEQUENCES

SEQ ID NO: 1

N-cadherin (also known as cadherin-2, cdh2)

From Mus musculus

Gene No. 12558, Accession No. AB008811

nucleotide (mRNA), 4321 bp

1 cacacacaca cgcacacaca cacacacaca cacttctcgg cgcgcacgac gcccgccctt
61 ctccccgccc cctccccagc tccttgatct cccgtctgtt ttattactcc tggtgcgagt
121 ccggcggact ccgaggcccg ctatttgtta ccaactcgct ctcattggcg gggaggagag
181 cagcggagaa gggggtgggg aggggagggg aagggaaggg gtggccactg ccggagccga
241 ctccgcgctg ctgttggtgc cgctgccgct tctgctgcct ctgctgccgc cgccgccgcc
301 tccggctcct cgctcggccc ctctccgcct ccatgtgccg gatagcggga gcgccgcgga
361 ccctgctgcc gcttctggcg gccttgcttc aggcgtctgt ggaggcttct ggtgaaattg
421 cattatgcaa gactggattt cctgaagatg tttacagcgc agtcttaccg aaggatgtgc
481 acgaaggaca gccccttctc aatgtgaaat tcagcaactg caatagaaaa aggaaagttc
541 agtatgaaag cagcgagcca gcagatttca aggtggacga ggacggcacg gtgtatgctg
601 tgagaagctt ccctctcact gcagagcagg caaagttcct gatatatgcc caagacaaag
661 aaacccagga aaagtggcag gtagctgtaa acctgagccg ggagccaacc ctgactgagg
721 agcctatgaa ggaaccacat gaaattgaag aaatagtatt ccctagacaa cttgccaagc
781 acagtggagc tctacaaagg cagaagagag actgggtcat cccgccaatc aacttgccag
841 aaaactccag aggacccttt cctcaagagc ttgtcagaat caggtctgat agagataaaa
901 acctttccct gagatacagc gtcactgggc caggagctga ccagcctcca acgggcatct
961 tcattatcaa ccccatctca ggacagctgt cagtcacaaa gcctctggat cgagagctga
1021 tagcccggtt tcacttgaga gcacatgcag tggacatcaa tggcaatcaa gtggagaacc
1081 ccattgacat tgtcatcaat gttattgaca tgaatgataa cagacctgag tttctgcacc
1141 aggtttggaa tgggtctgtt ccagagggat caaagcctgg gacgtatgtg atgacggtca
1201 ctgccattga tgcggatgat ccaaatgccc tgaatggaat gctgcggtac aggatcctgt
1261 cccaggcgcc cagcacacct tcacccaaca tgtttacaat caacaatgag actggggaca
1321 tcatcactgt ggcagctggt ctggatcgag agaaagtgca acagtatacg ttaataattc
1381 aagccacaga catggaaggc aatcccactt atggcctttc aaacacagcc acagccgtca
1441 tcacggtgac agatgtcaat gacaatcctc cagagtttac tgccatgact ttctacggag
1501 aagtccctga gaacagggtg gacgtcattg tagccaacct aactgtcacg gacaaagatc
1561 agccccacac gccggcctgg aatgcggcat acagaatcag tggtggagac cctacaggaa
1621 ggtttgccat cctgacagac cccaacagca atgatgggct agtcacagtg gtaaaaccaa
1681 ttgactttga aacgaatagg atgtttgtcc ttactgttgc tgcagaaaac caagtgccat
1741 tagctaaagg cattcagcac ccacctcagt cgacagccac tgtgtctgtg acagttattg
1801 atgtcaatga aaatccttat tttgccccaa atcctaaaat cattcgccaa gaggaaggcc
1861 tccacgcagg taccatgctg accacgctca ctgctcagga ccccgatcga tatatgcaac

1921 agaatatcag atacacaaaa ttgtctgatc ctgccaactg gctgaaaata gaccccgtga
1981 atgggcagat cactactatt gccgttttgg acagagaatc gccaaatgta aaaaacaaca
2041 tctataatgc taccttcctt gcttctgaca atggaatccc gcctatgagt gggacaggaa
2101 cactgcaaat ctatttactt gatatcaatg acaacgcccc tcaggtgtta cctcaagagg
2161 cggagacctg tgaaactcca gaacccaact caattaacat cacagcactt gattatgaca
2221 tagacccaaa cgccgggccg ttcgcgtttg atcttccctt atctccagtg actattaaaa
2281 gaaactggac catcaaccgg cttaatggtg attttgctca gctcaattta aagataaaat
2341 ttttggaagc tggtatctat gaagttccca tcattatcac agattcaggg aatcccccca
2401 agtccaacat ttccatcctg cgtgtgaaag tttgtcagtg tgactccaat ggagactgca
2461 cggacgtgga caggatcgtg ggtgcagggc ttggcacggg cgccatcatc gctatccttc
2521 tgtgtatcat catcctgctg atccttgttc tcatgtttgt ggtatggatg aaacggcggg
2581 ataaagagcg ccaagccaag cagcttttaa ttgacccaga agatgatgta agagataata
2641 tattgaaata tgatgaagaa ggtggaggag aagaagacca ggactatgac ttgagccagc
2701 tccagcaacc agatactgtg gagcctgatg ccatcaagcc cgtgggaatc agacggctag
2761 acgagaggcc tatccatgct gagccacagt acccagtccg atccgcagcc ccacaccctg
2821 gggatattgg ggacttcatt aatgagggcc ttaaagctgc tgacaacgac cccacggcgc
2881 caccgtatga ctccctctta gtctttgact acgagggcag cggctccacg gctggctcct
2941 tgagctccct caactcctcc agtagcggtg gggaccagga ctatgactac ctgaatgact
3001 ggggaccccg cttcaagaaa ctggcggaca tgtacggcgg tggtgacgac tgaacggcag
3061 gacggacttg gcttttggac aagtatgaac agtttcacct gatattccca aaaaaaagca
3121 tacagaagct aggctttaac tctgtagtcc actagcaccg tgcttgctgg aggctttggc
3181 gtaggctgcg aaccagtttg ggctcccagg gaatatcagt gatccaatac tgtctggaaa
3241 acaccgagct cagctacact tgaattttac agtaaagaag cactgggatt tatgtgcctt
3301 tttgtacctt tttcagattg gaattagttt tctgtttaag gctttaatgg tactgatttc
3361 tgaaatgata aggaaaagac aaaatatttt gtggcgggag cagaaagtta aatgtgatac
3421 gcttcaaccc acttttgtta caatgcattt gcttttgtta agatacagaa cgaaacaacc
3481 agattaaaaa aaattaactc atggagtgat tttgttacct ttgggtgggg ggggatgaga
3541 ccacaagata ggaaaatgta cattacttct agttttagac tttagatttt tttttttcac
3601 taaaatctta aaacttacgc agctggttgc agataaaggg agttttcata tcaccaattt
3661 gtagcaaaat gaattttttc ataaactaga atgttagaca cattttggtc ttaatccatg
3721 tacacttttt tattttctgt attttttcca cctcgctgta aaaatggtgt gtgtacataa
3781 tgtttatcag catagactat ggaggagtgc agagaactcg gaacatgtgt atgtattatt
3841 tggactttgg attcaggttt tttgcatgtt aatatctttc gttatgggta aagtatttac
3901 aaaacaaagt gacatttgat tcaactgttg agctgtagtt agaatactca atttttaatt
3961 ttttaatttt ttttaaattt ttttattttc tttttgtttg tttcgttttg gggagggta
4021 aaagttctta gcacaatgtt ttacataatt tgtaccaaaa aaattacaca caaaaaaaaa
4081 aaaaagaaaa gaaaagaaaa gtgaaagggg tggcctgttt cttgcagcac tagcaagtgt
4141 gtgtttttaa aaaacaaaac aaacaaacaa aaaaataaat aaaaagagga aaaagaaaaa
4201 aaaaaaagct tttaaactgg agagacttct gaaacagctt tgcgtctgtg ttgtgtacca
4261 gaatacaaac aatacacctc tgaccccagc gttctgaata aaaagctaat tttggatctg

4321 g

SEQ ID NO: 2
N-cadherin (also known as cadherin-2, cdh2)
From Mus musculus
Gene No. 12558, Accession No. AB008811
polypeptide, translation of SEQ ID NO: 1
MCRIAGAPRTLLPLLAALLQASVEASGEIALCKTGFPEDVYSAV
LPKDVHEGQPLLNVKFSNCNRKRKVQYESSEPADFKVDEDGTVYAVRSFPLTAEQAKF
LIYAQDKETQEKWQVAVNLSREPTLTEEPMKEPHEIEEIVFPRQLAKHSGALQRQKRD
WVIPPINLPENSRGPFPQELVRIRSDRDKNLSLRYSVTGPGADQPPTGIFIINPISGQ
LSVTKPLDRELIARFHLRAHAVDINGNQVENPIDIVINVIDMNDNRPEFLHQVWNGSV
PEGSKPGTYVMTVTAIDADDPNALNGMLRYRILSQAPSTPSPNMFTINNETGDIITVA
AGLDREKVQQYTLIIQATDMEGNPTYGLSNTATAVITVTDVNDNPPEFTAMTFYGEVP
ENRVDVIVANLTVTDKDQPHTPAWNAAYRISGGDPTGRFAILTDPNSNDGLVTVVKPI
DFETNRMFVLTVAAENQVPLAKGIQHPPQSTATVSVTVIDVNENPYFAPNPKIIRQEE
GLHAGTMLTTLTAQDPDRYMQQNIRYTKLSDPANWLKIDPVNGQITTIAVLDRESPNV
KNNIYNATFLASDNGIPPMSGTGTLQIYLLDINDNAPQVLPQEAETCETPEPNSINIT
ALDYDIDPNAGPFAFDLPLSPVTIKRNWTINRLNGDFAQLNLKIKFLEAGIYEVPIII
TDSGNPPKSNISILRVKVCQCDSNGDCTDVDRIVGAGLGTGAIIAILLCIIILLILVL
MFVVWMKRRDKERQAKQLLIDPEDDVRDNILKYDEEGGGEEDQDYDLSQLQQPDTVEP
DAIKPVGIRRLDERPIHAEPQYPVRSAAPHPGDIGDFINEGLKAADNDPTAPPYDSLL
VFDYEGSGSTAGSLSSLNSSSSGGDQDYDYLNDWGPRFKKLADMYGGGDD

SEQ ID NO: 3
O-cadherin (also known as cadherin-11, cdh11, or ob-cadherin)
From Xenopus laevis
Gene No. 100337621, Accession No. AF002983
nucleotide (RNA), 3237 bp
1 tcggcacgag ctggagtgta caggactttt aagatgctgc tgggtgtctg cactgtgtcc
61 atgtgaatgt ggcattttta ttttgaattc cctccggaga caagatttca tcaagagttt
121 cctttggata ttaagtcaaa gtgcaagcaa tggagattct ctataagaag gcaataatct
181 gggggattta ctaaaattaa acaaacagat tgacattcgc tggatttatc aagcaatttt
241 gcatttacaa cactaccaaa aatgaagaaa gacttttgct tacacggttt acttttatgt
301 ttgggaattg cgtattgtag tcatgccaca tctttaagaa aaaacaataa actaaggcaa
361 tcattccatg gtcaccatga aaaaggcaaa gaagggcaag ttttacatag gtcaaagaga
421 ggatgggttt ggaatcaatt ttttgtaata gaagaataca ccggaccaga tcctgtactc
481 gttggacggc ttcactcaga tgttgactct ggagattgga agataaaata catactctca

541 ggagagggtg ctgggaccat ttttgtcatt gatgacaaat cagggaatat ccatgcaacc
601 aagaccctgg atcgagaaga aagggctcag tataccttaa tggctcaggc agttgacaga
661 gaaacaaata aaccactgga accaccatca gagtttatcg ttaaagttca agacataaat
721 gataatcccc cggagttctt gcatgaaaac taccacgcaa atgtgcctga gatgtccaat
781 gtgggtacat cagtaattca agtaacagcc tctgatgcag atgatccaac atatggaaac
841 agcgctaagc ttgtgtatag tattctcgaa gggcagccat atttttcagt cgaagcacaa
901 tcaggaatca ttaggactgc ccttccaaac atggacagag aagccaagga agaataccat
961 gttgttattc aagcaaagga tatgggagga catatgggag gactctcagg gacaactaaa
1021 gtgacaataa cgctgacaga tgtcaatgac aatccaccaa agtttccaca aagtgcgtac
1081 cccatgtctg tgtcagaagc tgctgtccca ggggaagagg ttggcagaat aaaagctaaa
1141 gatccagaca ttggagaaaa tggcttaata aagtaccgta ttcttgaagg agatggggca
1201 gagatgtttg aaatcacagc tgattatgta actcaggaag gcgttgtaaa gctaaaaaag
1261 gtggtggatt atgaaaccaa gaagttctac agtatgaagg ttgaagctgt caacgttcat
1321 attgatccca gattccttag ccggggacca ttcaaagaca ctgctactgt taagatctca
1381 gtagaggatt ttgatgaacc gcctattttc ttagaaagaa gttacatttt ggaagtatat
1441 gaaaatgctc catcggatac tgtggtcgga agagtgcacg ctaaagaccc agatgctgct
1501 aacagcccaa ttaggtattc aatcgatcgc cacactgacc ttgacagatt cttcagcatc
1561 aacccagagg atggtgtcat caaaaccaca aagggtttgg atagagagga aagcccttgg
1621 cacaacatct cagtcattgc aactgaagtc cacaatcgaa ttcatgaaac tagagttcca
1681 gtagctatta aagtcttgga taagaatgac aatgctccgg aatttgcaaa gccctatgaa
1741 gcttttgtct gtgaaaatgc tccaatcaat caggagtttt tgaccatcac tgcagtagat
1801 aaagatgata cagccaatgg acttcgtttt ctctttagtt tccccccaga aattgtacat
1861 ccaaatccaa atttcaccat aatagacaaa cgagataaca cagcaagcat ccgtgttggc
1921 cgtggagttt tcagccgaca gaaacaagac ttgtatttgg ttcctattgt tataagtgat
1981 gggggaagcc caccgatgag cagcaccaat accctttctg tccgaatctg cagttgcaat
2041 agtgatggat cccaactatc ttgtaatgct gaaccccaat cccttaacgc tggactcagt
2101 actggagcac tgattgcaat ccttgcttgc attgtaattt tattagtgat tgtggttttg
2161 tttgtgactc tgaggagaga gaagaaggaa cctctaattg tctttgaaga ggaagatatc
2221 cgggaaaata taattacata tgatgatgaa ggtggtggag aggaagacac cgaagcattt
2281 gacattgcaa cactgcagaa tcctgatggg attaatggat ttatgccacg gaaagatatc
2341 aaacccgaat ttcaatataa ccccagagat attggaataa gaccagcacc aaacagtgtt
2401 gacgttgatg acttcattaa cacaaggata catgaggccg ataatgaccc tgcagctccg
2461 ccttatgact ccattcagat ctatggatac gaagggagag gttctgtggc tggctctctt
2521 agttcattag agtcagcctc tacagattca gatttggact atgattatct acaaaactgg
2581 ggacctcgat ttaagaaact agcaaattta tatgggtcca aagacacttg tgaagatgat
2641 tcttaacaaa taagttctga atttggcctt atgaactgca taatgtactg aaatatccag
2701 agtaaacatt aacaggtatt tttttaaagg aaaacatgaa aaaggcttct ttaaccttcc
2761 aaggtttaca aacaggattc cttccaaaac aagaactgtt aaatggtggt ggatactgtg
2821 aaaaccctat ggcctgtgta gaagttgtgt attcattttt ttttttgttt tttgtttttt
2881 ttccaagaaa ccacttgtaa aatgcagcct atttaaggga atggaaatgc aggaaaaacg

2941 caacaaaaaa ggggaatctt tacagtatta aacataacca tcaaatcttc tcaaacaaag
3001 cttccacaca aaaaaaaaaa aagataacag ttttgagctg taatttcgcc ttaaactatg
3061 gacactttat atgtagtgca tttttaaact tgaaaaaaat atatatataa tatccagcca
3121 gcttcaatcc atataatgta tgtacagtaa aatgtacaat tattctgtct cttgagcatc
3181 agacttgtta ctgctgattc ttgtaaatct tttttgctta taatcccctc gtgccga

SEQ ID NO: 4
O-cadherin (also known as cadherin-11, cdh11, or ob-cadherin)
From Xenopus laevis
Gene No. 100337621, Accession No. AF002983
polypeptide, translation of SEQ ID NO: 3
MKKDFCLHGLLLCLGIAYCSHATSLRKNNKLRQSFHGHHEKGKE
GQVLHRSKRGWVWNQFFVIEEYTGPDPVLVGRLHSDVDSGDWKIKYILSGEGAGTIFV
IDDKSGNIHATKTLDREERAQYTLMAQAVDRETNKPLEPPSEFIVKVQDINDNPPEFL
HENYHANVPEMSNVGTSVIQVTASDADDPTYGNSAKLVYSILEGQPYFSVEAQSGIIR
TALPNMDREAKEEYHVVIQAKDMGGHMGGLSGTTKVTITLTDVNDNPPKFPQSAYPMS
VSEAAVPGEEVGRIKAKDPDIGENGLIKYRILEGDGAEMFEITADYVTQEGVVKLKKV
VDYETKKFYSMKVEAVNVHIDPRFLSRGPFKDTATVKISVEDFDEPPIFLERSYILEV
YENAPSDTVVGRVHAKDPDAANSPIRYSIDRHTDLDRFFSINPEDGVIKTTKGLDREE
SPWHNISVIATEVHNRIHETRVPVAIKVLDKNDNAPEFAKPYEAFVCENAPINQEFLT
ITAVDKDDTANGLRFLFSFPPEIVHPNPNFTIIDKRDNTASIRVGRGVFSRQKQDLYL
VPIVISDGGSPPMSSTNTLSVRICSCNSDGSQLSCNAEPQSLNAGLSTGALIAILACI
VILLVIVVLFVTLRREKKEPLIVFEEEDIRENIITYDDEGGGEEDTEAFDIATLQNPD
GINGFMPRKDIKPEFQYNPRDIGIRPAPNSVDVDDFINTRIHEADNDPAAPPYDSIQI
YGYEGRGSVAGSLSSLESASTDSDLDYDYLQNWGPRFKKLANLYGSKDTCEDDS

SEQ ID NO: 5
CD133 (also known as PROM-1, prominin-1), isoform 2
From Mus musculus
Gene No. 19126, Accession No. BC028286
nucleotide (mRNA), 3701 bp
1 gtccaatcag tgcgctcaga ctcagagccc taggctcctg ctctttaaat taccgagcct
61 tgtggagacc ccggcacctg gccttaagct cagccctgag gatggtactt tgagtgaatg
121 accaccttgg agaccgttct tctgtttccc ttgttaccag ccaggaggca gaagagtcca
181 ccggtccagg aaagacccat ttcccttgag tttccagaaa gtacctcatg cttgagagat
241 caggccaaca actatggctc tcgtcttcag tgccctgctg ttactggggc tgtgtggaaa
301 gatctcttca gaaggtcagc ctgcattcca taacactcct ggggctatga attatgaatt
361 gcctaccacc aaatatgaga cccaagatac cttcaatgct gggattgttg gccctctcta

421 caaaatggtg cacatcttcc tcaacgtggt ccagccgaat gacttccctc tagatttgat
481 caaaaaactc atacagaaca agaactttga catctcagtt gattccaagg agccagaaat
541 catagtcttg gctctgaaga ttgccctcta tgagatcgga gtccttatct gcgccatcct
601 gggactgctg ttcattatcc tcatgcctct ggtgggctgc ttcttttgta tgtgccgttg
661 ctgcaacaaa tgcggcggag agatgcacca gcggcagaag cagaatgcgc catgcaggag
721 gaagtgcttg ggcctctccc tcctggtgat ttgtctgctc atgagccttg gcattatata
781 tggctttgtg gctaaccagc agaccaggac tcggatcaaa gggacccaga aactggcaaa
841 gagcaatttc agagactttc aaacactcct gactgaaaca ccaaagcaaa ttgactatgt
901 agtggagcag tacaccaaca ccaagaacaa ggcattctca gacctggatg gcatcggctc
961 cgtgctggga ggcagaataa aggaccaact aaaacccaaa gtaactcctg tcctcgaaga
1021 gattaaggcc atggcgacag ccatcaaaca gaccaaggat gccctgcaga acatgagcag
1081 cagcctgaaa agtctccaag atgcagccac ccagctcaat accaacctga gctctgtgag
1141 aaacagcatc gagaattcgc tcagcagcag tgactgtacc tcagatccag ccagcaagat
1201 ctgcgatagc atcagaccaa gcctaagcag tctggggagc agcctcaatt caagtcagct
1261 cccatcagtg gatagagaac tcaacactgt tactgaagtc gacaaaactg atctggagag
1321 cctcgtcaaa agggggtata cgacaattga tgaaataccc aatacaatac aaaaccaaac
1381 tgtggatgtc atcaaagacg tcaaaaatac cttggactcc attagctcca acattaagga
1441 catgagccaa agtattccta ttgaggatat gctgttacag gtctcccatt accttaataa
1501 cagcaacaga tacttaaacc aggagctgcc caagctggaa gaatatgact cgtactggtg
1561 gctgggtggc ttgattgtct gctttctgct gactctcatt gtgaccttct tttcctggg
1621 cttgctgtgt ggtgtgtttg gctatgacaa gcatgccacc ccaactagaa gaggctgtgt
1681 gtccaacact ggaggcatct tcctcatggc tggggttgga ttcggcttcc tttttgctg
1741 gatattgatg atccttgtgg ttcttacgtt tgttgttggt gcaaatgtgg aaaagttgct
1801 ctgcgaacct tatgaaaaca agaaattatt acaggttttg gacactccct atctgctcaa
1861 ggaacaatgg caattttatc tttctggcat gctattcaat aacccagaca ttaacatgac
1921 ctttgagcaa gtctacaggg attgcaaaag aggtcgaggt atatatgctg cttttcagct
1981 tgagaatgtc gtcaacgtca gtgatcattt caacattgac cagatttctg aaaacataaa
2041 tacggagttg gaaaacctga atgtgaacat tgatagcatt gaactgttgg ataacacagg
2101 aaggaagagc ctcgaggact ttgcacattc tgggatagat acaatcgatt attccacata
2161 cttgaaggag actgagaaat cccctactga agtgaatctg ctgacatttg cctctaccct
2221 ggaagcaaaa gcaaaccagt tgcctgaagg aaagctgaaa caggccttct tactggatgt
2281 acagaatata agagccatcc accagcatct cctccctcct gtgcagcaat cactgaaatt
2341 tgtgagggtg aggaatacgt taagacaaag tgtctggacc ctccagcaaa caagcaacaa
2401 gttgccggag aaagtgaaga agatccttgc ctctttggac tctgttcagc atttcctcac
2461 caataacgtt tccctcatcg ttatcgggga aacgaagaag tttgggaaaa caatactagg
2521 ctactttgaa cattatctgc actgggtctt ttatgccatc acagagaaga tgacatcctg
2581 caaacccatg gccaccgcga tggactctgc tgttaatggc attctgtgtg gctatgttgc
2641 ggaccctctg aatttgttct ggttcggcat agggaaagcc acggtgctct tacttccggc
2701 tgtaatcatt gctatcaagc tggccaagta ctatcgcagg atggattcag aggatgtata
2761 cgacgacccg tctcgatact gacaactgga gttgaagctg cttgaacaac aagatagtca

2821 acatggaaag catcacagat tttggatagt ttctgagtct tctagaacgt tccaagtgca
2881 gaagaaacct ggtggagact caggcgggca ctaggaacat ggcatcagtg gtcttagggt
2941 agcactttgt caggaatgaa cagtcatcat ggttataatc cacatatcca ttgcaactca
3001 tgaatgattc tctcctgttt tgtttttaac ttttcttttt acactgattt tctatttaga
3061 cactaaaaca tataggggtg cttattcccc ctggatacat ttacctgtga accagctatt
3121 ccggtgtcat agctgggtac ctaacttact tccatatgtg aagtgtgcta aacacaaacc
3181 agtttacaga agagatgtat tttgtgtata gtaaactgta tatataccct tttaccacag
3241 tcagtttttt aaacaaatga atactctaga tttttcttct aaatgaggtt actgttgggg
3301 tggttgtgac ctagtgatgc tgtagaaagg agtctgcatt cactaaaagt gtgtcaacct
3361 agagcaggca atgcccttcc ttgtggattt ctgtctgctc gttttggagc tacctgcggt
3421 ttagaaatag aattcaagaa caatcacgga gtttcccact tgatgccact gccaaagtca
3481 gaacaaggga tcttgagaga aggaactgtc gctcagctgg gagcggaatc attatcgcaa
3541 tcacaggtcc tggttcacag tttagtggca ctctctggtt tgtaagaatg ggcattacgt
3601 tcagtgtcat ctggtcatct gtgatgtgtg tcatcagcct gtcctgatgt tgagatttaa
3661 aataaagcat gaatgaacag aaaaaaaaaa aaaaaaaaaa a

SEQ ID NO: 6
CD133 (also known as PROM-1, prominin-1), isoform 2
From Mus musculus
Gene No. 19126, Accession No. BC028286
polypeptide, translation of SEQ ID NO: 5
MALVFSALLLLGLCGKISSEGQPAFHNTPGAMNYELPTTKYETQ
DTFNAGIVGPLYKMVHIFLNVVQPNDFPLDLIKKLIQNKNFDISVDSKEPEIIVLALK
IALYEIGVLICAILGLLFIILMPLVGCFFCMCRCCNKCGGEMHQRQKQNAPCRRKCLG
LSLLVICLLMSLGIIYGFVANQQTRTRIKGTQKLAKSNFRDFQTLLTETPKQIDYVVE
QYTNTKNKAFSDLDGIGSVLGGRIKDQLKPKVTPVLEEIKAMATAIKQTKDALQNMSS
SLKSLQDAATQLNTNLSSVRNSIENSLSSSDCTSDPASKICDSIRPSLSSLGSSLNSS
QLPSVDRELNTVTEVDKTDLESLVKRGYTTIDEIPNTIQNQTVDVIKDVKNTLDSISS
NIKDMSQSIPIEDMLLQVSHYLNNSNRYLNQELPKLEEYDSYWWLGGLIVCFLLTLIV
TFFFLGLLCGVFGYDKHATPTRRGCVSNTGGIFLMAGVGFGFLFCWILMILVVLTFVV
GANVEKLLCEPYENKKLLQVLDTPYLLKEQWQFYLSGMLFNNPDINMTFEQVYRDCKR
GRGIYAAFQLENVVNVSDHFNIDQISENINTELENLNVNIDSIELLDNTGRKSLEDFA
HSGIDTIDYSTYLKETEKSPTEVNLLTFASTLEAKANQLPEGKLKQAFLLDVQNIRAI
HQHLLPPVQQSLKFVRVRNTLRQSVWTLQQTSNKLPEKVKKILASLDSVQHFLTNNVS
LIVIGETKKFGKTILGYFEHYLHWVFYAITEKMTSCKPMATAMDSAVNGILCGYVADP
LNLFWFGIGKATVLLLPAVIIAIKLAKYYRRMDSEDVYDDPSRY

SEQ ID NO: 7
FGFR2 IIIc
From Mus musculus
Gene No. 14183, Accession No. M86441
nucleotide (mRNA), 3306 bp
1 gaattcccgc gcggccgcca gagctccggc ccgggggctg cctgtgtgtt cctggcccgg
61 cgtggcgact gctctccggg ctggcggggg ccgggcgtga gcccgggcct cagcgttcct
121 gagcgctgcg agtgttcact actcgccagc aaagtttgga gtaggcaacg caagctccag
181 tcctttcttc tgctgctgcc cagatccgag agcagctccg gtgtatgtct agctgttctg
241 cgatcccggc gcgcgtgaag cctcggaacc ttggcgccgg ctgctaccca aggaatcgtt
301 ctctttttgg agttttcctc cgagatcatc gcctgctcca tcccgatcca ctctgggctc
361 cggcgcagca ccgagcgcag aggagcgctg ccattcaagt ggcagccaca gcagcagcag
421 cagcagcagt gggagcagga acagcagtaa caacagcaac agcagcacag ccgcctcaga
481 gctttgctcc tgagcccctg tgggctgaag gcattgcagg tagcccatgg tctcagaaga
541 agtgtgcaga tgggattacc gtccacgtgg agatatggaa gaggaccagg gattggcact
601 gtgaccatgg tcagctgggg gcgcttcatc tgcctggtct tggtcaccat ggcaaccttg
661 tccctggccc ggccctcctt cagtttagtt gaggatacca ctttagaacc agaagagcca
721 ccaaccaaat accaaatctc ccaaccagaa gcgtacgtgg ttgcccccgg ggaatcgcta
781 gagttgcagt gcatgttgaa agatgccgcc gtgatcagtt ggactaagga tggggtgcac
841 ttggggccca acaataggac agtgcttatt ggggagtatc tccagataaa aggtgccaca
901 cctagagact ccggcctcta tgcttgtact gcagctagga cggtagacag tgaaacttgg
961 atcttcatgg tgaatgtcac agatgccatc tcatctggag atgatgagga cgacacagat
1021 agctccgaag acgttgtcag tgagaacagg agcaaccaga gagcaccgta ctggaccaac
1081 accgagaaga tggagaagcg gctccacgct tgtcctgccg ccaacactgt gaagttccgc
1141 tgtccggctg gggggaatcc aacgtccaca atgaggtggt taaaaaacgg gaaggagttt
1201 aagcaggagc atcgcattgg aggctataag gtacgaaacc agcactggag ccttattatg
1261 gaaagtgtgg tcccgtcaga caaaggcaac tacacctgcc tggtggagaa tgaatacggg
1321 tccatcaacc acacctacca cctggatgtc gttgaacgtt caccacaccg tcccatcctc
1381 caagctggac tgcctgcaaa tgcctccacg gtggtcggag gggatgtgga gtttgtctgc
1441 aaggtttaca gcgatgccca gccccacatc cagtggatca agcacgtgga aaagaacggc
1501 agtaaaaacg ggcctgatgg gctgccctac ctcaaggttc tgaaagctgc cggtgttaac
1561 accacggaca aagagattga ggttctctat attcggaatg taacttttga ggatgctggg
1621 gaatatacgt gcttggcggg taattctatc gggatatcct ttcactctgc atggttgaca
1681 gttctgccag cgcctgtgag agagaaggag atcacggctt ccccagatta tctggagata
1741 gctatttact gcataggggt cttcttaatc gcctgcatgg tggtgacagt catcttttgc
1801 cgaatgaaga ccacgaccaa gaagccagac ttcagcagcc agccagctgt gcacaagctg
1861 accaagcgca tccccctgcg gagacaggta acagtttcgg ccgagtccag ctcctccatg
1921 aactccaaca ccccgctggt gaggataaca acgcgtctgt cctcaacagc ggacaccccg
1981 atgctagcag gggtctccga gtatgagttg ccagaggatc caaagtggga attccccaga

2041 gataagctga cgctgggcaa acccctgggg gaaggttgct tcgggcaagt agtcatggct
2101 gaagcagtgg gaatcgataa agacaaaccc aaggaggcgg tcaccgtggc agtgaagatg
2161 ttgaaagatg atgccacaga gaaggacctg tctgatctgg tatcagagat ggagatgatg
2221 aagatgattg ggaaacataa gaacattatc aacctcctgg gggcctgcac gcaggatgga
2281 cctctctacg tcatagttga atatgcatcg aaaggcaacc tccgggaata cctccgagcc
2341 cggaggccac ctggcatgga gtactcctat gacattaacc gtgtccccga ggagcagatg
2401 accttcaagg acttggtgtc ctgcacctac cagctggcta gaggcatgga gtacttggct
2461 tcccaaaaat gtatccatcg agatttggct gccagaaacg tgttggtaac agaaaacaat
2521 gtgatgaaga tagcagactt tggcctggcc agggatatca acaacataga ctactataaa
2581 aagaccacaa atgggcgact tccagtcaag tggatggctc ctgaagccct ttttgataga
2641 gtttacactc atcagagcga tgtctggtcc ttcggggtgt taatgtggga gatctttact
2701 ttagggggct caccctaccc agggattccc gtggaggaac tttttaagct gctcaaagag
2761 ggacacagga tggacaagcc caccaactgc accaatgaac tgtacatgat gatgagggat
2821 tgctggcatg ctgtaccctc acagagaccc acattcaagc agttggtcga agacttggat
2881 cgaattctga ctctcacaac caatgaggaa tacttggatc tcacccagcc tctcgaacag
2941 tattctccta gttaccccga cacaagtagc tcttgttctt caggggacga ttctgtgttt
3001 tctccagacc ccatgcctta tgaaccctgt ctgcctcagt atccacacat aaacggcagt
3061 gttaaaacat gagtgaatgt gtcttcctgt ccccaaacag gacagcacca ggaacctact
3121 tacactgagc agagaggctg tgctccagag cctgtgacac gcctccactt gtatatatgg
3181 atcagaggag taaatagtgg gaagcatatt tgtcacgtgt gtaaagattt atacagttgg
3241 aacatgtact acaggaagga gactgttctg atagtgacag ccgccaccat gccacctttg
3301 accaca

SEQ ID NO: 8
FGFR2 IIIc
From Mus musculus
Gene No. 14183, Accession No. M86441
polypeptide, translation of SEQ ID NO: 7
MVSWGRFICLVLVTMATLSLARPSFSLVEDTTLEPEEPPTKYQI
SQPEAYVVAPGESLELQCMLKDAAVISWTKDGVHLGPNNRTVLIGEYLQIKGATPRDS
GLYACTAARTVDSETWIFMVNVTDAISSGDDEDDTDSSEDVVSENRSNQRAPYWTNTE
KMEKRLHACPAANTVKFRCPAGGNPTSTMRWLKNGKEFKQEHRIGGYKVRNQHWSLIM
ESVVPSDKGNYTCLVENEYGSINHTYHLDVVERSPHRPILQAGLPANASTVVGGDVEF
VCKVYSDAQPHIQWIKHVEKNGSKNGPDGLPYLKVLKAAGVNTTDKEIEVLYIRNVTF
EDAGEYTCLAGNSIGISFHSAWLTVLPAPVREKEITASPDYLEIAIYCIGVFLIACMV
VTVIFCRMKTTTKKPDFSSQPAVHKLTKRIPLRRQVTVSAESSSSMNSNTPLVRITTR
LSSTADTPMLAGVSEYELPEDPKWEFPRDKLTLGKPLGEGCFGQVVMAEAVGIDKDKP
KEAVTVAVKMLKDDATEKDLSDLVSEMEMMKMIGKHKNIINLLGACTQDGPLYVIVEY
ASKGNLREYLRARRPPGMEYSYDINRVPEEQMTFKDLVSCTYQLARGMEYLASQKCIH

RDLAARNVLVTENNVMKIADFGLARDINNIDYYKKTTNGRLPVKWMAPEALFDRVYTH
QSDVWSFGVLMWEIFTLGGSPYPGIPVEELFKLLKEGHRMDKPTNCTNELYMMMRDCW
HAVPSQRPTFKQLVEDLDRILTLTTNEEYLDLTQPLEQYSPSYPDTSSSCSSGDDSVF
SPDPMPYEPCLPQYPHINGSVKT

SEQ ID NO: 9
FGFR2 IIIb
From Mus musculus
Gene No. 14183, Accession No. M63503
nucleotide (mRNA), 3037 bp
1 ggcgagggga gagagccggg agaggcgagc ggcggcgcgg caggcgcgga acgggcgcac
61 ggacgatcga acgcgcggcc gccagagctc cggcgcgggg gctgcctgtg tgttcctggc
121 ccggcgtggc gactgctctc cgggctggcg ggggccgggc gtgagcccgg gcctcagcgt
181 tcctgagcgc tgcgagtgtt cactactcgc cagcaaagtt tggagtaggc aacgccaagc
241 tccagtcctt tcttctgctg ctgcccagat ccgagagcag ctccggtgtc atgtcctagc
301 tgttctgcga tccccggcgc gcgtgaagcc tcggaacctt cgcgccggct gctacccaag
361 gaatcgttct ctttttggag ttttcctccg agatcatcgc ctgctccatc ccgatccact
421 ctgggctccg gcgcagaccg agcgcagagg agcgctgcca ttcaagtggc agccacagca
481 gcagcagcag cagcagtggg agcaggaaca gcagtaacaa cagcaacagc agcacagccg
541 cctcagagct ttggctcctg agccccctgt gggctgaagg cattgcaggt agcccatggt
601 ctcagaagaa gtgtgcagat gggattaccg tccacgtgga gatatggaag aggaccaggg
661 attggcactg tgaccatggt cagctggggg cgcttcatct gcctggtctt ggtcaccatg
721 gcaaccttgt ccctggcccg gccctccttc agtttagttg aggataccac tttagaacca
781 gaaggagcac cgtactggac caacaccgag aagatggaga agcggctcca cgctgtccct
841 gccgccaaca ctgtgaagtt ccgctgtccg gctgggggga atccaacgcc cacaatgagg
901 tggttaaaaa acgggaagga gtttaagcag gagcatcgca ttggaggcta taaggtacga
961 aaccagcact ggagccttat tatggaaagt gtggtcccgt cagacaaagg caactacacc
1021 tgcctggtgg agaatgaata cgggtccatc aaccacacct accacctcga tgtcgttgaa
1081 cggtcaccac accggcccat cctccaagct ggactgcctg caaatgcctc cacggtggtc
1141 ggaggggatg tggagtttgt ctgcaaggtt tacagcgatg cccagcccca catccagtgg
1201 atcaagcacg tggaaaagaa cggcagtaaa tacgggcctg atgggctgcc ctacctcaag
1261 gtcctgaagc actcggggat aaatagctcc aatgcagaag tgctggctct gttcaatgtg
1321 acggagatgg atgctgggga atatatatgt aaggtctcca attatatagg gcaggccaac
1381 cagtctgcct ggctcactgt cctgcccaaa cagcaagcgc ctgtgagaga gaaggagatc
1441 acggcttccc cagattatct ggagatagct atttactgca taggggtctt cttaatcgcc
1501 tgcatggtgg tgacagtcat cttttgccga atgaagacca cgaccaagaa gccagacttc
1561 agcagccagc cagctgtgca caagctgacc aagcgcatcc ccctgcggag acaggtaaca
1621 gtttcggccg agtccagctc ctccatgaac tccaacaccc cgctggtgag gataacaacg
1681 cgtctgtcct caacagcgga caccccgatg ctagcagggg tctccgagta tgagttgcca

1741 gaggatccaa agtgggaatt ccccagagat aagctgacgc tgggcaaacc cctgggggaa
1801 ggttgcttcg ggcaagtagt catggctgaa gcagtgggaa tcgataaaga caaacccaag
1861 gaggcggtca ccgtggcagt gaagatgttg aaagatgatg ccacagagaa ggacctgtct
1921 gatctggtat cagagatgga gatgatgaag atgattggga aacataagaa cattatcaac
1981 ctcctggggg cctgcacgca ggatggacct ctctacgtca tagttgaata tgcatcgaaa
2041 ggcaacctcc gggaatacct ccgagcccgg aggccacctg gcatggagta ctcctatgac
2101 attaaccgtg tccccgagga gcagatgacc ttcaaggact tggtgtcctg cacctaccag
2161 ctggctagag gcatggagta cttggcttcc caaaaatgta tccatcgaga tttggctgcc
2221 agaaacgtgt tggtaacaga aaacaatgtg atgaagatag cagactttgg cctggccagg
2281 gatatcaaca acatagacta ctataaaaag accacaaatg ggcgacttcc agtcaagtgg
2341 atggctcctg aagccctttt tgatagagtt tacactcatc agagcgatgt ctggtccttc
2401 ggggtgttaa tgtgggagat ctttacttta gggggctcac cctacccagg gattcccgtg
2461 gaggaacttt ttaagctgct caaagaggga cacaggatgg acaagcccac caactgcacc
2521 aatgaactgt acatgatgat gagggattgc tggcatgctg taccctcaca gagacccaca
2581 ttcaagcagt tggtcgaaga cttggatcga attctgactc tcacaaccaa tgaggaatac
2641 ttggatctca cccagcctct cgaacagtat tctcctagtt accccgacac aaggagctct
2701 tgttcttcag gggacgattc tgtgttttct ccagacccca tgccttatga accctgtctg
2761 cctcagtatc cacacataaa cggcagtgtt aaaacatgag tgaatgtgtc ttcctgtccc
2821 caaacaggac agcaccagga acctacttac actgagcaga gaggctgtct cagagcctgt
2881 gacacgcctc cacttgtata tatggatcag aggagtaaat agtgggaagc atattgtcac
2941 gtgtgtaaag atttatacag ttcggaaaca tgttacctaa ccaggaaagg aagactgttt
3001 tcctgataag tggacagccg caagccacca tgccacc

SEQ ID NO: 10
FGFR2 IIIb
From Mus musculus
Gene No. 14183, Accession No. M63503
polypeptide, translation of SEQ ID NO: 9
MVSWGRFICLVLVTMATLSLARPSFSLVEDTTLEPEGAPYWTNT
EKMEKRLHAVPAANTVKFRCPAGGNPTPTMRWLKNGKEFKQEHRIGGYKVRNQHWSLI
MESVVPSDKGNYTCLVENEYGSINHTYHLDVVERSPHRPILQAGLPANASTVVGGDVE
FVCKVYSDAQPHIQWIKHVEKNGSKYGPDGLPYLKVLKHSGINSSNAEVLALFNVTEM
DAGEYICKVSNYIGQANQSAWLTVLPKQQAPVREKEITASPDYLEIAIYCIGVFLIAC
MVVTVIFCRMKTTTKKPDFSSQPAVHKLTKRIPLRRQVTVSAESSSSMNSNTPLVRIT
TRLSSTADTPMLAGVSEYELPEDPKWEFPRDKLTLGKPLGEGCFGQVVMAEAVGIDKD
KPKEAVTVAVKMLKDDATEKDLSDLVSEMEMMKMIGKHKNIINLLGACTQDGPLYVIV
EYASKGNLREYLRARRPPGMEYSYDINRVPEEQMTFKDLVSCTYQLARGMEYLASQKC
IHRDLAARNVLVTENNVMKIADFGLARDINNIDYYKKTTNGRLPVKWMAPEALFDRVY
THQSDVWSFGVLMWEIFTLGGSPYPGIPVEELFKLLKEGHRMDKPTNCTNELYMMMRD

CWHAVPSQRPTFKQLVEDLDRILTLTTNEEYLDLTQPLEQYSPSYPDTRSSCSSGDDS
VFSPDPMPYEPCLPQYPHINGSVKT

SEQ ID NO: 11
E-cadherin (also known as cadherin-1, cdh1)
From Xenopus (Silurana) tropicalis
Gene No. 779546, Accession No. XM_002935997
nucleotide (mRNA), 3344 bp
1 agagcaggga agtacagcgc tgcgctacaa gaactgagca aacgagcaga aaagtacaca
61 ttcctgatcc ttcggtcttt ccaaaagtcc ccaatggggt cacacaggcc atggttactt
121 ggtgctgtgg tgctgctggc actccttcag gtacagggag gactggcaga atggacacag
181 tgtcaaatgg gattttccaa ggaaaggtac agcttttcgg tacctaagaa cttggagaca
241 gacaaagcac tgggtagagt gatctttaac agctgtgagg gaccagtgag aattcagttt
301 gcctctaaag atcctaattt tgaaattcac aaagatggca cagtttatgt taagaatcct
361 accaagatga aagacaacag aaaaacattc cgtgtcctgg cttgggagaa tcaaggtcat
421 gtatactcta ccagtgtaac cttgaaaggg gaagggcatc accataagca ggacatttct
481 tctgtgaaac attcccacca cccaaaatct gagactggtt taaaaagaca aaaaagagac
541 tgggtgattc caccaatcgt aacatctgag aatgaaaagg gcccatttcc caaacggctt
601 gtgcagatca agtccagtaa tgcaaaggaa atcaaggttt tttacagtat cacaggccag
661 ggtgccgata cccctccaga aggagtgttc actattggac gggaggatgg atggctaaat
721 gtgacacgac ctttggacag agaagccatt gatagttaca ctcttttttc tcatgctgtg
781 tcagtaaatg ggcaaaatgt ggaagatccc atggaaatcc aaattaaagt acaagatcag
841 aatgataatg acccagtttt cacacaggag gtctttgaag gctatgtgcc tgaagggtct
901 aagccaggta cgcccgtcat gactgtatct gcaacagatg ccgatgatgc tatagacatg
961 tacaatggtg tgattactta ctccattctc aaccaagacc ctaaagagcc caacaatcaa
1021 atgttcacta ttgattccca gtctgggttg atcagcgtag ttacaactgg attagacaga
1081 gagaaaatac cagtgtacac actgactatt caagctgcag atggagaatt tgggaaagat
1141 cgcacaacaa ctgcaaaagc tgtgatcatt gtgacagaca ccaatgataa ccctcctgtg
1201 tttaacccaa cgcaatacat tgcagaggtt cctgaaaatg aagttggata tgaggttgca
1261 cgtcttacgg taacagatgc agatattgaa gggtcagatg cctggaatgc tgtgtacaag
1321 atcattaaag gaaatgaggc tggctttttc agcatccaaa cagatattga caacattggg
1381 ctactgaaaa cagtgaaggg tctggactat gagctgaaga agcagtatat tctgtcagtc
1441 attgtgacaa acaaagctaa cttttctgtt ccactacaaa cttcaactgc aacggtcact
1501 gtaactgtca cagatgtgaa tgaggcccca gtatttgtac cagtgttgaa agacgtgtct
1561 gtgccagagg atctgcccag tggccaagtt gttgctacct ataccgcaca ggatccagac
1621 aaggaacaga accagaaaat aagttacttc attggaaatg acccagcagg gtgggtgtct
1681 gtgaacagag ataatgggat tgtcactgga aatggaaact tggatcggga atcaaagttt
1741 gtgctaaaca acacctacaa agtcataatc ttggccgctg acagtggcac tccttctgcc
1801 actgggactg gaacccttgt gcttaatctc attgatgtta atgataatgg cccattttg

1861 gatccccaac aaaatagttt ctgccagaag gatccaggct ttcgtgtatt taatatcatt
1921 gacaaagatc tttaccctaa cacataccca tatacagtag acctgactgg tgaatccaat
1981 gaaaactgga ctgctacagt gacagaacag agtttacttg agctgagacc taaaaaggaa
2041 ctggatattg gacgatacga agttttgatc tcattgagag acaatcaggg actgacagat
2101 gtgacaaagc tacagattac aatctgtcaa tgtaatggtg accaaatgca atgtgaggaa
2161 aaggctgctc aagcaggagg tttggggata tcagccatag ttggaatcct tggagggatc
2221 ctagcgcttc ttttattgtt gttgctgctc ttactgtttg tacgacgaaa gaaagtggta
2281 aaagaacctt tattaccacc agaagatgag actcgggaca atgtattttt ctatgatgaa
2341 gaaggcggtg gtgaggaaga ccaggatttt gatctaagcc agcttcaccg tggtctagat
2401 gctcgtccag atataatccg taatgatgtc gttccagttt tagctgctcc ccagtatcga
2461 ccccgtcctg ccaatccaga tgaaattgga aatttcattg atgagaactt gcatgcagct
2521 gacaatgacc ccactgctcc tccatacgac tcgctccttg tgttcgatta cgaaggcagt
2581 ggctctgagg ccgcatcact cagctctctt aactcttcca actctgattt agatcaggat
2641 tacagtgctt tgaataactg gggacctcgt ttcaccaaac tggcagaaat gtatggagga
2701 gatgaggatt agaatgtgca ctgcaatacc attttgattc taaacagtaa actaaaaacc
2761 ataattgtgt atgcagtctt tggaattcac tttgttttct cctgctctta aaacagagat
2821 aaggactgct caaaagttac tcctcctgct tttgtaaaat cgttcaaaaa tattttatgt
2881 atatgtatat atgaaaaaat cgtatttttt gtactatttg tgttcttata tccctgcaat
2941 ttgtaataca agaggatctt tatctgctta attataaata taaaatgccc gatatgattc
3001 actatgattt taatgtgttg agaaatcttt ttttaaaaag gtttccagac acctgacgct
3061 tggaagggaa ttccataaaa atataattga attgggggga gattgtgttt tgccatggtc
3121 tgatatacat tttcatatat atacatatga tcattcacag agtacagtca acatttggaa
3181 tttgatgagc ttgctggtcg aactgaaaaa aaaatgtatt atagctgggg taaaaattaa
3241 tgtatgagct aaatggggca caattttgat atctctgcat ttgtatttta cttggcatgt
3301 atacttttgt aataaaataa agatatacat taatatacaa cata

SEQ ID NO: 12
E-cadherin (also known as cadherin-1, cdh1)
From Xenopus (Silurana) tropicalis
Gene No. 779546, Accession No. XM_002935997
polypeptide (translation of SEQ ID NO: 11), 872 amino acids
MGSHRPWLLGAVVLLALLQVQGGLAEWTQCQMGFSKERYSFSVP
KNLETDKALGRVIFNSCEGPVRIQFASKDPNFEIHKDGTVYVKNPTKMKDNRKTFRVL
AWENQGHVYSTSVTLKGEGHHHKQDISSVKHSHHPKSETGLKRQKRDWVIPPIVTSEN
EKGPFPKRLVQIKSSNAKEIKVFYSITGQGADTPPEGVFTIGREDGWLNVTRPLDREA
IDSYTLFSHAVSVNGQNVEDPMEIQIKVQDQNDNDPVFTQEVFEGYVPEGSKPGTPVM
TVSATDADDAIDMYNGVITYSILNQDPKEPNNQMFTIDSQSGLISVVTTGLDREKIPV
YTLTIQAADGEFGKDRTTTAKAVIIVTDTNDNPPVFNPTQYIAEVPENEVGYEVARLT
VTDADIEGSDAWNAVYKIIKGNEAGFFSIQTDIDNIGLLKTVKGLDYELKKQYILSVI

VTNKANFSVPLQTSTATVTVTVTDVNEAPVFVPVLKDVSVPEDLPSGQVVATYTAQDP
DKEQNQKISYFIGNDPAGWVSVNRDNGIVTGNGNLDRESKFVLNNTYKVIILAADSGT
PSATGTGTLVLNLIDVNDNGPFLDPQQNSFCQKDPGFRVFNIIDKDLYPNTYPYTVDL
TGESNENWTATVTEQSLLELRPKKELDIGRYEVLISLRDNQGLTDVTKLQITICQCNG
DQMQCEEKAAQAGGLGISAIVGILGGILALLLLLLLLLFVRRKKVVKEPLLPPEDET
RDNVFFYDEEGGGEEDQDFDLSQLHRGLDARPDIIRNDVVPVLAAPQYRPRPANPDEI
GNFIDENLHAADNDPTAPPYDSLLVFDYEGSGSEAASLSSLNSSNSDLDQDYSALNNW
GPRFTKLAEMYGGDED

SEQ ID NO: 13
Vimentin
From homo sapiens
Accession No. BC000163
nucleotide (mRNA), 1862 bp
1 gtccccgcgc cagagacgca gccgcgctcc caccacccac acccaccgcg ccctcgttcg
61 cctcttctcc gggagccagt ccgcgccacc gccgccgccc aggccatcgc caccctccgc
121 agccatgtcc accaggtccg tgtcctcgtc ctcctaccgc aggatgttcg gcggcccggg
181 caccgcgagc cggccgagct ccagccggag ctacgtgact acgtccaccc gcacctacag
241 cctgggcagc gcgctgcgcc ccagcaccag ccgcagcctc tacgcctcgt ccccgggcgg
301 cgtgtatgcc acgcgctcct ctgccgtgcg cctgcggagc agcgtgcccg gggtgcggct
361 cctgcaggac tcggtggact tctcgctggc cgacgccatc aacaccgagt tcaagaacac
421 ccgcaccaac gagaaggtgg agctgcagga gctgaatgac cgcttcgcca actacatcga
481 caaggtgcgc ttcctggagc agcagaataa gatcctgctg gccgagctcg agcagctcaa
541 gggccaaggc aagtcgcgcc tgggggacct ctacgaggag gagatgcggg agctgcgccg
601 gcaggtggac cagctaacca acgacaaagc ccgcgtcgag gtggagcgcg acaacctggc
661 cgaggacatc atgcgcctcc gggagaaatt gcaggaggag atgcttcaga gagaggaagc
721 cgaaaacacc ctgcaatctt tcagacagga tgttgacaat gcgtctctgg cacgtcttga
781 ccttgaacgc aaagtggaat ctttgcaaga agagattgcc tttttgaaga aactccacga
841 agaggaaatc caggagctgc aggctcagat tcaggaacag catgtccaaa tcgatgtgga
901 tgtttccaag cctgacctca cggctgccct gcgtgacgta cgtcagcaat atgaaagtgt
961 ggctgccaag aacctgcagg aggcagaaga atggtacaaa tccaagtttg ctgacctctc
1021 tgaggctgcc aaccggaaca atgacgccct gcgccaggca aagcaggagt ccactgagta
1081 ccggagacag gtgcagtccc tcacctgtga agtggatgcc cttaaaggaa ccaatgagtc
1141 cctggaacgc cagatgcgtg aaatggaaga gaactttgcc gttgaagctg ctaactacca
1201 agacactatt ggccgcctgc aggatgagat tcagaatatg aaggaggaaa tggctcgtca
1261 ccttcgtgaa taccaagacc tgctcaatgt taagatggcc cttgacattg agattgccac
1321 ctacaggaag ctgctggaag gcgaggagag caggatttct ctgcctcttc caaacttttc
1381 ctccctgaac ctgagggaaa ctaatctgga ttcactccct ctggttgata cccactcaaa
1441 aaggacactt ctgattaaga cggttgaaac tagagatgga caggttatca acgaaacttc

1501 tcagcatcac gatgaccttg aataaaaatt gcacacactc agtgcagcaa tatattacca
1561 gcaagaataa aaaagaaatc catatcttaa agaaacagct ttcaagtgcc tttctgcagt
1621 ttttcaggag cgcaagatag atttggaata ggaataagct ctagttctta acaaccgaca
1681 ctcctacaag atttagaaaa aagtttacaa cataatctag tttacagaaa aatcttgtgc
1741 tagaatactt tttaaaaggt attttgaata ccattaaaac tgcttttttt tttccagcaa
1801 gtatccaacc aacttggttc tgcttcaata aatctttgga aaaactcaaa aaaaaaaaaa
1861 aa

SEQ ID NO: 14
Vimentin
From homo sapiens
Accession No. BC000163
polypeptide (translation of SEQ ID NO: 13), 466 amino acids
MSTRSVSSSSYRRMFGGPGTASRPSSSRSYVTTSTRTYSLGSAL
RPSTSRSLYASSPGGVYATRSSAVRLRSSVPGVRLLQDSVDFSLADAINTEFKNTRTN
EKVELQELNDRFANYIDKVRFLEQQNKILLAELEQLKGQGKSRLGDLYEEEMRELRRQ
VDQLTNDKARVEVERDNLAEDIMRLREKLQEEMLQREEAENTLQSFRQDVDNASLARL
DLERKVESLQEEIAFLKKLHEEEIQELQAQIQEQHVQIDVDVSKPDLTAALRDVRQQY
ESVAAKNLQEAEEWYKSKFADLSEAANRNNDALRQAKQESTEYRRQVQSLTCEVDALK
GTNESLERQMREMEENFAVEAANYQDTIGRLQDEIQNMKEEMARHLREYQDLLNVKMA
LDIEIATYRKLLEGEESRISLPLPNFSSLNLRETNLDSLPLVDTHSKRTLLIKTVETR
DGQVINETSQHHDDLE

SEQ ID NO: 15
N-cadherin
From homo sapiens
CCDS ID No. CCDS11891.1
nucleotide, 2721 bp
ATGTGCCGGATAGCGGGAGCGCTGCGGACCCTGCTGCCGCTGCTGGCGGCCCTGCTTCAGGCGTCTGTAG
AGGCTTCTGGTGAAATCGCATTATGCAAGACTGGATTTCCTGAAGATGTTTACAGTGCAGTCTTATCGAA
GGATGTGCATGAAGGACAGCCTCTTCTCAATGTGAAGTTTAGCAACTGCAATGGAAAAAGAAAAGTACAA
TATGAGAGCAGTGAGCCTGCAGATTTTAAGGTGGATGAAGATGGCATGGTGTATGCCGTGAGAAGCTTTC
CACTCTCTTCTGAGCATGCCAAGTTCCTGATATATGCCCAAGACAAAGAGACCCAGGAAAAGTGGCAAGT
GGCAGTAAAATTGAGCCTGAAGCCAACCTTAACTGAGGAGTCAGTGAAGGAGTCAGCAGAAGTTGAAGAA
ATAGTGTTCCCAAGACAATTCAGTAAGCACAGTGGCCACCTACAAAGGCAGAAGAGAGACTGGGTCATCC
CTCCAATCAACTTGCCAGAAAACTCCAGGGGACCTTTTCCTCAAGAGCTTGTCAGGATCAGGTCTGATAG
AGATAAAAACCTTTCACTGCGGTACAGTGTAACTGGGCCAGGAGCTGACCAGCCTCCAACTGGTATCTTC
ATTATCAACCCCATCTCGGGTCAGCTGTCGGTGACAAAGCCCCTGGATCGCGAGCAGATAGCCCGGTTTC

ATTTGAGGGCACATGCAGTAGATATTAATGGAAATCAAGTGGAGAACCCCATTGACATTGTCATCAATGT
TATTGACATGAATGACAACAGACCTGAGTTCTTACACCAGGTTTGGAATGGGACAGTTCCTGAGGGATCA
AAGCCTGGAACATATGTGATGACCGTAACAGCAATTGATGCTGACGATCCCAATGCCCTCAATGGGATGT
TGAGGTACAGAATCGTGTCTCAGGCTCCAAGCACCCCTTCACCCAACATGTTTACAATCAACAATGAGAC
TGGTGACATCATCACAGTGGCAGCTGGACTTGATCGAGAAAAAGTGCAACAGTATACGTTAATAATTCAA
GCTACAGACATGGAAGGCAATCCCACATATGGCCTTTCAAACACAGCCACGGCCGTCATCACAGTGACAG
ATGTCAATGACAATCCTCCAGAGTTTACTGCCATGACGTTTTATGGTGAAGTTCCTGAGAACAGGGTAGA
CATCATAGTAGCTAATCTAACTGTGACCGATAAGGATCAACCCCATACACCAGCCTGGAACGCAGTGTAC
AGAATCAGTGGCGGAGATCCTACTGGACGGTTCGCCATCCAGACCGACCCAAACAGCAACGACGGGTTAG
TCACCGTGGTCAAACCAATCGACTTTGAAACAAATAGGATGTTTGTCCTTACTGTTGCTGCAGAAAATCA
AGTGCCATTAGCCAAGGGAATTCAGCACCCCCCTCAGTCAACTGCAACCGTGTCTGTTACAGTTATTGAC
GTAAATGAAAACCCTTATTTTGCCCCCAATCCTAAGATCATTCGCCAAGAAGAAGGGCTTCATGCCGGTA
CCATGTTGACAACATTCACTGCTCAGGACCCAGATCGATATATGCAGCAAAATATTAGATACACTAAATT
ATCTGATCCTGCCAATTGGCTAAAAATAGATCCTGTGAATGGACAAATAACTACAATTGCTGTTTTGGAC
CGAGAATCACCAAATGTGAAAAACAATATATATAATGCTACTTTCCTTGCTTCTGACAATGGAATTCCTC
CTATGAGTGGAACAGGAACGCTGCAGATCTATTTACTTGATATTAATGACAATGCCCCTCAAGTGTTACC
TCAAGAGGCAGAGACTTGCGAAACTCCAGACCCCAATTCAATTAATATTACAGCACTTGATTATGACATT
GATCCAAATGCTGGACCATTTGCTTTTGATCTTCCTTTATCTCCAGTGACTATTAAGAGAAATTGGACCA
TCACTCGGCTTAATGGTGATTTTGCTCAGCTTAATTTAAAGATAAAATTTCTTGAAGCTGGTATCTATGA
AGTTCCCATCATAATCACAGATTCGGGTAATCCTCCCAAATCAAATATTTCCATCCTGCGTGTGAAGGTT
TGCCAGTGTGACTCCAACGGGGACTGCACAGATGTGGACAGGATTGTGGGTGCGGGGCTTGGCACCGGTG
CCATCATTGCCATCCTGCTCTGCATCATCATCCTGCTTATCCTTGTGCTGATGTTTGTGGTATGGATGAA
ACGCCGGGATAAAGAACGCCAGGCCAAACAACTTTTAATTGATCCAGAAGATGATGTAAGAGATAATATT
TTAAAATATGATGAAGAAGGTGGAGGAGAAGAAGACCAGGACTATGACTTGAGCCAGCTGCAGCAGCCTG
ACACTGTGGAGCCTGATGCCATCAAGCCTGTGGGAATCCGACGAATGGATGAAAGACCCATCCACGCCGA
GCCCCAGTATCCGGTCCGATCTGCAGCCCCACACCCTGGAGACATTGGGGACTTCATTAATGAGGGCCTT
AAAGCGGCTGACAATGACCCCACAGCTCCACCATATGACTCCCTGTTAGTGTTTGACTATGAAGGCAGTG
GCTCCACTGCTGGGTCCTTGAGCTCCCTTAATTCCTCAAGTAGTGGTGGTGAGCAGGACTATGATTACCT
GAACGACTGGGGGCCACGGTTCAAGAAACTTGCTGACATGTATGGTGGAGGTGATGACTGA

SEQ ID NO: 16
N-cadherin
From homo sapiens
CCDS ID No. CCDS11891.1
polypeptide (translation of SEQ ID NO: 15), 906 amino acids















SEQ ID NO: 17
O-cadherin (also known as ob-cadherin)
From homo sapiens
CCDS ID No. CCDS10803.1
nucleotide, 2391 bp
ATGAAGGAGAACTACTGTTTACAAGCCGCCCTGGTGTGCCTGGGCATGCTGTGCCACAGCCATGCCTTTG
CCCCAGAGCGGCGGGGGCACCTGCGGCCCTCCTTCCATGGGCACCATGAGAAGGGCAAGGAGGGGCAGGT
GCTACAGCGCTCCAAGCGTGGCTGGGTCTGGAACCAGTTCTTCGTGATAGAGGAGTACACCGGGCCTGAC
CCCGTGCTTGTGGGCAGGCTTCATTCAGATATTGACTCTGGTGATGGGAACATTAAATACATTCTCTCAG
GGGAAGGAGCTGGAACCATTTTTGTGATTGATGACAAATCAGGGAACATTCATGCCACCAAGACGTTGGA
TCGAGAAGAGAGAGCCCAGTACACGTTGATGGCTCAGGCGGTGGACAGGGACACCAATCGGCCACTGGAG
CCACCGTCGGAATTCATTGTCAAGGTCCAGGACATTAATGACAACCCTCCGGAGTTCCTGCACGAGACCT
ATCATGCCAACGTGCCTGAGAGGTCCAATGTGGGAACGTCAGTAATCCAGGTGACAGCTTCAGATGCAGA
TGACCCCACTTATGGAAATAGCGCCAAGTTAGTGTACAGTATCCTCGAAGGACAACCCTATTTTTCGGTG
GAAGCACAGACAGGTATCATCAGAACAGCCCTACCCAACATGGACAGGGAGGCCAAGGAGGAGTACCACG
TGGTGATCCAGGCCAAGGACATGGGTGGACATATGGGCGGACTCTCAGGGACAACCAAAGTGACGATCAC
ACTGACCGATGTCAATGACAACCCACCAAAGTTTCCGCAGAGCGTATACCAGATGTCTGTGTCAGAAGCA
GCCGTCCCTGGGGAGGAAGTAGGAAGAGTGAAAGCTAAAGATCCAGACATTGGAGAAAATGGCTTAGTCA
CATACAATATTGTTGATGGAGATGGTATGGAATCGTTTGAAATCACAACGGACTATGAAACACAGGAGGG
GGTGATAAAGCTGAAAAAGCCTGTAGATTTTGAAACCAAAAGAGCCTATAGCTTGAAGGTAGAGGCAGCC
AACGTGCACATCGACCCGAAGTTTATCAGCAATGGCCCTTTCAAGGACACTGTGACCGTCAAGATCTCAG
TAGAAGATGCTGATGAGCCCCCTATGTTCTTGGCCCCAAGTTACATCCACGAAGTCCAAGAAAATGCAGC
TGCTGGCACCGTGGTTGGGAGAGTGCATGCCAAAGACCCTGATGCTGCCAACAGCCCGATAAGGTATTCC
ATCGATCGTCACACTGACCTCGACAGATTTTTCACTATTAATCCAGAGGATGGTTTTATTAAAACTACAA
AACCTCTGGATAGAGAGGAAACAGCCTGGCTCAACATCACTGTCTTTGCAGCAGAAATCCACAATCGGCA
TCAGGAAGCCAAAGTCCCAGTGGCCATTAGGGTCCTTGATGTCAACGATAATGCTCCCAAGTTTGCTGCC
CCTTATGAAGGTTTCATCTGTGAGAGTGATCAGACCAAGCCACTTTCCAACCAGCCAATTGTTACAATTA
GTGCAGATGACAAGGATGACACGGCCAATGGACCAAGATTTATCTTCAGCCTACCCCCTGAAATCATTCA
CAATCCAAATTTCACAGTCAGAGACAACCGAGATAACACAGCAGGCGTGTACGCCCGGCGTGGAGGGTTC

AGTCGGCAGAAGCAGGACTTGTACCTTCTGCCCATAGTGATCAGCGATGGCGGCATCCCGCCCATGAGTA
GCACCAACACCCTCACCATCAAAGTCTGCGGGTGCGACGTGAACGGGGCACTGCTCTCCTGCAACGCAGA
GGCCTACATTCTGAACGCCGGCCTGAGCACAGGCGCCCTGATCGCCATCCTCGCCTGCATCGTCATTCTC
CTGGTCATTGTAGTATTGTTTGTGACCCTGAGAAGGCAAAAGAAAGAACCACTCATTGTCTTTGAGGAAG
AAGATGTCCGTGAGAACATCATTACTTATGATGATGAAGGGGGTGGGGAAGAAGACACAGAAGCCTTTGA
TATTGCCACCCTCCAGAATCCTGATGGTATCAATGGATTTATCCCCCGCAAAGACATCAAACCTGAGTAT
CAGTACATGCCTAGACCTGGGCTCCGGCCAGCGCCCAACAGCGTGGATGTCGATGACTTCATCAACACGA
GAATACAGGAGGCAGACAATGACCCCACGGCTCCTCCTTATGACTCCATTCAAATCTACGGTTATGAAGG
CAGGGGCTCAGTGGCCGGGTCCCTGAGCTCCCTAGAGTCGGCCACCACAGATTCAGACTTGGACTATGAT
TATCTACAGAACTGGGGACCTCGTTTTAAGAAACTAGCAGATTTGTATGGTTCCAAAGACACTTTTGATG
ACGATTCTTAA

SEQ ID NO: 18
O-cadherin (also known as ob-cadherin)
From homo sapiens
CCDS ID No. CCDS10803.1
polypeptide (translation of SEQ ID NO: 17), 796 amino acids
MKENYCLQAALVCLGMLCHSHAFAPERRGHLRPSFHGHHEKGKEGQVLQRSKRGWVWNQFFVIEEYTGPD
PVLVGRLHSDIDSGDGNIKYILSGEGAGTIFVIDDKSGNIHATKTLDREERAQYTLMAQAVDRDTNRPLE
PPSEFIVKVQDINDNPPEFLHETYHANVPERSNVGTSVIQVTASDADDPTYGNSAKLVYSILEGQPYFSV
EAQTGIIRTALPNMDREAKEEYHVVIQAKDMGGHMGGLSGTTKVTITLTDVNDNPPKFPQSVYQMSVSEA
AVPGEEVGRVKAKDPDIGENGLVTYNIVDGDGMESFEITTDYETQEGVIKLKKPVDFETKRAYSLKVEAA
NVHIDPKFISNGPFKDTVTVKISVEDADEPPMFLAPSYIHEVQENAAAGTVVGRVHAKDPDAANSPIRYS
IDRHTDLDRFFTINPEDGFIKTTKPLDREETAWLNITVFAAEIHNRHQEAKVPVAIRVLDVNDNAPKFAA
PYEGFICESDQTKPLSNQPIVTISADDKDDTANGPRFIFSLPPEIIHNPNFTVRDNRDNTAGVYARRGGF
SRQKQDLYLLPIVISDGGIPPMSSTNTLTIKVCGCDVNGALLSCNAEAYILNAGLSTGALIAILACIVIL
LVIVVLFVTLRRQKKEPLIVFEEEDVRENIITYDDEGGGEEDTEAFDIATLQNPDGINGFIPRKDIKPEY
QYMPRPGLRPAPNSVDVDDFINTRIQEADNDPTAPPYDSIQIYGYEGRGSVAGSLSSLESATTDSDLDYD
YLQNWGPRFKKLADLYGSKDTFDDDS

SEQ ID NO: 19
CD133 (also known as PROM1)
From homo sapiens
CCDS ID No. CCDS47029.1
nucleotide, 2598 bp
ATGGCCCTCGTACTCGGCTCCCTGTTGCTGCTGGGGCTGTGCGGGAACTCCTTTTCAGGAGGGCAGCCTT
CATCCACAGATGCTCCTAAGGCTTGGAATTATGAATTGCCTGCAACAAATTATGAGACCCAAGACTCCCA
TAAAGCTGGACCCATTGGCATTCTCTTTGAACTAGTGCATATCTTTCTCTATGTGGTACAGCCGCGTGAT

TTCCCAGAAGATACTTTGAGAAAATTCTTACAGAAGGCATATGAATCCAAAATTGATTATGACAAGCCAG
AAACTGTAATCTTAGGTCTAAAGATTGTCTACTATGAAGCAGGGATTATTCTATGCTGTGTCCTGGGGCT
GCTGTTTATTATTCTGATGCCTCTGGTGGGGTATTTCTTTGTATGTGTCGTTGCTGTAACAAATGTGGT
GGAGAAATGCACCAGCGACAGAAGGAAAATGGGCCCTTCCTGAGGAAATGCTTTGCAATCTCCCTGTTGG
TGATTTGTATAATAATAAGCATTGGCATCTTCTATGGTTTTGTGGCAAATCACCAGGTAAGAACCCGGAT
CAAAAGGAGTCGGAAACTGGCAGATAGCAATTTCAAGGACTTGCGAACTCTCTTGAATGAAACTCCAGAG
CAAATCAAATATATATTGGCCCAGTACAACACTACCAAGGACAAGGCGTTCACAGATCTGAACAGTATCA
ATTCAGTGCTAGGAGGCGGAATTCTTGACCGACTGAGACCCAACATCATCCCTGTTCTTGATGAGATTAA
GTCCATGGCAACAGCGATCAAGGAGACCAAAGAGGCGTTGGAGAACATGAACAGCACCTTGAAGAGCTTG
CACCAACAAAGTACACAGCTTAGCAGCAGTCTGACCAGCGTGAAAACTAGCCTGCGGTCATCTCTCAATG
ACCCTCTGTGCTTGGTGCATCCATCAAGTGAAACCTGCAACAGCATCAGATTGTCTCTAAGCCAGCTGAA
TAGCAACCCTGAACTGAGGCAGCTTCCACCCGTGGATGCAGAACTTGACAACGTTAATAACGTTCTTAGG
ACAGATTTGGATGGCCTGGTCCAACAGGGCTATCAATCCCTTAATGATATACCTGACAGAGTACAACGCC
AAACCACGACTGTCGTAGCAGGTATCAAAAGGGTCTTGAATTCCATTGGTTCAGATATCGACAATGTAAC
TCAGCGTCTTCCTATTCAGGATATACTCTCAGCATTCTCTGTTTATGTTAATAACACTGAAAGTTACATC
CACAGAAATTTACCTACATTGGAAGAGTATGATTCATACTGGTGGCTGGGTGGCCTGGTCATCTGCTCTC
TGCTGACCCTCATCGTGATTTTTTACTACCTGGGCTTACTGTGTGGCGTGTGCGGCTATGACAGGCATGC
CACCCCGACCACCCGAGGCTGTGTCTCCAACACCGGAGGCGTCTTCCTCATGGTTGGAGTTGGATTAAGT
TTCCTCTTTTGCTGGATATTGATGATCATTGTGGTTCTTACCTTTGTCTTTGGTGCAAATGTGGAAAAAC
TGATCTGTGAACCTTACACGAGCAAGGAATTATTCCGGGTTTTGGATACACCCTACTTACTAAATGAAGA
CTGGGAATACTATCTCTCTGGGAAGCTATTTAATAAATCAAAAATGAAGCTCACTTTTGAACAAGTTTAC
AGTGACTGCAAAAAAAATAGAGGCACTTACGGCACTCTTCACCTGCAGAACAGCTTCAATATCAGTGAAC
ATCTCAACATTAATGAGCATACTGGAAGCATAAGCAGTGAATTGGAAAGTCTGAAGGTAAATCTTAATAT
CTTTCTGTTGGGTGCAGCAGGAAGAAAAAACCTTCAGGATTTTGCTGCTTGTGGAATAGACAGAATGAAT
TATGACAGCTACTTGGCTCAGACTGGTAAATCCCCCGCAGGAGTGAATCTTTTATCATTTGCATATGATC
TAGAAGCAAAAGCAAACAGTTTGCCCCCAGGAAATTTGAGGAACTCCCTGAAAAGAGATGCACAAACTAT
TAAAACAATTCACCAGCAACGAGTCCTTCCTATAGAACAATCACTGAGCACTCTATACCAAAGCGTCAAG
ATACTTCAACGCACAGGGAATGGATTGTTGGAGAGAGTAACTAGGATTCTAGCTTCTCTGGATTTTGCTC
AGAACTTCATCACAAACAATACTTCCTCTGTTATTATTGAGGAAACTAAGAAGTATGGGAGAACAATAAT
AGGATATTTTGAACATTATCTGCAGTGGATCGAGTTCTCTATCAGTGAGAAAGTGGCATCGTGCAAACCT
GTGGCCACCGCTCTAGATACTGCTGTTGATGTCTTTCTGTGTAGCTACATTATCGACCCCTTGAATTTGT
TTTGGTTTGGCATAGGAAAAGCTACTGTATTTTTACTTCCGGCTCTAATTTTTGCGGTAAAACTGGCTAA
GTACTATCGTCGAATGGATTCGGAGGACGTGTACGATGATGTTGAAACTATACCCATGAAAAATATGGAA
AATGGTAATAATGGTTATCATAAAGATCATGTATATGGTATTCACAATCCTGTTATGACAAGCCCATCAC
AACATTGA

SEQ ID NO: 20
CD133 (also known as PROM1)
From homo sapiens
CCDS ID No. CCDS47029.1
poplypeptide (translation of SEQ ID NO: 19), 865 amino acids














SEQ ID NO: 21
FGFR2, isoform 1
From homo sapiens
CCDS ID No. CCDS31298.1
nucleotide, 2466 bp
ATGGTCAGCTGGGGTCGTTTCATCTGCCTGGTCGTGGTCACCATGGCAACCTTGTCCCTGGCCCGGCCCT
CCTTCAGTTTAGTTGAGGATACCACATTAGAGCCAGAAGAGCCACCAACCAAATACCAAATCTCTCAACC
AGAAGTGTACGTGGCTGCGCCAGGGGAGTCGCTAGAGGTGCGCTGCCTGTTGAAAGATGCCGCCGTGATC
AGTTGGACTAAGGATGGGGTGCACTTGGGGCCCAACAATAGGACAGTGCTTATTGGGGAGTACTTGCAGA
TAAAGGGCGCCACGCCTAGAGACTCCGGCCTCTATGCTTGTACTGCCAGTAGGACTGTAGACAGTGAAAC
TTGGTACTTCATGGTGAATGTCACAGATGCCATCTCATCCGGAGATGATGAGGATGACACCGATGGTGCG
GAAGATTTTGTCAGTGAGAACAGTAACAACAAGAGAGCACCATACTGGACCAACACAGAAAAGATGGAAA
AGCGGCTCCATGCTGTGCCTGCGGCCAACACTGTCAAGTTTCGCTGCCCAGCCGGGGGGAACCCAATGCC
AACCATGCGGTGGCTGAAAAACGGGAAGGAGTTTAAGCAGGAGCATCGCATTGGAGGCTACAAGGTACGA
AACCAGCACTGGAGCCTCATTATGGAAAGTGTGGTCCCATCTGACAAGGGAAATTATACCTGTGTAGTGG
AGAATGAATACGGGTCCATCAATCACACGTACCACCTGGATGTTGTGGAGCGATCGCCTCACCGGCCCAT
CCTCCAAGCCGGACTGCCGGCAAATGCCTCCACAGTGGTCGGAGGAGACGTAGAGTTTGTCTGCAAGGTT
TACAGTGATGCCCAGCCCCACATCCAGTGGATCAAGCACGTGGAAAAGAACGGCAGTAAATACGGGCCCG
ACGGGCTGCCCTACCTCAAGGTTCTCAAGGCCGCCGGTGTTAACACCACGGACAAAGAGATTGAGGTTCT

CTATATTCGGAATGTAACTTTTGAGGACGCTGGGGAATATACGTGCTTGGCGGGTAATTCTATTGGGATA
TCCTTTCACTCTGCATGGTTGACAGTTCTGCCAGCGCCTGGAAGAGAAAAGGAGATTACAGCTTCCCCAG
ACTACCTGGAGATAGCCATTTACTGCATAGGGGTCTTCTTAATCGCCTGTATGGTGGTAACAGTCATCCT
GTGCCGAATGAAGAACACGACCAAGAAGCCAGACTTCAGCAGCCAGCCGGCTGTGCACAAGCTGACCAAA
CGTATCCCCCTGCGGAGACAGGTAACAGTTTCGGCTGAGTCCAGCTCCTCCATGAACTCCAACACCCCGC
TGGTGAGGATAACAACACGCCTCTCTTCAACGGCAGACACCCCCATGCTGGCAGGGGTCTCCGAGTATGA
ACTTCCAGAGGACCCAAAATGGGAGTTTCCAAGAGATAAGCTGACACTGGGCAAGCCCCTGGGAGAAGGT
TGCTTTGGGCAAGTGGTCATGGCGGAAGCAGTGGGAATTGACAAAGACAAGCCCAAGGAGGCGGTCACCG
TGGCCGTGAAGATGTTGAAAGATGATGCCACAGAGAAAGACCTTTCTGATCTGGTGTCAGAGATGGAGAT
GATGAAGATGATTGGGAAACACAAGAATATCATAAATCTTCTTGGAGCCTGCACACAGGATGGGCCTCTC
TATGTCATAGTTGAGTATGCCTCTAAAGGCAACCTCCGAGAATACCTCCGAGCCCGGAGGCCACCCGGGA
TGGAGTACTCCTATGACATTAACCGTGTTCCTGAGGAGCAGATGACCTTCAAGGACTTGGTGTCATGCAC
CTACCAGCTGGCCAGAGGCATGGAGTACTTGGCTTCCCAAAAATGTATTCATCGAGATTTAGCAGCCAGA
AATGTTTTGGTAACAGAAAACAATGTGATGAAAATAGCAGACTTTGGACTCGCCAGAGATATCAACAATA
TAGACTATTACAAAAAGACCACCAATGGGCGGCTTCCAGTCAAGTGGATGGCTCCAGAAGCCCTGTTTGA
TAGAGTATACACTCATCAGAGTGATGTCTGGTCCTTCGGGGTGTTAATGTGGGAGATCTTCACTTTAGGG
GGCTCGCCCTACCCAGGGATTCCCGTGGAGGAACTTTTTAAGCTGCTGAAGGAAGGACACAGAATGGATA
AGCCAGCCAACTGCACCAACGAACTGTACATGATGATGAGGGACTGTTGGCATGCAGTGCCCTCCCAGAG
ACCAACGTTCAAGCAGTTGGTAGAAGACTTGGATCGAATTCTCACTCTCACAACCAATGAGGAATACTTG
GACCTCAGCCAACCTCTCGAACAGTATTCACCTAGTTACCCTGACACAAGAAGTTCTTGTTCTTCAGGAG
ATGATTCTGTTTTTTCTCCAGACCCCATGCCTTACGAACCATGCCTTCCTCAGTATCCACACATAAACGG
CAGTGTTAAAACATGA

SEQ ID NO: 22
FGFR2, isoform 1
From homo sapiens
CCDS ID No. CCDS31298.1
polypeptide (translation of SEQ ID NO: 21), 821 amino acids














SEQ ID NO: 23
E-cadherin (also known as CDH1)
From homo sapiens
CCDS ID No. CCDS10869.1
nucleotide, 2649 bp
ATGGGCCCTTGGAGCCGCAGCCTCTCGGCGCTGCTGCTGCTGCTGCAGGTCTCCTCTTGGCTCTGCCAGG
AGCCGGAGCCCTGCCACCCTGGCTTTGACGCCGAGAGCTACACGTTCACGGTGCCCCGGCGCCACCTGGA
GAGAGGCCGCGTCCTGGGCAGAGTGAATTTTGAAGATTGCACCGGTCGACAAAGGACAGCCTATTTTTCC
CTCGACACCCGATTCAAAGTGGGCACAGATGGTGTGATTACAGTCAAAAGGCCTCTACGGTTTCATAACC
CACAGATCCATTTCTTGGTCTACGCCTGGGACTCCACCTACAGAAAGTTTTCCACCAAAGTCACGCTGAA
TACAGTGGGGCACCACCACCGCCCCCCGCCCCATCAGGCCTCCGTTTCTGGAATCCAAGCAGAATTGCTC
ACATTTCCCAACTCCTCTCCTGGCCTCAGAAGACAGAAGAGAGACTGGGTTATTCCTCCCATCAGCTGCC
CAGAAAATGAAAAAGGCCCATTTCCTAAAAACCTGGTTCAGATCAAATCCAACAAAGACAAAGAAGGCAA
GGTTTTCTACAGCATCACTGGCCAAGGAGCTGACACACCCCCTGTTGGTGTCTTTATTATTGAAAGAGAA
ACAGGATGGCTGAAGGTGACAGAGCCTCTGGATAGAGAACGCATTGCCACATACACTCTCTTCTCTCACG
CTGTGTCATCCAACGGGAATGCAGTTGAGGATCCAATGGAGATTTTGATCACGGTAACCGATCAGAATGA
CAACAAGCCCGAATTCACCCAGGAGGTCTTTAAGGGGTCTGTCATGGAAGGTGCTCTTCCAGGAACCTCT
GTGATGGAGGTCACAGCCACAGACGCGGACGATGATGTGAACACCTACAATGCCGCCATCGCTTACACCA
TCCTCAGCCAAGATCCTGAGCTCCCTGACAAAAATATGTTCACCATTAACAGGAACACAGGAGTCATCAG
TGTGGTCACCACTGGGCTGGACCGAGAGAGTTTCCCTACGTATACCCTGGTGGTTCAAGCTGCTGACCTT
CAAGGTGAGGGGTTAAGCACAACAGCAACAGCTGTGATCACAGTCACTGACACCAACGATAATCCTCCGA
TCTTCAATCCCACCACGTACAAGGGTCAGGTGCCTGAGAACGAGGCTAACGTCGTAATCACCACACTGAA
AGTGACTGATGCTGATGCCCCCAATACCCCAGCGTGGGAGGCTGTATACACCATATTGAATGATGATGGT
GGACAATTTGTCGTCACCACAAATCCAGTGAACAACGATGGCATTTTGAAAACAGCAAAGGGCTTGGATT
TTGAGGCCAAGCAGCAGTACATTCTACACGTAGCAGTGACGAATGTGGTACCTTTTGAGGTCTCTCTCAC
CACCTCCACAGCCACCGTCACCGTGGATGTGCTGGATGTGAATGAAGCCCCCATCTTTGTGCCTCCTGAA
AAGAGAGTGGAAGTGTCCGAGGACTTTGGCGTGGGCCAGGAAATCACATCCTACACTGCCCAGGAGCCAG
ACACATTTATGGAACAGAAAATAACATATCGGATTTGGAGAGACACTGCCAACTGGCTGGAGATTAATCC
GGACACTGGTGCCATTTCCACTCGGGCTGAGCTGGACAGGGAGGATTTTGAGCACGTGAAGAACAGCACG
TACACAGCCCTAATCATAGCTACAGACAATGGTTCTCCAGTTGCTACTGGAACAGGGACACTTCTGCTGA
TCCTGTCTGATGTGAATGACAACGCCCCCATACCAGAACCTCGAACTATATTCTTCTGTGAGAGGAATCC
AAAGCCTCAGGTCATAAACATCATTGATGCAGACCTTCCTCCCAATACATCTCCCTTCACAGCAGAACTA
ACACACGGGGCGAGTGCCAACTGGACCATTCAGTACAACGACCCAACCCAAGAATCTATCATTTTGAAGC
CAAAGATGGCCTTAGAGGTGGGTGACTACAAAATCAATCTCAAGCTCATGGATAACCAGAATAAAGACCA
AGTGACCACCTTAGAGGTCAGCGTGTGTGACTGTGAAGGGGCCGCTGGCGTCTGTAGGAAGGCACAGCCT
GTCGAAGCAGGATTGCAAATTCCTGCCATTCTGGGGATTCTTGGAGGAATTCTTGCTTTGCTAATTCTGA
TTCTGCTGCTCTTGCTGTTTCTTCGGAGGAGAGCGGTGGTCAAAGAGCCCTTACTGCCCCCAGAGGATGA

CACCCGGGACAACGTTTATTACTATGATGAAGAAGGAGGCGGAGAAGAGGACCAGGACTTTGACTTGAGC
CAGCTGCACAGGGGCCTGGACGCTCGGCCTGAAGTGACTCGTAACGACGTTGCACCAACCCTCATGAGTG
TCCCCCGGTATCTTCCCCGCCCTGCCAATCCCGATGAAATTGGAAATTTTATTGATGAAAATCTGAAAGC
GGCTGATACTGACCCCACAGCCCCGCCTTATGATTCTCTGCTCGTGTTTGACTATGAAGGAAGCGGTTCC
GAAGCTGCTAGTCTGAGCTCCCTGAACTCCTCAGAGTCAGACAAAGACCAGGACTATGACTACTTGAACG
AATGGGGCAATCGCTTCAAGAAGCTGGCTGACATGTACGGAGGCGGCGAGGACGACTAG

SEQ ID NO: 24
E-cadherin (also known as CDH1)
From homo sapiens
CCDS ID No. CCDS10869.1
polypeptide (translation of SEQ ID NO: 23), 882 amino acids














Further embodiments of the invention are as follows:
1. A method for detecting a circulating tumor cell (CTC) in a biological sample, the method comprising detecting at least one epithelial mesenchymal transition (EMT) biomarker in the biological sample.
2. The method of embodiment 1, wherein the sample is a blood sample.
3. The method of any one of the preceding embodiments, wherein the at least one EMT biomarker is vimentin, N-cadherin, O-cadherin, E-cadherin, FGFR2 splice variant isoforms, or CD133.
4. The method of any one of the preceding embodiments, wherein the method is performed at the time of or prior to cancer metastasis.
5. The method of any one of the preceding embodiments, wherein the at least one EMT biomarker is detected by flow cytometry, ferromagnetic enrichment, ferromagnetic sorting, or EMT antigen-antibody binding.
6. The method of any one of the preceding embodiments, comprising detecting at least two EMT biomarkers.
7. A kit for detecting a circulating tumor cell (CTC) in a biological sample, the kit comprising an antibody to at least one EMT biomarker and instructions for use.
8. The kit of embodiment 7, wherein the antibody is linked to a fluorescent reporter molecule, radionuclide, enzyme, or magnetic bead.
9. The kit of embodiment 7 or 8, wherein the at least one EMT biomarker is vimentin, N-cadherin, O-cadherin, E-cadherin, FGFR2 splice variant isoforms, or CD133.
10. A method of predicting responsiveness of a subject having cancer to a course of treatment, the method comprising:
   determining the level or presence of expression of at least one EMT biomarker in a sample from the subject to obtain a gene expression pattern or biomarker profile in CTCs for the subject; and
   predicting responsiveness of the subject to the cancer drug based on the gene expression pattern or biomarker pattern obtained.
11. The method of embodiment 10, wherein the at least one EMT biomarker is vimentin, N-cadherin, O-cadherin, E-cadherin, FGFR2 splice variant isoforms, or CD 133.
12. A method of targeting delivery of a cancer drug in a subject having cancer comprising administering to the subject the cancer drug linked to an antibody specific for at least one EMT biomarker.
13. The method of embodiment 12, wherein the at least one EMT biomarker is vimentin, N-cadherin, O-cadherin, E-cadherin, FGFR2 splice variant isoforms, or CD 133.
14. A method of determining a prognosis cancer prognosis to a subject, the method comprising:
   determining the level of expression of at least one EMT biomarker in a sample from the subject to determine the number of CTCs in the subject and to obtain a gene expression pattern for the subject; and
   providing a prognosis to the subject based on the gene expression pattern obtained.
15. The method of embodiment 14, wherein the at least one EMT biomarker is vimentin, N-cadherin, O-cadherin, E-cadherin, FGFR2 splice variant isoforms, or CD 133.
16. The method of embodiment 14 or 15, wherein the cancer is selected from prostate, colon, and breast cancer, solid tumor malignancies, or sarcomas.
17. A method for monitoring progression of cancer in a subject undergoing therapeutic treatment, the method comprising:
   detecting the number of CTCs based on the expression of at least one EMT biomarker in a first and a second sample taken from the subject at a first and a second time; and comparing the first and second levels of expression;
   wherein a detected difference in number of CTCs based on the level of expression of the at least one EMT biomarker in the first and second samples indicates a change in the progression of the cancer.
18. The method of embodiment 17, wherein an increase in the detected level of the at least one EMT biomarker in the second sample relative to the first sample indicates progression of the cancer.
19. The method of embodiment 17, wherein a decrease in the detected level of the at least one EMT biomarker in the second sample relative to the first sample indicates that the therapeutic treatment is effective.
20. The method of embodiment 19, wherein the decrease indicates remission of the cancer.
21. The method of embodiment 17, whereby no difference in the detected level of the at least one EMT biomarker in the second sample relative to the first sample indicates arrest or stability in the progression of the cancer.
22. The method of any of embodiments 17 - 21, wherein the at least one EMT biomarker is vimentin, N-cadherin, O-cadherin, E-cadherin, FGFR2 splice variant isoforms, or CD133.
23. The method of any of embodiments 17 - 21, wherein the cancer is selected from prostate, colon, and breast cancer.
24. A method for detecting cancer in a subject, the method comprising
   detecting the presence of at least one EMT biomarker in a sample from the subject;
   comparing the detected amount of the at least one EMT biomarker from the sample to a control sample;
   correlating the detected amount of the at least one EMT biomarker from the sample to the presence of CTCs in the sample;
   wherein the presence of CTCs in the sample indicates the presence of cancer in the subject.
25. The method of embodiment 24, wherein the at least one EMT biomarker is vimentin, N-cadherin, O-cadherin, E-cadherin, FGFR2 splice variant isoforms, or CD 133.
26. The method of embodiment 24 or 25, wherein the cancer is selected from prostate, colon, and breast cancer.
27. A method of treating cancer in a subject comprising administering to the subject a cancer drug linked to an antibody that specifically binds at least one EMT biomarker.
28. The method of embodiment 27, wherein the at least one EMT biomarker is vimentin, N-cadherin, O-cadherin, E-cadherin, FGFR2 splice variant isoforms, or CD 133.
29. The method of embodiment 27 or 28, wherein the cancer is selected from prostate, colon, and breast cancer.

## Claims

1. A method for detecting a circulating tumor cell (CTC) in a biological sample, the method comprising detecting O-cadherin in the biological sample.

2. The method of claim 1, wherein the sample is a blood sample.

3. The method of any one of the preceding claims, wherein the method is performed at the time of or prior to cancer metastasis.

4. The method of any one of the preceding claims, wherein O-cadherin is detected by flow cytometry, ferromagnetic enrichment, ferromagnetic sorting, or antigen-antibody binding.

5. The method of any one of the preceding claims, further comprising detecting in the biological sample at least one additional biomarker selected from vimentin, N-cadherin, E-cadherin, FGFR2 splice variant isoforms, and CD133.

6. A kit for detecting a circulating tumor cell (CTC) in a biological sample, the kit comprising an antibody to O-cadherin and instructions for use, in particular, wherein the antibody is linked to a fluorescent reporter molecule, radionuclide, enzyme, or magnetic bead.

7. A method of predicting responsiveness of a subject having cancer to a course of treatment, the method comprising:
determining the level or presence of expression of O-cadherin in a sample from the subject to obtain a gene expression pattern or biomarker profile in CTCs for the subject; and
predicting responsiveness of the subject to the cancer drug based on the gene expression pattern or biomarker pattern obtained.

8. A method of targeting delivery of a cancer drug in a subject having cancer comprising administering to the subject the cancer drug linked to an antibody specific for O-cadherin.

9. A method of determining a cancer prognosis in a subject, the method comprising:
determining the level of expression of O-cadherin in a sample from the subject to determine the number of CTCs in the subject and to obtain a gene expression pattern for the subject; and
providing a prognosis to the subject based on the gene expression pattern obtained.

10. The method of any one of claims 7-9, wherein the cancer is selected from prostate, colon, breast cancer, solid tumor malignancies, and sarcomas.

11. A method for monitoring progression of cancer in a subject undergoing therapeutic treatment, the method comprising:
detecting the number of CTCs based on the expression of O-cadherin in a first and a second sample taken from the subject at a first and a second time; and
comparing the first and second levels of expression,
wherein a detected difference in number of CTCs based on the level of expression of O-cadherin in the first and second samples indicates a change in the progression of the cancer.

12. The method of claim 11, wherein an increase in the detected level of O-cadherin in the second sample relative to the first sample indicates progression of the cancer,
wherein a decrease in the detected level of O-cadherin in the second sample relative to the first sample indicates that the therapeutic treatment is effective, and
whereby no difference in the detected level of O-cadherin in the second sample relative to the first sample indicates arrest or stability in the progression of the cancer.

13. The method of claim 12, wherein the decrease indicates remission of the cancer.

14. A method for detecting cancer in a subject, the method comprising:
detecting the presence of O-cadherin in a sample from the subject;
comparing the detected amount of O-cadherin from the sample to a control sample; and
correlating the detected amount of O-cadherin from the sample to the presence of CTCs in the sample,
wherein the presence of CTCs in the sample indicates the presence of cancer in the subject.

15. The method of any one of claims 11-14, wherein the cancer is selected from prostate, colon, and breast cancer.


SEQUENCE LISTING

<110>  DUKE UNIVERSITY

<120>  BIOMARKERS FOR CIRCULATING TUMOR CELLS

<130>  EP83170IFZ268pau

<140>  not yet assigned
<141>  2010-09-24

<150>  EP10844915.8
<151>  2010-09-24

<150>  PCT/US2010/050223
<151>  2010-09-24

<150>  61/298,845
<151>  2010-01-27

<150>  61/308,780
<151>  2010-02-26

<150>  61/309,131
<151>  2010-03-01

<160>  24

<170>  PatentIn version 3.5

<210>  1
<211>  4321
<212>  DNA
<213>  Mus musculus

<400>  1
cacacacaca cgcacacaca cacacacaca cacttctcgg cgcgcacgac gcccgccctt      60

ctccccgccc cctccccagc tccttgatct cccgtctgtt ttattactcc tggtgcgagt     120

ccggcggact ccgaggcccg ctatttgtta ccaactcgct ctcattggcg gggaggagag     180

cagcggagaa gggggtgggg aggggagggg aagggaaggg gtggccactg ccggagccga     240

ctccgcgctg ctgttggtgc cgctgccgct tctgctgcct ctgctgccgc cgccgccgcc     300

tccggctcct cgctcggccc ctctccgcct ccatgtgccg gatagcggga gcgccgcgga     360

ccctgctgcc gcttctggcg gccttgcttc aggcgtctgt ggaggcttct ggtgaaattg     420

cattatgcaa gactggattt cctgaagatg tttacagcgc agtcttaccg aaggatgtgc     480

acgaaggaca gccccttctc aatgtgaaat tcagcaactg caatagaaaa aggaaagttc     540

agtatgaaag cagcgagcca gcagatttca aggtggacga ggacggcacg gtgtatgctg     600

tgagaagctt ccctctcact gcagagcagg caaagttcct gatatatgcc caagacaaag     660

aaacccagga aaagtggcag gtagctgtaa acctgagccg ggagccaacc ctgactgagg     720

agcctatgaa ggaaccacat gaaattgaag aaatagtatt ccctagacaa cttgccaagc     780

acagtggagc tctacaaagg cagaagagag actgggtcat cccgccaatc aacttgccag     840

aaaactccag aggacccttt cctcaagagc ttgtcagaat caggtctgat agagataaaa 900

acctttccct gagatacagc gtcactgggc caggagctga ccagcctcca acgggcatct 960

tcattatcaa ccccatctca ggacagctgt cagtcacaaa gcctctggat cgagagctga 1020

tagcccggtt tcacttgaga gcacatgcag tggacatcaa tggcaatcaa gtggagaacc 1080

ccattgacat tgtcatcaat gttattgaca tgaatgataa cagacctgag tttctgcacc 1140

aggtttggaa tgggtctgtt ccagagggat caaagcctgg gacgtatgtg atgacggtca 1200

ctgccattga tgcggatgat ccaaatgccc tgaatggaat gctgcggtac aggatcctgt 1260

cccaggcgcc cagcacacct tcacccaaca tgtttacaat caacaatgag actggggaca 1320

tcatcactgt ggcagctggt ctggatcgag agaaagtgca acagtatacg ttaataattc 1380

aagccacaga catggaaggc aatcccactt atggcctttc aaacacagcc acagccgtca 1440

tcacggtgac agatgtcaat gacaatcctc cagagtttac tgccatgact ttctacggag 1500

aagtccctga gaacagggtg gacgtcattg tagccaacct aactgtcacg gacaaagatc 1560

agccccacac gccggcctgg aatgcggcat acagaatcag tggtggagac cctacaggaa 1620

ggtttgccat cctgacagac cccaacagca atgatgggct agtcacagtg gtaaaaccaa 1680

ttgactttga aacgaatagg atgtttgtcc ttactgttgc tgcagaaaac caagtgccat 1740

tagctaaagg cattcagcac ccacctcagt cgacagccac tgtgtctgtg acagttattg 1800

atgtcaatga aaatccttat tttgccccaa atcctaaaat cattcgccaa gaggaaggcc 1860

tccacgcagg taccatgctg accacgctca ctgctcagga ccccgatcga tatatgcaac 1920

agaatatcag atacacaaaa ttgtctgatc ctgccaactg gctgaaaata gaccccgtga 1980

atgggcagat cactactatt gccgttttgg acagagaatc gccaaatgta aaaaacaaca 2040

tctataatgc taccttcctt gcttctgaca atggaatccc gcctatgagt gggacaggaa 2100

cactgcaaat ctatttactt gatatcaatg acaacgcccc tcaggtgtta cctcaagagg 2160

cggagacctg tgaaactcca gaacccaact caattaacat cacagcactt gattatgaca 2220

tagacccaaa cgccgggccg ttcgcgtttg atcttccctt atctccagtg actattaaaa 2280

gaaactggac catcaaccgg cttaatggtg attttgctca gctcaattta aagataaaat 2340

ttttggaagc tggtatctat gaagttccca tcattatcac agattcaggg aatcccccca 2400

agtccaacat ttccatcctg cgtgtgaaag tttgtcagtg tgactccaat ggagactgca 2460

cggacgtgga caggatcgtg ggtgcagggc ttggcacggg cgccatcatc gctatccttc 2520

tgtgtatcat catcctgctg atccttgttc tcatgtttgt ggtatggatg aaacggcggg 2580

ataaagagcg ccaagccaag cagcttttaa ttgacccaga agatgatgta agagataata 2640

tattgaaata tgatgaagaa ggtggaggag aagaagacca ggactatgac ttgagccagc 2700

tccagcaacc agatactgtg gagcctgatg ccatcaagcc cgtgggaatc agacggctag 2760

acgagaggcc tatccatgct gagccacagt acccagtccg atccgcagcc ccacaccctg 2820

gggatattgg ggacttcatt aatgagggcc ttaaagctgc tgacaacgac cccacggcgc 2880

caccgtatga ctccctctta gtctttgact acgagggcag cggctccacg gctggctcct 2940

tgagctccct caactcctcc agtagcggtg gggaccagga ctatgactac ctgaatgact 3000

ggggaccccg cttcaagaaa ctggcggaca tgtacggcgg tggtgacgac tgaacggcag 3060

gacggacttg gcttttggac aagtatgaac agtttcacct gatattccca aaaaaaagca 3120

tacagaagct aggctttaac tctgtagtcc actagcaccg tgcttgctgg aggctttggc 3180

gtaggctgcg aaccagtttg ggctcccagg gaatatcagt gatccaatac tgtctggaaa 3240

acaccgagct cagctacact tgaattttac agtaaagaag cactgggatt tatgtgcctt 3300

tttgtacctt tttcagattg gaattagttt tctgtttaag gctttaatgg tactgatttc 3360

tgaaatgata aggaaaagac aaaatatttt gtggcgggag cagaaagtta aatgtgatac 3420

gcttcaaccc acttttgtta caatgcattt gcttttgtta agatacagaa cgaaacaacc 3480

agattaaaaa aaattaactc atggagtgat tttgttacct ttggggtggg ggggatgaga 3540

ccacaagata ggaaaatgta cattacttct agttttagac tttagatttt tttttttcac 3600

taaaatctta aaacttacgc agctggttgc agataaaggg agttttcata tcaccaattt 3660

gtagcaaaat gaattttttc ataaactaga atgttagaca cattttggtc ttaatccatg 3720

tacacttttt tattttctgt attttttcca cctcgctgta aaaatggtgt gtgtacataa 3780

tgtttatcag catagactat ggaggagtgc agagaactcg gaacatgtgt atgtattatt 3840

tggactttgg attcaggttt tttgcatgtt aatatctttc gttatgggta aagtatttac 3900

aaaacaaagt gacatttgat tcaactgttg agctgtagtt agaatactca atttttaatt 3960

ttttaatttt ttttaaattt ttttattttc tttttgtttg tttcgttttg gggaggggta 4020

aaagttctta gcacaatgtt ttacataatt tgtaccaaaa aaattacaca caaaaaaaaa 4080

aaaaagaaaa gaaaagaaaa gtgaaagggg tggcctgttt cttgcagcac tagcaagtgt 4140

gtgtttttaa aaaacaaaac aaacaaacaa aaaaataaat aaaaagagga aaaagaaaaa 4200

aaaaaaagct tttaaactgg agagacttct gaaacagctt tgcgtctgtg ttgtgtacca 4260

gaatacaaac aatacacctc tgaccccagc gttctgaata aaaagctaat tttggatctg 4320

g 4321

<210> 2
<211> 906
<212> PRT
<213> Mus musculus

<400> 2

Met Cys Arg Ile Ala Gly Ala Pro Arg Thr Leu Leu Pro Leu Leu Ala
1               5                   10                  15

Ala Leu Leu Gln Ala Ser Val Glu Ala Ser Gly Glu Ile Ala Leu Cys
            20                  25                  30

Lys Thr Gly Phe Pro Glu Asp Val Tyr Ser Ala Val Leu Pro Lys Asp
        35                  40                  45

Val His Glu Gly Gln Pro Leu Leu Asn Val Lys Phe Ser Asn Cys Asn
    50                  55                  60

Arg Lys Arg Lys Val Gln Tyr Glu Ser Ser Glu Pro Ala Asp Phe Lys
65                  70                  75                  80

Val Asp Glu Asp Gly Thr Val Tyr Ala Val Arg Ser Phe Pro Leu Thr
                85                  90                  95

Ala Glu Gln Ala Lys Phe Leu Ile Tyr Ala Gln Asp Lys Glu Thr Gln
            100                 105                 110

Glu Lys Trp Gln Val Ala Val Asn Leu Ser Arg Glu Pro Thr Leu Thr
        115                 120                 125

Glu Glu Pro Met Lys Glu Pro His Glu Ile Glu Glu Ile Val Phe Pro
    130                 135                 140

Arg Gln Leu Ala Lys His Ser Gly Ala Leu Gln Arg Gln Lys Arg Asp
145                 150                 155                 160

Trp Val Ile Pro Pro Ile Asn Leu Pro Glu Asn Ser Arg Gly Pro Phe
                165                 170                 175

Pro Gln Glu Leu Val Arg Ile Arg Ser Asp Arg Asp Lys Asn Leu Ser
            180                 185                 190

Leu Arg Tyr Ser Val Thr Gly Pro Gly Ala Asp Gln Pro Pro Thr Gly
        195                 200                 205

Ile Phe Ile Ile Asn Pro Ile Ser Gly Gln Leu Ser Val Thr Lys Pro
    210                 215                 220

Leu Asp Arg Glu Leu Ile Ala Arg Phe His Leu Arg Ala His Ala Val
225                 230                 235                 240

Asp Ile Asn Gly Asn Gln Val Glu Asn Pro Ile Asp Ile Val Ile Asn
                245                 250                 255

Val Ile Asp Met Asn Asp Asn Arg Pro Glu Phe Leu His Gln Val Trp
            260                 265                 270

Asn Gly Ser Val Pro Glu Gly Ser Lys Pro Gly Thr Tyr Val Met Thr
        275                 280                 285

Val Thr Ala Ile Asp Ala Asp Asp Pro Asn Ala Leu Asn Gly Met Leu
    290                 295                 300

Arg Tyr Arg Ile Leu Ser Gln Ala Pro Ser Thr Pro Ser Pro Asn Met
305                 310                 315                 320

Phe Thr Ile Asn Asn Glu Thr Gly Asp Ile Ile Thr Val Ala Ala Gly
                325                 330                 335

Leu Asp Arg Glu Lys Val Gln Gln Tyr Thr Leu Ile Ile Gln Ala Thr
            340                 345                 350

Asp Met Glu Gly Asn Pro Thr Tyr Gly Leu Ser Asn Thr Ala Thr Ala
        355                 360                 365

Val Ile Thr Val Thr Asp Val Asn Asp Asn Pro Pro Glu Phe Thr Ala
    370                 375                 380

Met Thr Phe Tyr Gly Glu Val Pro Glu Asn Arg Val Asp Val Ile Val
385                 390                 395                 400

Ala Asn Leu Thr Val Thr Asp Lys Asp Gln Pro His Thr Pro Ala Trp
                405                 410                 415

Asn Ala Ala Tyr Arg Ile Ser Gly Gly Asp Pro Thr Gly Arg Phe Ala
            420                 425                 430

Ile Leu Thr Asp Pro Asn Ser Asn Asp Gly Leu Val Thr Val Val Lys
        435                 440                 445

Pro Ile Asp Phe Glu Thr Asn Arg Met Phe Val Leu Thr Val Ala Ala
    450                 455                 460

Glu Asn Gln Val Pro Leu Ala Lys Gly Ile Gln His Pro Pro Gln Ser
465                 470                 475                 480

Thr Ala Thr Val Ser Val Thr Val Ile Asp Val Asn Glu Asn Pro Tyr
                485                 490                 495

Phe Ala Pro Asn Pro Lys Ile Ile Arg Gln Glu Glu Gly Leu His Ala

500                 505                 510

Gly Thr Met Leu Thr Thr Leu Thr Ala Gln Asp Pro Asp Arg Tyr Met
        515                 520                 525

Gln Gln Asn Ile Arg Tyr Thr Lys Leu Ser Asp Pro Ala Asn Trp Leu
    530                 535                 540

Lys Ile Asp Pro Val Asn Gly Gln Ile Thr Thr Ile Ala Val Leu Asp
545                 550                 555                 560

Arg Glu Ser Pro Asn Val Lys Asn Asn Ile Tyr Asn Ala Thr Phe Leu
                565                 570                 575

Ala Ser Asp Asn Gly Ile Pro Pro Met Ser Gly Thr Gly Thr Leu Gln
            580                 585                 590

Ile Tyr Leu Leu Asp Ile Asn Asp Asn Ala Pro Gln Val Leu Pro Gln
        595                 600                 605

Glu Ala Glu Thr Cys Glu Thr Pro Glu Pro Asn Ser Ile Asn Ile Thr
    610                 615                 620

Ala Leu Asp Tyr Asp Ile Asp Pro Asn Ala Gly Pro Phe Ala Phe Asp
625                 630                 635                 640

Leu Pro Leu Ser Pro Val Thr Ile Lys Arg Asn Trp Thr Ile Asn Arg
                645                 650                 655

Leu Asn Gly Asp Phe Ala Gln Leu Asn Leu Lys Ile Lys Phe Leu Glu
            660                 665                 670

Ala Gly Ile Tyr Glu Val Pro Ile Ile Ile Thr Asp Ser Gly Asn Pro
        675                 680                 685

Pro Lys Ser Asn Ile Ser Ile Leu Arg Val Lys Val Cys Gln Cys Asp
    690                 695                 700

Ser Asn Gly Asp Cys Thr Asp Val Asp Arg Ile Val Gly Ala Gly Leu
705                 710                 715                 720

Gly Thr Gly Ala Ile Ile Ala Ile Leu Leu Cys Ile Ile Ile Leu Leu
                725                 730                 735

Ile Leu Val Leu Met Phe Val Val Trp Met Lys Arg Arg Asp Lys Glu
            740                 745                 750

Arg Gln Ala Lys Gln Leu Leu Ile Asp Pro Glu Asp Asp Val Arg Asp
        755                 760                 765

Asn Ile Leu Lys Tyr Asp Glu Glu Gly Gly Gly Glu Glu Asp Gln Asp
    770                 775                 780

Tyr Asp Leu Ser Gln Leu Gln Gln Pro Asp Thr Val Glu Pro Asp Ala
785                 790                 795                 800

Ile Lys Pro Val Gly Ile Arg Arg Leu Asp Glu Arg Pro Ile His Ala
                805                 810                 815

Glu Pro Gln Tyr Pro Val Arg Ser Ala Ala Pro His Pro Gly Asp Ile
            820                 825                 830

Gly Asp Phe Ile Asn Glu Gly Leu Lys Ala Ala Asp Asn Asp Pro Thr
        835                 840                 845

Ala Pro Pro Tyr Asp Ser Leu Leu Val Phe Asp Tyr Glu Gly Ser Gly
    850                 855                 860

Ser Thr Ala Gly Ser Leu Ser Ser Leu Asn Ser Ser Ser Ser Gly Gly
865                 870                 875                 880

Asp Gln Asp Tyr Asp Tyr Leu Asn Asp Trp Gly Pro Arg Phe Lys Lys
                885                 890                 895

Leu Ala Asp Met Tyr Gly Gly Gly Asp Asp
            900                 905

<210> 3
<211> 3237
<212> DNA
<213> Xenopus laevis

<400> 3
tcggcacgag ctggagtgta caggactttt aagatgctgc tgggtgtctg cactgtgtcc 60

atgtgaatgt ggcattttta ttttgaattc cctccggaga caagatttca tcaagagttt 120

cctttggata ttaagtcaaa gtgcaagcaa tggagattct ctataagaag gcaataatct 180

gggggattta ctaaaattaa acaaacagat tgacattcgc tggatttatc aagcaatttt 240

gcatttacaa cactaccaaa aatgaagaaa gacttttgct tacacggttt acttttatgt 300

ttgggaattg cgtattgtag tcatgccaca tctttaagaa aaaacaataa actaaggcaa 360

tcattccatg gtcaccatga aaaaggcaaa gaagggcaag ttttacatag gtcaaagaga 420

ggatgggttt ggaatcaatt ttttgtaata gaagaataca ccggaccaga tcctgtactc 480

gttggacggc ttcactcaga tgttgactct ggagattgga agataaaata catactctca 540

ggagagggtg ctgggaccat ttttgtcatt gatgacaaat cagggaatat ccatgcaacc 600

aagaccctgg atcgagaaga aagggctcag tataccttaa tggctcaggc agttgacaga 660

gaaacaaata aaccactgga accaccatca gagtttatcg ttaaagttca agacataaat 720

gataatcccc cggagttctt gcatgaaaac taccacgcaa atgtgcctga gatgtccaat 780

gtgggtacat cagtaattca agtaacagcc tctgatgcag atgatccaac atatggaaac 840

agcgctaagc ttgtgtatag tattctcgaa gggcagccat atttttcagt cgaagcacaa 900

tcaggaatca ttaggactgc ccttccaaac atggacagag aagccaagga agaataccat 960

gttgttattc aagcaaagga tatgggagga catatgggag gactctcagg gacaactaaa 1020

gtgacaataa cgctgacaga tgtcaatgac aatccaccaa agtttccaca aagtgcgtac 1080

cccatgtctg tgtcagaagc tgctgtccca ggggaagagg ttggcagaat aaaagctaaa 1140

gatccagaca ttggagaaaa tggcttaata aagtaccgta ttcttgaagg agatggggca 1200

gagatgtttg aaatcacagc tgattatgta actcaggaag gcgttgtaaa gctaaaaaag 1260

gtggtggatt atgaaaccaa gaagttctac agtatgaagg ttgaagctgt caacgttcat 1320

attgatccca gattccttag ccggggacca ttcaaagaca ctgctactgt taagatctca 1380

gtagaggatt ttgatgaacc gcctattttc ttagaaagaa gttacatttt ggaagtatat 1440

gaaaatgctc catcggatac tgtggtcgga agagtgcacg ctaaagaccc agatgctgct 1500

aacagcccaa ttaggtattc aatcgatcgc cacactgacc ttgacagatt cttcagcatc 1560

aacccagagg atggtgtcat caaaaccaca aagggtttgg atagagagga aagcccttgg 1620

cacaacatct cagtcattgc aactgaagtc cacaatcgaa ttcatgaaac tagagttcca 1680

gtagctatta aagtcttgga taagaatgac aatgctccgg aatttgcaaa gccctatgaa 1740

gcttttgtct gtgaaaatgc tccaatcaat caggagtttt tgaccatcac tgcagtagat 1800

aaagatgata cagccaatgg acttcgtttt ctctttagtt tccccccaga aattgtacat 1860

ccaaatccaa atttcaccat aatagacaaa cgagataaca cagcaagcat ccgtgttggc 1920

cgtggagttt tcagccgaca gaaacaagac ttgtatttgg ttcctattgt tataagtgat 1980

gggggaagcc caccgatgag cagcaccaat accctttctg tccgaatctg cagttgcaat 2040

agtgatggat cccaactatc ttgtaatgct gaaccccaat cccttaacgc tggactcagt 2100

actggagcac tgattgcaat ccttgcttgc attgtaattt tattagtgat tgtggttttg 2160

tttgtgactc tgaggagaga gaagaaggaa cctctaattg tctttgaaga ggaagatatc 2220

cgggaaaata taattacata tgatgatgaa ggtggtggag aggaagacac cgaagcattt 2280

gacattgcaa cactgcagaa tcctgatggg attaatggat ttatgccacg gaaagatatc 2340

aaacccgaat ttcaatataa ccccagagat attggaataa gaccagcacc aaacagtgtt 2400

gacgttgatg acttcattaa cacaaggata catgaggccg ataatgaccc tgcagctccg 2460

ccttatgact ccattcagat ctatggatac gaagggagag gttctgtggc tggctctctt 2520

agttcattag agtcagcctc tacagattca gatttggact atgattatct acaaaactgg 2580

ggacctcgat ttaagaaact agcaaattta tatgggtcca aagacacttg tgaagatgat 2640

tcttaacaaa taagttctga atttggcctt atgaactgca taatgtactg aaatatccag 2700

agtaaacatt aacaggtatt tttttaaagg aaaacatgaa aaaggcttct ttaaccttcc 2760

aaggtttaca aacaggattc cttccaaaac aagaactgtt aaatggtggt ggatactgtg 2820

aaaaccctat ggcctgtgta gaagttgtgt attcattttt ttttttgttt tttgtttttt 2880

ttccaagaaa ccacttgtaa aatgcagcct atttaaggga atggaaatgc aggaaaaacg 2940

caacaaaaaa ggggaatctt tacagtatta aacataacca tcaaatcttc tcaaacaaag 3000

cttccacaca aaaaaaaaaa aagataacag ttttgagctg taatttcgcc ttaaactatg 3060

gacactttat atgtagtgca tttttaaact tgaaaaaaat atatatataa tatccagcca 3120

gcttcaatcc atataatgta tgtacagtaa aatgtacaat tattctgtct cttgagcatc 3180

agacttgtta ctgctgattc ttgtaaatct tttttgctta taatcccctc gtgccga 3237

<210> 4
<211> 794
<212> PRT
<213> Xenopus laevis

<400> 4

Met Lys Lys Asp Phe Cys Leu His Gly Leu Leu Leu Cys Leu Gly Ile
1               5                   10                  15

Ala Tyr Cys Ser His Ala Thr Ser Leu Arg Lys Asn Asn Lys Leu Arg
            20                  25                  30

Gln Ser Phe His Gly His His Glu Lys Gly Lys Glu Gly Gln Val Leu
        35                  40                  45

His Arg Ser Lys Arg Gly Trp Val Trp Asn Gln Phe Phe Val Ile Glu
    50                  55                  60

Glu Tyr Thr Gly Pro Asp Pro Val Leu Val Gly Arg Leu His Ser Asp
65                  70                  75                  80

Val Asp Ser Gly Asp Trp Lys Ile Lys Tyr Ile Leu Ser Gly Glu Gly
                85                  90                  95

Ala Gly Thr Ile Phe Val Ile Asp Asp Lys Ser Gly Asn Ile His Ala
            100                 105                 110

Thr Lys Thr Leu Asp Arg Glu Glu Arg Ala Gln Tyr Thr Leu Met Ala
        115                 120                 125

Gln Ala Val Asp Arg Glu Thr Asn Lys Pro Leu Glu Pro Pro Ser Glu
    130                 135                 140

Phe Ile Val Lys Val Gln Asp Ile Asn Asp Asn Pro Pro Glu Phe Leu
145                 150                 155                 160

His Glu Asn Tyr His Ala Asn Val Pro Glu Met Ser Asn Val Gly Thr
                165                 170                 175

Ser Val Ile Gln Val Thr Ala Ser Asp Ala Asp Asp Pro Thr Tyr Gly
            180                 185                 190

Asn Ser Ala Lys Leu Val Tyr Ser Ile Leu Glu Gly Gln Pro Tyr Phe
        195                 200                 205

Ser Val Glu Ala Gln Ser Gly Ile Ile Arg Thr Ala Leu Pro Asn Met
    210                 215                 220

Asp Arg Glu Ala Lys Glu Glu Tyr His Val Val Ile Gln Ala Lys Asp
225                 230                 235                 240

Met Gly Gly His Met Gly Gly Leu Ser Gly Thr Thr Lys Val Thr Ile
                245                 250                 255

Thr Leu Thr Asp Val Asn Asp Asn Pro Pro Lys Phe Pro Gln Ser Ala
            260                 265                 270

Tyr Pro Met Ser Val Ser Glu Ala Ala Val Pro Gly Glu Glu Val Gly
        275                 280                 285

Arg Ile Lys Ala Lys Asp Pro Asp Ile Gly Glu Asn Gly Leu Ile Lys
    290                 295                 300

Tyr Arg Ile Leu Glu Gly Asp Gly Ala Glu Met Phe Glu Ile Thr Ala
305                 310                 315                 320

Asp Tyr Val Thr Gln Glu Gly Val Val Lys Leu Lys Lys Val Val Asp
                325                 330                 335

Tyr Glu Thr Lys Lys Phe Tyr Ser Met Lys Val Glu Ala Val Asn Val
            340                 345                 350

His Ile Asp Pro Arg Phe Leu Ser Arg Gly Pro Phe Lys Asp Thr Ala
        355                 360                 365

Thr Val Lys Ile Ser Val Glu Asp Phe Asp Glu Pro Pro Ile Phe Leu
    370                 375                 380

Glu Arg Ser Tyr Ile Leu Glu Val Tyr Glu Asn Ala Pro Ser Asp Thr
385                 390                 395                 400

Val Val Gly Arg Val His Ala Lys Asp Pro Asp Ala Ala Asn Ser Pro
                405                 410                 415

Ile Arg Tyr Ser Ile Asp Arg His Thr Asp Leu Asp Arg Phe Phe Ser
            420                 425                 430

Ile Asn Pro Glu Asp Gly Val Ile Lys Thr Thr Lys Gly Leu Asp Arg
        435                 440                 445

Glu Glu Ser Pro Trp His Asn Ile Ser Val Ile Ala Thr Glu Val His
    450                 455                 460

Asn Arg Ile His Glu Thr Arg Val Pro Val Ala Ile Lys Val Leu Asp
465                 470                 475                 480

Lys Asn Asp Asn Ala Pro Glu Phe Ala Lys Pro Tyr Glu Ala Phe Val
                485                 490                 495

Cys Glu Asn Ala Pro Ile Asn Gln Glu Phe Leu Thr Ile Thr Ala Val
            500                 505                 510

Asp Lys Asp Asp Thr Ala Asn Gly Leu Arg Phe Leu Phe Ser Phe Pro
        515                 520                 525

Pro Glu Ile Val His Pro Asn Pro Asn Phe Thr Ile Ile Asp Lys Arg
    530                 535                 540

Asp Asn Thr Ala Ser Ile Arg Val Gly Arg Gly Val Phe Ser Arg Gln
545                 550                 555                 560

Lys Gln Asp Leu Tyr Leu Val Pro Ile Val Ile Ser Asp Gly Gly Ser
                565                 570                 575

Pro Pro Met Ser Ser Thr Asn Thr Leu Ser Val Arg Ile Cys Ser Cys
            580                 585                 590

Asn Ser Asp Gly Ser Gln Leu Ser Cys Asn Ala Glu Pro Gln Ser Leu
        595                 600                 605

Asn Ala Gly Leu Ser Thr Gly Ala Leu Ile Ala Ile Leu Ala Cys Ile

610                 615                 620

Val Ile Leu Leu Val Ile Val Val Leu Phe Val Thr Leu Arg Arg Glu
625                 630                 635                 640

Lys Lys Glu Pro Leu Ile Val Phe Glu Glu Glu Asp Ile Arg Glu Asn
                645                 650                 655

Ile Ile Thr Tyr Asp Asp Glu Gly Gly Gly Glu Glu Asp Thr Glu Ala
            660                 665                 670

Phe Asp Ile Ala Thr Leu Gln Asn Pro Asp Gly Ile Asn Gly Phe Met
        675                 680                 685

Pro Arg Lys Asp Ile Lys Pro Glu Phe Gln Tyr Asn Pro Arg Asp Ile
    690                 695                 700

Gly Ile Arg Pro Ala Pro Asn Ser Val Asp Val Asp Asp Phe Ile Asn
705                 710                 715                 720

Thr Arg Ile His Glu Ala Asp Asn Asp Pro Ala Ala Pro Pro Tyr Asp
                725                 730                 735

Ser Ile Gln Ile Tyr Gly Tyr Glu Gly Arg Gly Ser Val Ala Gly Ser
            740                 745                 750

Leu Ser Ser Leu Glu Ser Ala Ser Thr Asp Ser Asp Leu Asp Tyr Asp
        755                 760                 765

Tyr Leu Gln Asn Trp Gly Pro Arg Phe Lys Lys Leu Ala Asn Leu Tyr
    770                 775                 780

Gly Ser Lys Asp Thr Cys Glu Asp Asp Ser
785                 790

<210>  5
<211>  3701
<212>  DNA
<213>  Mus musculus

<400>  5
gtccaatcag tgcgctcaga ctcagagccc taggctcctg ctctttaaat taccgagcct      60

tgtggagacc ccggcacctg gccttaagct cagccctgag gatggtactt tgagtgaatg     120

accaccttgg agaccgttct tctgtttccc ttgttaccag ccaggaggca gaagagtcca     180

ccggtccagg aaagacccat ttcccttgag tttccagaaa gtacctcatg cttgagagat     240

caggccaaca actatggctc tcgtcttcag tgccctgctg ttactggggc tgtgtggaaa     300

gatctcttca gaaggtcagc ctgcattcca taacactcct ggggctatga attatgaatt 360
gcctaccacc aaatatgaga cccaagatac cttcaatgct gggattgttg gccctctcta 420
caaaatggtg cacatcttcc tcaacgtggt ccagccgaat gacttccctc tagatttgat 480
caaaaaactc atacagaaca agaactttga catctcagtt gattccaagg agccagaaat 540
catagtcttg gctctgaaga ttgccctcta tgagatcgga gtccttatct gcgccatcct 600
gggactgctg ttcattatcc tcatgcctct ggtgggctgc ttcttttgta tgtgccgttg 660
ctgcaacaaa tgcggcggag agatgcacca gcggcagaag cagaatgcgc catgcaggag 720
gaagtgcttg ggcctctccc tcctggtgat ttgtctgctc atgagccttg gcattatata 780
tggctttgtg gctaaccagc agaccaggac tcggatcaaa gggacccaga aactggcaaa 840
gagcaatttc agagactttc aaacactcct gactgaaaca ccaaagcaaa ttgactatgt 900
agtggagcag tacaccaaca ccaagaacaa ggcattctca gacctggatg gcatcggctc 960
cgtgctggga ggcagaataa aggaccaact aaaacccaaa gtaactcctg tcctcgaaga 1020
gattaaggcc atggcgacag ccatcaaaca gaccaaggat gccctgcaga acatgagcag 1080
cagcctgaaa agtctccaag atgcagccac ccagctcaat accaacctga gctctgtgag 1140
aaacagcatc gagaattcgc tcagcagcag tgactgtacc tcagatccag ccagcaagat 1200
ctgcgatagc atcagaccaa gcctaagcag tctggggagc agcctcaatt caagtcagct 1260
cccatcagtg gatagagaac tcaacactgt tactgaagtc gacaaaactg atctggagag 1320
cctcgtcaaa agggggtata cgacaattga tgaaataccc aatacaatac aaaaccaaac 1380
tgtggatgtc atcaaagacg tcaaaaatac cttggactcc attagctcca acattaagga 1440
catgagccaa agtattccta ttgaggatat gctgttacag gtctcccatt accttaataa 1500
cagcaacaga tacttaaacc aggagctgcc caagctggaa gaatatgact cgtactggtg 1560
gctgggtggc ttgattgtct gctttctgct gactctcatt gtgaccttct ttttcctggg 1620
cttgctgtgt ggtgtgtttg gctatgacaa gcatgccacc ccaactagaa gaggctgtgt 1680
gtccaacact ggaggcatct tcctcatggc tggggttgga ttcggcttcc ttttttgctg 1740
gatattgatg atccttgtgg ttcttacgtt tgttgttggt gcaaatgtgg aaaagttgct 1800
ctgcgaacct tatgaaaaca agaaattatt acaggttttg gacactccct atctgctcaa 1860
ggaacaatgg caattttatc tttctggcat gctattcaat aacccagaca ttaacatgac 1920
ctttgagcaa gtctacaggg attgcaaaag aggtcgaggt atatatgctg cttttcagct 1980
tgagaatgtc gtcaacgtca gtgatcattt caacattgac cagatttctg aaaacataaa 2040
tacggagttg gaaaacctga atgtgaacat tgatagcatt gaactgttgg ataacacagg 2100
aaggaagagc ctcgaggact ttgcacattc tgggatagat acaatcgatt attccacata 2160
cttgaaggag actgagaaat cccctactga agtgaatctg ctgacatttg cctctaccct 2220

ggaagcaaaa gcaaaccagt tgcctgaagg aaagctgaaa caggccttct tactggatgt 2280

acagaatata agagccatcc accagcatct cctccctcct gtgcagcaat cactgaaatt 2340

tgtgagggtg aggaatacgt taagacaaag tgtctggacc ctccagcaaa caagcaacaa 2400

gttgccggag aaagtgaaga agatccttgc ctctttggac tctgttcagc atttcctcac 2460

caataacgtt tccctcatcg ttatcgggga aacgaagaag tttgggaaaa caatactagg 2520

ctactttgaa cattatctgc actgggtctt ttatgccatc acagagaaga tgacatcctg 2580

caaacccatg gccaccgcga tggactctgc tgttaatggc attctgtgtg gctatgttgc 2640

ggaccctctg aatttgttct ggttcggcat agggaaagcc acggtgctct tacttccggc 2700

tgtaatcatt gctatcaagc tggccaagta ctatcgcagg atggattcag aggatgtata 2760

cgacgacccg tctcgatact gacaactgga gttgaagctg cttgaacaac aagatagtca 2820

acatggaaag catcacagat tttggatagt ttctgagtct tctagaacgt tccaagtgca 2880

gaagaaacct ggtggagact caggcgggca ctaggaacat ggcatcagtg gtcttagggt 2940

agcactttgt caggaatgaa cagtcatcat ggttataatc cacatatcca ttgcaactca 3000

tgaatgattc tctcctgttt tgtttttaac ttttcttttt acactgattt tctatttaga 3060

cactaaaaca tataggggtg cttattcccc ctggatacat ttacctgtga accagctatt 3120

ccggtgtcat agctgggtac ctaacttact tccatatgtg aagtgtgcta aacacaaacc 3180

agtttacaga agagatgtat tttgtgtata gtaaactgta tatataccct tttaccacag 3240

tcagtttttt aaacaaatga atactctaga tttttcttct aaatgaggtt actgttgggg 3300

tggttgtgac ctagtgatgc tgtagaaagg agtctgcatt cactaaaagt gtgtcaacct 3360

agagcaggca atgcccttcc ttgtggattt ctgtctgctc gttttggagc tacctgcggt 3420

ttagaaatag aattcaagaa caatcacgga gtttcccact tgatgccact gccaaagtca 3480

gaacaaggga tcttgagaga aggaactgtc gctcagctgg gagcggaatc attatcgcaa 3540

tcacaggtcc tggttcacag tttagtggca ctctctggtt tgtaagaatg ggcattacgt 3600

tcagtgtcat ctggtcatct gtgatgtgtg tcatcagcct gtcctgatgt tgagatttaa 3660

aataaagcat gaatgaacag aaaaaaaaaa aaaaaaaaaa a 3701

<210> 6
<211> 842
<212> PRT
<213> Mus musculus

<400> 6

Met Ala Leu Val Phe Ser Ala Leu Leu Leu Leu Gly Leu Cys Gly Lys
1               5                   10                  15

Ile Ser Ser Glu Gly Gln Pro Ala Phe His Asn Thr Pro Gly Ala Met
            20                  25                  30

Asn Tyr Glu Leu Pro Thr Thr Lys Tyr Glu Thr Gln Asp Thr Phe Asn
        35                  40                  45

Ala Gly Ile Val Gly Pro Leu Tyr Lys Met Val His Ile Phe Leu Asn
    50                  55                  60

Val Val Gln Pro Asn Asp Phe Pro Leu Asp Leu Ile Lys Lys Leu Ile
65                  70                  75                  80

Gln Asn Lys Asn Phe Asp Ile Ser Val Asp Ser Lys Glu Pro Glu Ile
                85                  90                  95

Ile Val Leu Ala Leu Lys Ile Ala Leu Tyr Glu Ile Gly Val Leu Ile
            100                 105                 110

Cys Ala Ile Leu Gly Leu Leu Phe Ile Ile Leu Met Pro Leu Val Gly
        115                 120                 125

Cys Phe Phe Cys Met Cys Arg Cys Cys Asn Lys Cys Gly Gly Glu Met
    130                 135                 140

His Gln Arg Gln Lys Gln Asn Ala Pro Cys Arg Arg Lys Cys Leu Gly
145                 150                 155                 160

Leu Ser Leu Leu Val Ile Cys Leu Leu Met Ser Leu Gly Ile Ile Tyr
                165                 170                 175

Gly Phe Val Ala Asn Gln Gln Thr Arg Thr Arg Ile Lys Gly Thr Gln
            180                 185                 190

Lys Leu Ala Lys Ser Asn Phe Arg Asp Phe Gln Thr Leu Leu Thr Glu
        195                 200                 205

Thr Pro Lys Gln Ile Asp Tyr Val Val Glu Gln Tyr Thr Asn Thr Lys
    210                 215                 220

Asn Lys Ala Phe Ser Asp Leu Asp Gly Ile Gly Ser Val Leu Gly Gly
225                 230                 235                 240

Arg Ile Lys Asp Gln Leu Lys Pro Lys Val Thr Pro Val Leu Glu Glu
                245                 250                 255

Ile Lys Ala Met Ala Thr Ala Ile Lys Gln Thr Lys Asp Ala Leu Gln
            260                 265                 270

Asn Met Ser Ser Ser Leu Lys Ser Leu Gln Asp Ala Ala Thr Gln Leu
        275                 280                 285

Asn Thr Asn Leu Ser Ser Val Arg Asn Ser Ile Glu Asn Ser Leu Ser
    290                 295                 300

Ser Ser Asp Cys Thr Ser Asp Pro Ala Ser Lys Ile Cys Asp Ser Ile
305                 310                 315                 320

Arg Pro Ser Leu Ser Ser Leu Gly Ser Ser Leu Asn Ser Ser Gln Leu
                325                 330                 335

Pro Ser Val Asp Arg Glu Leu Asn Thr Val Thr Glu Val Asp Lys Thr
            340                 345                 350

Asp Leu Glu Ser Leu Val Lys Arg Gly Tyr Thr Thr Ile Asp Glu Ile
        355                 360                 365

Pro Asn Thr Ile Gln Asn Gln Thr Val Asp Val Ile Lys Asp Val Lys
    370                 375                 380

Asn Thr Leu Asp Ser Ile Ser Ser Asn Ile Lys Asp Met Ser Gln Ser
385                 390                 395                 400

Ile Pro Ile Glu Asp Met Leu Leu Gln Val Ser His Tyr Leu Asn Asn
                405                 410                 415

Ser Asn Arg Tyr Leu Asn Gln Glu Leu Pro Lys Leu Glu Glu Tyr Asp
            420                 425                 430

Ser Tyr Trp Trp Leu Gly Gly Leu Ile Val Cys Phe Leu Leu Thr Leu
        435                 440                 445

Ile Val Thr Phe Phe Phe Leu Gly Leu Leu Cys Gly Val Phe Gly Tyr
    450                 455                 460

Asp Lys His Ala Thr Pro Thr Arg Arg Gly Cys Val Ser Asn Thr Gly
465                 470                 475                 480

Gly Ile Phe Leu Met Ala Gly Val Gly Phe Gly Phe Leu Phe Cys Trp
                485                 490                 495

Ile Leu Met Ile Leu Val Val Leu Thr Phe Val Val Gly Ala Asn Val
            500                 505                 510

Glu Lys Leu Leu Cys Glu Pro Tyr Glu Asn Lys Lys Leu Leu Gln Val
        515                 520                 525

Leu Asp Thr Pro Tyr Leu Leu Lys Glu Gln Trp Gln Phe Tyr Leu Ser
    530                 535                 540

Gly Met Leu Phe Asn Asn Pro Asp Ile Asn Met Thr Phe Glu Gln Val
545                 550                 555                 560

Tyr Arg Asp Cys Lys Arg Gly Arg Gly Ile Tyr Ala Ala Phe Gln Leu
                565                 570                 575

Glu Asn Val Val Asn Val Ser Asp His Phe Asn Ile Asp Gln Ile Ser
            580                 585                 590

Glu Asn Ile Asn Thr Glu Leu Glu Asn Leu Asn Val Asn Ile Asp Ser
        595                 600                 605

Ile Glu Leu Leu Asp Asn Thr Gly Arg Lys Ser Leu Glu Asp Phe Ala
    610                 615                 620

His Ser Gly Ile Asp Thr Ile Asp Tyr Ser Thr Tyr Leu Lys Glu Thr
625                 630                 635                 640

Glu Lys Ser Pro Thr Glu Val Asn Leu Leu Thr Phe Ala Ser Thr Leu
                645                 650                 655

Glu Ala Lys Ala Asn Gln Leu Pro Glu Gly Lys Leu Lys Gln Ala Phe
            660                 665                 670

Leu Leu Asp Val Gln Asn Ile Arg Ala Ile His Gln His Leu Leu Pro
        675                 680                 685

Pro Val Gln Gln Ser Leu Lys Phe Val Arg Val Arg Asn Thr Leu Arg
    690                 695                 700

Gln Ser Val Trp Thr Leu Gln Gln Thr Ser Asn Lys Leu Pro Glu Lys
705                 710                 715                 720

Val Lys Lys Ile Leu Ala Ser Leu Asp Ser Val Gln His Phe Leu Thr
                725                 730                 735

Asn Asn Val Ser Leu Ile Val Ile Gly Glu Thr Lys Lys Phe Gly Lys
            740                 745                 750

Thr Ile Leu Gly Tyr Phe Glu His Tyr Leu His Trp Val Phe Tyr Ala
        755                 760                 765

Ile Thr Glu Lys Met Thr Ser Cys Lys Pro Met Ala Thr Ala Met Asp

770                 775                 780

Ser Ala Val Asn Gly Ile Leu Cys Gly Tyr Val Ala Asp Pro Leu Asn
785                 790                 795                 800

Leu Phe Trp Phe Gly Ile Gly Lys Ala Thr Val Leu Leu Leu Pro Ala
                805                 810                 815

Val Ile Ile Ala Ile Lys Leu Ala Lys Tyr Tyr Arg Arg Met Asp Ser
            820                 825                 830

Glu Asp Val Tyr Asp Asp Pro Ser Arg Tyr
        835                 840

<210>  7
<211>  3306
<212>  DNA
<213>  Mus musculus

<400>  7
gaattcccgc gcggccgcca gagctccggc ccgggggctg cctgtgtgtt cctggcccgg      60

cgtggcgact gctctccggg ctggcggggg ccgggcgtga gcccgggcct cagcgttcct     120

gagcgctgcg agtgttcact actcgccagc aaagtttgga gtaggcaacg caagctccag     180

tcctttcttc tgctgctgcc cagatccgag agcagctccg gtgtatgtct agctgttctg     240

cgatcccggc gcgcgtgaag cctcggaacc ttggcgccgg ctgctaccca aggaatcgtt     300

ctctttttgg agttttcctc cgagatcatc gcctgctcca tcccgatcca ctctgggctc     360

cggcgcagca ccgagcgcag aggagcgctg ccattcaagt ggcagccaca gcagcagcag     420

cagcagcagt gggagcagga acagcagtaa caacagcaac agcagcacag ccgcctcaga     480

gctttgctcc tgagcccctg tgggctgaag gcattgcagg tagcccatgg tctcagaaga     540

agtgtgcaga tgggattacc gtccacgtgg agatatggaa gaggaccagg gattggcact     600

gtgaccatgg tcagctgggg gcgcttcatc tgcctggtct tggtcaccat ggcaaccttg     660

tccctggccc ggccctcctt cagtttagtt gaggatacca ctttagaacc agaagagcca     720

ccaaccaaat accaaatctc ccaaccagaa gcgtacgtgg ttgcccccgg ggaatcgcta     780

gagttgcagt gcatgttgaa agatgccgcc gtgatcagtt ggactaagga tggggtgcac     840

ttggggccca acaataggac agtgcttatt ggggagtatc tccagataaa aggtgccaca     900

cctagagact ccggcctcta tgcttgtact gcagctagga cggtagacag tgaaacttgg     960

atcttcatgg tgaatgtcac agatgccatc tcatctggag atgatgagga cgacacagat    1020

agctccgaag acgttgtcag tgagaacagg agcaaccaga gagcaccgta ctggaccaac    1080

accgagaaga tggagaagcg gctccacgct tgtcctgccg ccaacactgt gaagttccgc    1140

tgtccggctg gggggaatcc aacgtccaca atgaggtggt taaaaaacgg gaaggagttt 1200

aagcaggagc atcgcattgg aggctataag gtacgaaacc agcactggag ccttattatg 1260

gaaagtgtgg tcccgtcaga caaaggcaac tacacctgcc tggtggagaa tgaatacggg 1320

tccatcaacc acacctacca cctggatgtc gttgaacgtt caccacaccg tcccatcctc 1380

caagctggac tgcctgcaaa tgcctccacg gtggtcggag gggatgtgga gtttgtctgc 1440

aaggtttaca gcgatgccca gccccacatc cagtggatca agcacgtgga aaagaacggc 1500

agtaaaaacg ggcctgatgg gctgccctac ctcaaggttc tgaaagctgc cggtgttaac 1560

accacggaca aagagattga ggttctctat attcggaatg taacttttga ggatgctggg 1620

gaatatacgt gcttggcggg taattctatc gggatatcct ttcactctgc atggttgaca 1680

gttctgccag cgcctgtgag agagaaggag atcacggctt ccccagatta tctggagata 1740

gctatttact gcataggggt cttcttaatc gcctgcatgg tggtgacagt catcttttgc 1800

cgaatgaaga ccacgaccaa gaagccagac ttcagcagcc agccagctgt gcacaagctg 1860

accaagcgca tccccctgcg gagacaggta acagtttcgg ccgagtccag ctcctccatg 1920

aactccaaca ccccgctggt gaggataaca acgcgtctgt cctcaacagc ggacaccccg 1980

atgctagcag gggtctccga gtatgagttg ccagaggatc caaagtggga attccccaga 2040

gataagctga cgctgggcaa acccctgggg gaaggttgct tcgggcaagt agtcatggct 2100

gaagcagtgg gaatcgataa agacaaaccc aaggaggcgg tcaccgtggc agtgaagatg 2160

ttgaaagatg atgccacaga gaaggacctg tctgatctgg tatcagagat ggagatgatg 2220

aagatgattg ggaaacataa gaacattatc aacctcctgg gggcctgcac gcaggatgga 2280

cctctctacg tcatagttga atatgcatcg aaaggcaacc tccgggaata cctccgagcc 2340

cggaggccac ctggcatgga gtactcctat gacattaacc gtgtccccga ggagcagatg 2400

accttcaagg acttggtgtc ctgcacctac cagctggcta gaggcatgga gtacttggct 2460

tcccaaaaat gtatccatcg agatttggct gccagaaacg tgttggtaac agaaaacaat 2520

gtgatgaaga tagcagactt tggcctggcc agggatatca acaacataga ctactataaa 2580

aagaccacaa atgggcgact tccagtcaag tggatggctc ctgaagccct tttgatagaa 2640

gtttacactc atcagagcga tgtctggtcc ttcggggtgt taatgtggga gatctttact 2700

ttagggggct caccctaccc agggattccc gtggaggaac tttttaagct gctcaaagag 2760

ggacacagga tggacaagcc caccaactgc accaatgaac tgtacatgat gatgagggat 2820

tgctggcatg ctgtaccctc acagagaccc acattcaagc agttggtcga agacttggat 2880

cgaattctga ctctcacaac caatgaggaa tacttggatc tcacccagcc tctcgaacag 2940

tattctccta gttaccccga cacaagtagc tcttgttctt caggggacga ttctgtgttt 3000

tctccagacc ccatgcctta tgaaccctgt ctgcctcagt atccacacat aaacggcagt 3060

gttaaaacat gagtgaatgt gtcttcctgt ccccaaacag gacagcacca ggaacctact 3120

tacactgagc agagaggctg tgctccagag cctgtgacac gcctccactt gtatatatgg 3180

atcagaggag taaatagtgg gaagcatatt tgtcacgtgt gtaaagattt atacagttgg 3240

aacatgtact acaggaagga gactgttctg atagtgacag ccgccaccat gccacctttg 3300

accaca 3306

<210> 8
<211> 821
<212> PRT
<213> Mus musculus

<400> 8

Met Val Ser Trp Gly Arg Phe Ile Cys Leu Val Leu Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Glu Pro Pro Thr Lys Tyr Gln Ile Ser Gln Pro Glu
        35                  40                  45

Ala Tyr Val Val Ala Pro Gly Glu Ser Leu Glu Leu Gln Cys Met Leu
    50                  55                  60

Lys Asp Ala Ala Val Ile Ser Trp Thr Lys Asp Gly Val His Leu Gly
65                  70                  75                  80

Pro Asn Asn Arg Thr Val Leu Ile Gly Glu Tyr Leu Gln Ile Lys Gly
                85                  90                  95

Ala Thr Pro Arg Asp Ser Gly Leu Tyr Ala Cys Thr Ala Ala Arg Thr
            100                 105                 110

Val Asp Ser Glu Thr Trp Ile Phe Met Val Asn Val Thr Asp Ala Ile
        115                 120                 125

Ser Ser Gly Asp Asp Glu Asp Asp Thr Asp Ser Ser Glu Asp Val Val
    130                 135                 140

Ser Glu Asn Arg Ser Asn Gln Arg Ala Pro Tyr Trp Thr Asn Thr Glu
145                 150                 155                 160

Lys Met Glu Lys Arg Leu His Ala Cys Pro Ala Ala Asn Thr Val Lys
                165                 170                 175

Phe Arg Cys Pro Ala Gly Gly Asn Pro Thr Ser Thr Met Arg Trp Leu
            180                 185                 190

Lys Asn Gly Lys Glu Phe Lys Gln Glu His Arg Ile Gly Gly Tyr Lys
        195                 200                 205

Val Arg Asn Gln His Trp Ser Leu Ile Met Glu Ser Val Val Pro Ser
    210                 215                 220

Asp Lys Gly Asn Tyr Thr Cys Leu Val Glu Asn Glu Tyr Gly Ser Ile
225                 230                 235                 240

Asn His Thr Tyr His Leu Asp Val Val Glu Arg Ser Pro His Arg Pro
                245                 250                 255

Ile Leu Gln Ala Gly Leu Pro Ala Asn Ala Ser Thr Val Val Gly Gly
            260                 265                 270

Asp Val Glu Phe Val Cys Lys Val Tyr Ser Asp Ala Gln Pro His Ile
        275                 280                 285

Gln Trp Ile Lys His Val Glu Lys Asn Gly Ser Lys Asn Gly Pro Asp
    290                 295                 300

Gly Leu Pro Tyr Leu Lys Val Leu Lys Ala Ala Gly Val Asn Thr Thr
305                 310                 315                 320

Asp Lys Glu Ile Glu Val Leu Tyr Ile Arg Asn Val Thr Phe Glu Asp
                325                 330                 335

Ala Gly Glu Tyr Thr Cys Leu Ala Gly Asn Ser Ile Gly Ile Ser Phe
            340                 345                 350

His Ser Ala Trp Leu Thr Val Leu Pro Ala Pro Val Arg Glu Lys Glu
        355                 360                 365

Ile Thr Ala Ser Pro Asp Tyr Leu Glu Ile Ala Ile Tyr Cys Ile Gly
    370                 375                 380

Val Phe Leu Ile Ala Cys Met Val Val Thr Val Ile Phe Cys Arg Met
385                 390                 395                 400

Lys Thr Thr Thr Lys Lys Pro Asp Phe Ser Ser Gln Pro Ala Val His
                405                 410                 415

Lys Leu Thr Lys Arg Ile Pro Leu Arg Arg Gln Val Thr Val Ser Ala
            420                 425                 430

Glu Ser Ser Ser Ser Met Asn Ser Asn Thr Pro Leu Val Arg Ile Thr
        435                 440                 445

Thr Arg Leu Ser Ser Thr Ala Asp Thr Pro Met Leu Ala Gly Val Ser
    450                 455                 460

Glu Tyr Glu Leu Pro Glu Asp Pro Lys Trp Glu Phe Pro Arg Asp Lys
465                 470                 475                 480

Leu Thr Leu Gly Lys Pro Leu Gly Glu Gly Cys Phe Gly Gln Val Val
                485                 490                 495

Met Ala Glu Ala Val Gly Ile Asp Lys Asp Lys Pro Lys Glu Ala Val
            500                 505                 510

Thr Val Ala Val Lys Met Leu Lys Asp Asp Ala Thr Glu Lys Asp Leu
        515                 520                 525

Ser Asp Leu Val Ser Glu Met Glu Met Met Lys Met Ile Gly Lys His
    530                 535                 540

Lys Asn Ile Ile Asn Leu Leu Gly Ala Cys Thr Gln Asp Gly Pro Leu
545                 550                 555                 560

Tyr Val Ile Val Glu Tyr Ala Ser Lys Gly Asn Leu Arg Glu Tyr Leu
                565                 570                 575

Arg Ala Arg Arg Pro Pro Gly Met Glu Tyr Ser Tyr Asp Ile Asn Arg
            580                 585                 590

Val Pro Glu Glu Gln Met Thr Phe Lys Asp Leu Val Ser Cys Thr Tyr
        595                 600                 605

Gln Leu Ala Arg Gly Met Glu Tyr Leu Ala Ser Gln Lys Cys Ile His
    610                 615                 620

Arg Asp Leu Ala Ala Arg Asn Val Leu Val Thr Glu Asn Asn Val Met
625                 630                 635                 640

Lys Ile Ala Asp Phe Gly Leu Ala Arg Asp Ile Asn Asn Ile Asp Tyr
                645                 650                 655

Tyr Lys Lys Thr Thr Asn Gly Arg Leu Pro Val Lys Trp Met Ala Pro
            660                 665                 670

Glu Ala Leu Phe Asp Arg Val Tyr Thr His Gln Ser Asp Val Trp Ser
        675                 680                 685

Phe Gly Val Leu Met Trp Glu Ile Phe Thr Leu Gly Gly Ser Pro Tyr
    690                 695                 700

Pro Gly Ile Pro Val Glu Glu Leu Phe Lys Leu Leu Lys Glu Gly His
705                 710                 715                 720

Arg Met Asp Lys Pro Thr Asn Cys Thr Asn Glu Leu Tyr Met Met Met
                725                 730                 735

Arg Asp Cys Trp His Ala Val Pro Ser Gln Arg Pro Thr Phe Lys Gln
            740                 745                 750

Leu Val Glu Asp Leu Asp Arg Ile Leu Thr Leu Thr Thr Asn Glu Glu
        755                 760                 765

Tyr Leu Asp Leu Thr Gln Pro Leu Glu Gln Tyr Ser Pro Ser Tyr Pro
    770                 775                 780

Asp Thr Ser Ser Ser Cys Ser Ser Gly Asp Asp Ser Val Phe Ser Pro
785                 790                 795                 800

Asp Pro Met Pro Tyr Glu Pro Cys Leu Pro Gln Tyr Pro His Ile Asn
                805                 810                 815

Gly Ser Val Lys Thr
            820

<210>  9
<211>  3037
<212>  DNA
<213>  Mus musculus

<400>  9
ggcgagggga gagagccggg agaggcgagc ggcggcgcgg caggcgcgga acgggcgcac      60

ggacgatcga acgcgcggcc gccagagctc cggcgcgggg gctgcctgtg tgttcctggc     120

ccggcgtggc gactgctctc cgggctggcg ggggccgggc gtgagcccgg gcctcagcgt     180

tcctgagcgc tgcgagtgtt cactactcgc cagcaaagtt tggagtaggc aacgccaagc     240

tccagtcctt tcttctgctg ctgcccagat ccgagagcag ctccggtgtc atgtcctagc     300

tgttctgcga tccccggcgc gcgtgaagcc tcggaacctt cgcgccggct gctacccaag     360

gaatcgttct ctttttggag ttttcctccg agatcatcgc ctgctccatc ccgatccact     420

ctgggctccg gcgcagaccg agcgcagagg agcgctgcca ttcaagtggc agccacagca     480

gcagcagcag cagcagtggg agcaggaaca gcagtaacaa cagcaacagc agcacagccg     540

cctcagagct ttggctcctg agccccctgt gggctgaagg cattgcaggt agcccatggt     600

ctcagaagaa gtgtgcagat gggattaccg tccacgtgga gatatggaag aggaccaggg 660
attggcactg tgaccatggt cagctggggg cgcttcatct gcctggtctt ggtcaccatg 720
gcaaccttgt ccctggcccg gccctccttc agtttagttg aggataccac tttagaacca 780
gaaggagcac cgtactggac caacaccgag aagatggaga agcggctcca cgctgtccct 840
gccgccaaca ctgtgaagtt ccgctgtccg gctgggggga atccaacgcc cacaatgagg 900
tggttaaaaa acgggaagga gtttaagcag gagcatcgca ttggaggcta taaggtacga 960
aaccagcact ggagccttat tatggaaagt gtggtcccgt cagacaaagg caactacacc 1020
tgcctggtgg agaatgaata cgggtccatc aaccacacct accacctcga tgtcgttgaa 1080
cggtcaccac accggcccat cctccaagct ggactgcctg caaatgcctc cacggtggtc 1140
ggaggggatg tggagtttgt ctgcaaggtt tacagcgatg cccagcccca catccagtgg 1200
atcaagcacg tggaaaagaa cggcagtaaa tacgggcctg atgggctgcc ctacctcaag 1260
gtcctgaagc actcggggat aaatagctcc aatgcagaag tgctggctct gttcaatgtg 1320
acggagatgg atgctgggga atatatatgt aaggtctcca attatatagg gcaggccaac 1380
cagtctgcct ggctcactgt cctgcccaaa cagcaagcgc ctgtgagaga gaaggagatc 1440
acggcttccc cagattatct ggagatagct atttactgca taggggtctt cttaatcgcc 1500
tgcatggtgg tgacagtcat cttttgccga atgaagacca cgaccaagaa gccagacttc 1560
agcagccagc cagctgtgca caagctgacc aagcgcatcc ccctgcggag acaggtaaca 1620
gtttcggccg agtccagctc ctccatgaac tccaacaccc cgctggtgag gataacaacg 1680
cgtctgtcct caacagcgga cacccccatg ctagcagggg tctccgagta tgagttgcca 1740
gaggatccaa agtgggaatt ccccagagat aagctgacgc tgggcaaacc cctgggggaa 1800
ggttgcttcg ggcaagtagt catggctgaa gcagtgggaa tcgataaaga caaacccaag 1860
gaggcggtca ccgtggcagt gaagatgttg aaagatgatg ccacagagaa ggacctgtct 1920
gatctggtat cagagatgga gatgatgaag atgattggga aacataagaa cattatcaac 1980
ctcctggggg cctgcacgca ggatggacct ctctacgtca tagttgaata tgcatcgaaa 2040
ggcaacctcc gggaatacct ccgagcccgg aggccacctg gcatggagta ctcctatgac 2100
attaaccgtg tccccgagga gcagatgacc ttcaaggact tggtgtcctg cacctaccag 2160
ctggctagag gcatggagta cttggcttcc caaaaatgta tccatcgaga tttggctgcc 2220
agaaacgtgt tggtaacaga aaacaatgtg atgaagatag cagactttgg cctggccagg 2280
gatatcaaca acatagacta ctataaaaag accacaaatg ggcgacttcc agtcaagtgg 2340
atggctcctg aagccctttt tgatagagtt tacactcatc agagcgatgt ctggtccttc 2400
ggggtgttaa tgtgggagat ctttacttta gggggctcac cctacccagg gattcccgtg 2460

gaggaacttt ttaagctgct caaagaggga cacaggatgg acaagcccac caactgcacc 2520

aatgaactgt acatgatgat gagggattgc tggcatgctg taccctcaca gagacccaca 2580

ttcaagcagt tggtcgaaga cttggatcga attctgactc tcacaaccaa tgaggaatac 2640

ttggatctca cccagcctct cgaacagtat tctcctagtt accccgacac aaggagctct 2700

tgttcttcag gggacgattc tgtgttttct ccagacccca tgccttatga accctgtctg 2760

cctcagtatc cacacataaa cggcagtgtt aaaacatgag tgaatgtgtc ttcctgtccc 2820

caaacaggac agcaccagga acctacttac actgagcaga gaggctgtct cagagcctgt 2880

gacacgcctc cacttgtata tatggatcag aggagtaaat agtgggaagc atattgtcac 2940

gtgtgtaaag atttatacag ttcggaaaca tgttacctaa ccaggaaagg aagactgttt 3000

tcctgataag tggacagccg caagccacca tgccacc 3037

<210> 10
<211> 707
<212> PRT
<213> Mus musculus

<400> 10

Met Val Ser Trp Gly Arg Phe Ile Cys Leu Val Leu Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Gly Ala Pro Tyr Trp Thr Asn Thr Glu Lys Met Glu
        35                  40                  45

Lys Arg Leu His Ala Val Pro Ala Ala Asn Thr Val Lys Phe Arg Cys
    50                  55                  60

Pro Ala Gly Gly Asn Pro Thr Pro Thr Met Arg Trp Leu Lys Asn Gly
65                  70                  75                  80

Lys Glu Phe Lys Gln Glu His Arg Ile Gly Gly Tyr Lys Val Arg Asn
                85                  90                  95

Gln His Trp Ser Leu Ile Met Glu Ser Val Val Pro Ser Asp Lys Gly
            100                 105                 110

Asn Tyr Thr Cys Leu Val Glu Asn Glu Tyr Gly Ser Ile Asn His Thr
        115                 120                 125

Tyr His Leu Asp Val Val Glu Arg Ser Pro His Arg Pro Ile Leu Gln
    130                 135                 140

Ala Gly Leu Pro Ala Asn Ala Ser Thr Val Val Gly Gly Asp Val Glu
145                 150                 155                 160

Phe Val Cys Lys Val Tyr Ser Asp Ala Gln Pro His Ile Gln Trp Ile
                165                 170                 175

Lys His Val Glu Lys Asn Gly Ser Lys Tyr Gly Pro Asp Gly Leu Pro
            180                 185                 190

Tyr Leu Lys Val Leu Lys His Ser Gly Ile Asn Ser Ser Asn Ala Glu
        195                 200                 205

Val Leu Ala Leu Phe Asn Val Thr Glu Met Asp Ala Gly Glu Tyr Ile
    210                 215                 220

Cys Lys Val Ser Asn Tyr Ile Gly Gln Ala Asn Gln Ser Ala Trp Leu
225                 230                 235                 240

Thr Val Leu Pro Lys Gln Gln Ala Pro Val Arg Glu Lys Glu Ile Thr
                245                 250                 255

Ala Ser Pro Asp Tyr Leu Glu Ile Ala Ile Tyr Cys Ile Gly Val Phe
            260                 265                 270

Leu Ile Ala Cys Met Val Val Thr Val Ile Phe Cys Arg Met Lys Thr
        275                 280                 285

Thr Thr Lys Lys Pro Asp Phe Ser Ser Gln Pro Ala Val His Lys Leu
    290                 295                 300

Thr Lys Arg Ile Pro Leu Arg Arg Gln Val Thr Val Ser Ala Glu Ser
305                 310                 315                 320

Ser Ser Ser Met Asn Ser Asn Thr Pro Leu Val Arg Ile Thr Thr Arg
                325                 330                 335

Leu Ser Ser Thr Ala Asp Thr Pro Met Leu Ala Gly Val Ser Glu Tyr
            340                 345                 350

Glu Leu Pro Glu Asp Pro Lys Trp Glu Phe Pro Arg Asp Lys Leu Thr
        355                 360                 365

Leu Gly Lys Pro Leu Gly Glu Gly Cys Phe Gly Gln Val Val Met Ala
    370                 375                 380

Glu Ala Val Gly Ile Asp Lys Asp Lys Pro Lys Glu Ala Val Thr Val
385                 390                 395                 400

Ala Val Lys Met Leu Lys Asp Asp Ala Thr Glu Lys Asp Leu Ser Asp
                405                 410                 415

Leu Val Ser Glu Met Glu Met Met Lys Met Ile Gly Lys His Lys Asn
            420                 425                 430

Ile Ile Asn Leu Leu Gly Ala Cys Thr Gln Asp Gly Pro Leu Tyr Val
        435                 440                 445

Ile Val Glu Tyr Ala Ser Lys Gly Asn Leu Arg Glu Tyr Leu Arg Ala
    450                 455                 460

Arg Arg Pro Pro Gly Met Glu Tyr Ser Tyr Asp Ile Asn Arg Val Pro
465                 470                 475                 480

Glu Glu Gln Met Thr Phe Lys Asp Leu Val Ser Cys Thr Tyr Gln Leu
                485                 490                 495

Ala Arg Gly Met Glu Tyr Leu Ala Ser Gln Lys Cys Ile His Arg Asp
            500                 505                 510

Leu Ala Ala Arg Asn Val Leu Val Thr Glu Asn Asn Val Met Lys Ile
        515                 520                 525

Ala Asp Phe Gly Leu Ala Arg Asp Ile Asn Asn Ile Asp Tyr Tyr Lys
    530                 535                 540

Lys Thr Thr Asn Gly Arg Leu Pro Val Lys Trp Met Ala Pro Glu Ala
545                 550                 555                 560

Leu Phe Asp Arg Val Tyr Thr His Gln Ser Asp Val Trp Ser Phe Gly
                565                 570                 575

Val Leu Met Trp Glu Ile Phe Thr Leu Gly Gly Ser Pro Tyr Pro Gly
            580                 585                 590

Ile Pro Val Glu Glu Leu Phe Lys Leu Leu Lys Glu Gly His Arg Met
        595                 600                 605

Asp Lys Pro Thr Asn Cys Thr Asn Glu Leu Tyr Met Met Met Arg Asp
    610                 615                 620

Cys Trp His Ala Val Pro Ser Gln Arg Pro Thr Phe Lys Gln Leu Val
625                 630                 635                 640

Glu Asp Leu Asp Arg Ile Leu Thr Leu Thr Thr Asn Glu Glu Tyr Leu

645                 650                 655

Asp Leu Thr Gln Pro Leu Glu Gln Tyr Ser Pro Ser Tyr Pro Asp Thr
            660                 665                 670

Arg Ser Ser Cys Ser Ser Gly Asp Asp Ser Val Phe Ser Pro Asp Pro
        675                 680                 685

Met Pro Tyr Glu Pro Cys Leu Pro Gln Tyr Pro His Ile Asn Gly Ser
    690                 695                 700

Val Lys Thr
705

<210>  11
<211>  3344
<212>  DNA
<213>  Xenopus tropicalis

<400>  11
agagcaggga agtacagcgc tgcgctacaa gaactgagca aacgagcaga aaagtacaca      60

ttcctgatcc ttcggtcttt ccaaaagtcc ccaatggggt cacacaggcc atggttactt     120

ggtgctgtgg tgctgctggc actccttcag gtacagggag gactggcaga atggacacag     180

tgtcaaatgg gattttccaa ggaaaggtac agcttttcgg tacctaagaa cttggagaca     240

gacaaagcac tgggtagagt gatctttaac agctgtgagg gaccagtgag aattcagttt     300

gcctctaaag atcctaattt tgaaattcac aaagatggca cagtttatgt taagaatcct     360

accaagatga aagacaacag aaaaacattc cgtgtcctgg cttgggagaa tcaaggtcat     420

gtatactcta ccagtgtaac cttgaaaggg gaagggcatc accataagca ggacatttct     480

tctgtgaaac attcccacca cccaaaatct gagactggtt taaaaagaca aaaaagagac     540

tgggtgattc caccaatcgt aacatctgag aatgaaaagg gcccatttcc caaacggctt     600

gtgcagatca agtccagtaa tgcaaaggaa atcaaggttt tttacagtat cacaggccag     660

ggtgccgata cccctccaga aggagtgttc actattggac gggaggatgg atggctaaat     720

gtgacacgac ctttggacag agaagccatt gatagttaca ctcttttttc tcatgctgtg     780

tcagtaaatg ggcaaaatgt ggaagatccc atggaaatcc aaattaaagt acaagatcag     840

aatgataatg acccagtttt cacacaggag gtctttgaag gctatgtgcc tgaagggtct     900

aagccaggta cgcccgtcat gactgtatct gcaacagatg ccgatgatgc tatagacatg     960

tacaatggtg tgattactta ctccattctc aaccaagacc ctaaagagcc caacaatcaa    1020

atgttcacta ttgattccca gtctgggttg atcagcgtag ttacaactgg attagacaga    1080

gagaaaatac cagtgtacac actgactatt caagctgcag atggagaatt tgggaaagat    1140

cgcacaacaa ctgcaaaagc tgtgatcatt gtgacagaca ccaatgataa ccctcctgtg 1200

tttaacccaa cgcaatacat tgcagaggtt cctgaaaatg aagttggata tgaggttgca 1260

cgtcttacgg taacagatgc agatattgaa gggtcagatg cctggaatgc tgtgtacaag 1320

atcattaaag gaaatgaggc tggctttttc agcatccaaa cagatattga caacattggg 1380

ctactgaaaa cagtgaaggg tctggactat gagctgaaga agcagtatat tctgtcagtc 1440

attgtgacaa acaaagctaa cttttctgtt ccactacaaa cttcaactgc aacggtcact 1500

gtaactgtca cagatgtgaa tgaggcccca gtatttgtac cagtgttgaa agacgtgtct 1560

gtgccagagg atctgcccag tggccaagtt gttgctacct ataccgcaca ggatccagac 1620

aaggaacaga accagaaaat aagttacttc attggaaatg acccagcagg gtgggtgtct 1680

gtgaacagag ataatgggat tgtcactgga aatggaaact tggatcggga atcaaagttt 1740

gtgctaaaca acacctacaa agtcataatc ttggccgctg acagtggcac tccttctgcc 1800

actgggactg gaacccttgt gcttaatctc attgatgtta atgataatgg cccatttttg 1860

gatccccaac aaaatagttt ctgccagaag gatccaggct ttcgtgtatt taatatcatt 1920

gacaaagatc tttaccctaa cacataccca tatacagtag acctgactgg tgaatccaat 1980

gaaaactgga ctgctacagt gacagaacag agtttacttg agctgagacc taaaaaggaa 2040

ctggatattg gacgatacga agttttgatc tcattgagag acaatcaggg actgacagat 2100

gtgacaaagc tacagattac aatctgtcaa tgtaatggtg accaaatgca atgtgaggaa 2160

aaggctgctc aagcaggagg tttggggata tcagccatag ttggaatcct tggagggatc 2220

ctagcgcttc ttttattgtt gttgctgctc ttactgtttg tacgacgaaa gaaagtggta 2280

aaagaacctt tattaccacc agaagatgag actcgggaca atgtattttt ctatgatgaa 2340

gaaggcggtg gtgaggaaga ccaggatttt gatctaagcc agcttcaccg tggtctagat 2400

gctcgtccag atataatccg taatgatgtc gttccagttt tagctgctcc ccagtatcga 2460

ccccgtcctg ccaatccaga tgaaattgga aatttcattg atgagaactt gcatgcagct 2520

gacaatgacc ccactgctcc tccatacgac tcgctccttg tgttcgatta cgaaggcagt 2580

ggctctgagg ccgcatcact cagctctctt aactcttcca actctgattt agatcaggat 2640

tacagtgctt tgaataactg gggacctcgt ttcaccaaac tggcagaaat gtatggagga 2700

gatgaggatt agaatgtgca ctgcaatacc attttgattc taaacagtaa actaaaaacc 2760

ataattgtgt atgcagtctt tggaattcac tttgttttct cctgctctta aaacagagat 2820

aaggactgct caaaagttac tcctcctgct tttgtaaaat cgttcaaaaa tattttatgt 2880

atatgtatat atgaaaaaat cgtatttttt gtactatttg tgttcttata tccctgcaat 2940

ttgtaataca agaggatctt tatctgctta attataaata taaaatgccc gatatgattc 3000

actatgattt taatgtgttg agaaatcttt ttttaaaaag gtttccagac acctgacgct 3060

tggaagggaa ttccataaaa atataattga attgggggga gattgtgttt tgccatggtc 3120

tgatatacat tttcatatat atacatatga tcattcacag agtacagtca acatttggaa 3180

tttgatgagc ttgctggtcg aactgaaaaa aaaatgtatt atagctgggg taaaaattaa 3240

tgtatgagct aaatggggca caattttgat atctctgcat ttgtatttta cttggcatgt 3300

atacttttgt aataaaataa agatatacat taatatacaa cata 3344

<210> 12
<211> 872
<212> PRT
<213> Xenopus tropicalis

<400> 12

Met Gly Ser His Arg Pro Trp Leu Leu Gly Ala Val Val Leu Leu Ala
1               5                   10                  15

Leu Leu Gln Val Gln Gly Gly Leu Ala Glu Trp Thr Gln Cys Gln Met
            20                  25                  30

Gly Phe Ser Lys Glu Arg Tyr Ser Phe Ser Val Pro Lys Asn Leu Glu
        35                  40                  45

Thr Asp Lys Ala Leu Gly Arg Val Ile Phe Asn Ser Cys Glu Gly Pro
    50                  55                  60

Val Arg Ile Gln Phe Ala Ser Lys Asp Pro Asn Phe Glu Ile His Lys
65                  70                  75                  80

Asp Gly Thr Val Tyr Val Lys Asn Pro Thr Lys Met Lys Asp Asn Arg
                85                  90                  95

Lys Thr Phe Arg Val Leu Ala Trp Glu Asn Gln Gly His Val Tyr Ser
            100                 105                 110

Thr Ser Val Thr Leu Lys Gly Glu Gly His His His Lys Gln Asp Ile
        115                 120                 125

Ser Ser Val Lys His Ser His His Pro Lys Ser Glu Thr Gly Leu Lys
    130                 135                 140

Arg Gln Lys Arg Asp Trp Val Ile Pro Pro Ile Val Thr Ser Glu Asn
145                 150                 155                 160

Glu Lys Gly Pro Phe Pro Lys Arg Leu Val Gln Ile Lys Ser Ser Asn
                165                 170                 175

Ala Lys Glu Ile Lys Val Phe Tyr Ser Ile Thr Gly Gln Gly Ala Asp
            180                 185                 190

Thr Pro Pro Glu Gly Val Phe Thr Ile Gly Arg Glu Asp Gly Trp Leu
        195                 200                 205

Asn Val Thr Arg Pro Leu Asp Arg Glu Ala Ile Asp Ser Tyr Thr Leu
    210                 215                 220

Phe Ser His Ala Val Ser Val Asn Gly Gln Asn Val Glu Asp Pro Met
225                 230                 235                 240

Glu Ile Gln Ile Lys Val Gln Asp Gln Asn Asp Asn Asp Pro Val Phe
                245                 250                 255

Thr Gln Glu Val Phe Glu Gly Tyr Val Pro Glu Gly Ser Lys Pro Gly
            260                 265                 270

Thr Pro Val Met Thr Val Ser Ala Thr Asp Ala Asp Asp Ala Ile Asp
        275                 280                 285

Met Tyr Asn Gly Val Ile Thr Tyr Ser Ile Leu Asn Gln Asp Pro Lys
    290                 295                 300

Glu Pro Asn Asn Gln Met Phe Thr Ile Asp Ser Gln Ser Gly Leu Ile
305                 310                 315                 320

Ser Val Val Thr Thr Gly Leu Asp Arg Glu Lys Ile Pro Val Tyr Thr
                325                 330                 335

Leu Thr Ile Gln Ala Ala Asp Gly Glu Phe Gly Lys Asp Arg Thr Thr
            340                 345                 350

Thr Ala Lys Ala Val Ile Ile Val Thr Asp Thr Asn Asp Asn Pro Pro
        355                 360                 365

Val Phe Asn Pro Thr Gln Tyr Ile Ala Glu Val Pro Glu Asn Glu Val
    370                 375                 380

Gly Tyr Glu Val Ala Arg Leu Thr Val Thr Asp Ala Asp Ile Glu Gly
385                 390                 395                 400

Ser Asp Ala Trp Asn Ala Val Tyr Lys Ile Ile Lys Gly Asn Glu Ala
                405                 410                 415

Gly Phe Phe Ser Ile Gln Thr Asp Ile Asp Asn Ile Gly Leu Leu Lys
            420                 425                 430

Thr Val Lys Gly Leu Asp Tyr Glu Leu Lys Lys Gln Tyr Ile Leu Ser
        435                 440                 445

Val Ile Val Thr Asn Lys Ala Asn Phe Ser Val Pro Leu Gln Thr Ser
    450                 455                 460

Thr Ala Thr Val Thr Val Thr Val Thr Asp Val Asn Glu Ala Pro Val
465                 470                 475                 480

Phe Val Pro Val Leu Lys Asp Val Ser Val Pro Glu Asp Leu Pro Ser
                485                 490                 495

Gly Gln Val Val Ala Thr Tyr Thr Ala Gln Asp Pro Asp Lys Glu Gln
            500                 505                 510

Asn Gln Lys Ile Ser Tyr Phe Ile Gly Asn Asp Pro Ala Gly Trp Val
        515                 520                 525

Ser Val Asn Arg Asp Asn Gly Ile Val Thr Gly Asn Gly Asn Leu Asp
    530                 535                 540

Arg Glu Ser Lys Phe Val Leu Asn Asn Thr Tyr Lys Val Ile Ile Leu
545                 550                 555                 560

Ala Ala Asp Ser Gly Thr Pro Ser Ala Thr Gly Thr Gly Thr Leu Val
                565                 570                 575

Leu Asn Leu Ile Asp Val Asn Asp Asn Gly Pro Phe Leu Asp Pro Gln
            580                 585                 590

Gln Asn Ser Phe Cys Gln Lys Asp Pro Gly Phe Arg Val Phe Asn Ile
        595                 600                 605

Ile Asp Lys Asp Leu Tyr Pro Asn Thr Tyr Pro Tyr Thr Val Asp Leu
    610                 615                 620

Thr Gly Glu Ser Asn Glu Asn Trp Thr Ala Thr Val Thr Glu Gln Ser
625                 630                 635                 640

Leu Leu Glu Leu Arg Pro Lys Lys Glu Leu Asp Ile Gly Arg Tyr Glu
                645                 650                 655

Val Leu Ile Ser Leu Arg Asp Asn Gln Gly Leu Thr Asp Val Thr Lys
            660                 665                 670

Leu Gln Ile Thr Ile Cys Gln Cys Asn Gly Asp Gln Met Gln Cys Glu
        675                 680                 685

Glu Lys Ala Ala Gln Ala Gly Gly Leu Gly Ile Ser Ala Ile Val Gly
    690                 695                 700

Ile Leu Gly Gly Ile Leu Ala Leu Leu Leu Leu Leu Leu Leu Leu Leu
705                 710                 715                 720

Leu Phe Val Arg Arg Lys Lys Val Val Lys Glu Pro Leu Leu Pro Pro
                725                 730                 735

Glu Asp Glu Thr Arg Asp Asn Val Phe Phe Tyr Asp Glu Glu Gly Gly
            740                 745                 750

Gly Glu Glu Asp Gln Asp Phe Asp Leu Ser Gln Leu His Arg Gly Leu
        755                 760                 765

Asp Ala Arg Pro Asp Ile Ile Arg Asn Asp Val Val Pro Val Leu Ala
    770                 775                 780

Ala Pro Gln Tyr Arg Pro Arg Pro Ala Asn Pro Asp Glu Ile Gly Asn
785                 790                 795                 800

Phe Ile Asp Glu Asn Leu His Ala Ala Asp Asn Asp Pro Thr Ala Pro
                805                 810                 815

Pro Tyr Asp Ser Leu Leu Val Phe Asp Tyr Glu Gly Ser Gly Ser Glu
            820                 825                 830

Ala Ala Ser Leu Ser Ser Leu Asn Ser Ser Asn Ser Asp Leu Asp Gln
        835                 840                 845

Asp Tyr Ser Ala Leu Asn Asn Trp Gly Pro Arg Phe Thr Lys Leu Ala
    850                 855                 860

Glu Met Tyr Gly Gly Asp Glu Asp
865                 870

<210>  13
<211>  1862
<212>  DNA
<213>  Homo sapiens

<400>  13
gtccccgcgc cagagacgca gccgcgctcc caccacccac acccaccgcg ccctcgttcg      60

cctcttctcc gggagccagt ccgcgccacc gccgccgccc aggccatcgc caccctccgc     120

agccatgtcc accaggtccg tgtcctcgtc ctcctaccgc aggatgttcg gcggcccggg     180

caccgcgagc cggccgagct ccagccggag ctacgtgact acgtccaccc gcacctacag     240

cctgggcagc gcgctgcgcc ccagcaccag ccgcagcctc tacgcctcgt ccccgggcgg 300

cgtgtatgcc acgcgctcct ctgccgtgcg cctgcggagc agcgtgcccg gggtgcggct 360

cctgcaggac tcggtggact tctcgctggc cgacgccatc aacaccgagt tcaagaacac 420

ccgcaccaac gagaaggtgg agctgcagga gctgaatgac cgcttcgcca actacatcga 480

caaggtgcgc ttcctggagc agcagaataa gatcctgctg gccgagctcg agcagctcaa 540

gggccaaggc aagtcgcgcc tgggggacct ctacgaggag gagatgcggg agctgcgccg 600

gcaggtggac cagctaacca acgacaaagc ccgcgtcgag gtggagcgcg acaacctggc 660

cgaggacatc atgcgcctcc gggagaaatt gcaggaggag atgcttcaga gagaggaagc 720

cgaaaacacc ctgcaatctt tcagacagga tgttgacaat gcgtctctgg cacgtcttga 780

ccttgaacgc aaagtggaat ctttgcaaga agagattgcc tttttgaaga aactccacga 840

agaggaaatc caggagctgc aggctcagat tcaggaacag catgtccaaa tcgatgtgga 900

tgtttccaag cctgacctca cggctgccct gcgtgacgta cgtcagcaat atgaaagtgt 960

ggctgccaag aacctgcagg aggcagaaga atggtacaaa tccaagtttg ctgacctctc 1020

tgaggctgcc aaccggaaca atgacgccct gcgccaggca aagcaggagt ccactgagta 1080

ccggagacag gtgcagtccc tcacctgtga agtggatgcc cttaaaggaa ccaatgagtc 1140

cctggaacgc cagatgcgtg aaatggaaga gaactttgcc gttgaagctg ctaactacca 1200

agacactatt ggccgcctgc aggatgagat tcagaatatg aaggaggaaa tggctcgtca 1260

ccttcgtgaa taccaagacc tgctcaatgt taagatggcc cttgacattg agattgccac 1320

ctacaggaag ctgctggaag gcgaggagag caggatttct ctgcctcttc caaacttttc 1380

ctccctgaac ctgagggaaa ctaatctgga ttcactccct ctggttgata cccactcaaa 1440

aaggacactt ctgattaaga cggttgaaac tagagatgga caggttatca acgaaacttc 1500

tcagcatcac gatgaccttg aataaaaatt gcacacactc agtgcagcaa tatattacca 1560

gcaagaataa aaaagaaatc catatcttaa agaaacagct ttcaagtgcc tttctgcagt 1620

ttttcaggag cgcaagatag atttggaata ggaataagct ctagttctta acaaccgaca 1680

ctcctacaag atttagaaaa aagtttacaa cataatctag tttacagaaa aatcttgtgc 1740

tagaatactt tttaaaaggt attttgaata ccattaaaac tgcttttttt tttccagcaa 1800

gtatccaacc aacttggttc tgcttcaata aatctttgga aaaactcaaa aaaaaaaaaa 1860

aa 1862

<210> 14
<211> 466
<212> PRT
<213> Homo sapiens

<400>  14

Met Ser Thr Arg Ser Val Ser Ser Ser Ser Tyr Arg Arg Met Phe Gly
1               5                   10                  15

Gly Pro Gly Thr Ala Ser Arg Pro Ser Ser Ser Arg Ser Tyr Val Thr
            20                  25                  30

Thr Ser Thr Arg Thr Tyr Ser Leu Gly Ser Ala Leu Arg Pro Ser Thr
        35                  40                  45

Ser Arg Ser Leu Tyr Ala Ser Ser Pro Gly Gly Val Tyr Ala Thr Arg
    50                  55                  60

Ser Ser Ala Val Arg Leu Arg Ser Ser Val Pro Gly Val Arg Leu Leu
65                  70                  75                  80

Gln Asp Ser Val Asp Phe Ser Leu Ala Asp Ala Ile Asn Thr Glu Phe
                85                  90                  95

Lys Asn Thr Arg Thr Asn Glu Lys Val Glu Leu Gln Glu Leu Asn Asp
            100                 105                 110

Arg Phe Ala Asn Tyr Ile Asp Lys Val Arg Phe Leu Glu Gln Gln Asn
        115                 120                 125

Lys Ile Leu Leu Ala Glu Leu Glu Gln Leu Lys Gly Gln Gly Lys Ser
    130                 135                 140

Arg Leu Gly Asp Leu Tyr Glu Glu Glu Met Arg Glu Leu Arg Arg Gln
145                 150                 155                 160

Val Asp Gln Leu Thr Asn Asp Lys Ala Arg Val Glu Val Glu Arg Asp
                165                 170                 175

Asn Leu Ala Glu Asp Ile Met Arg Leu Arg Glu Lys Leu Gln Glu Glu
            180                 185                 190

Met Leu Gln Arg Glu Glu Ala Glu Asn Thr Leu Gln Ser Phe Arg Gln
        195                 200                 205

Asp Val Asp Asn Ala Ser Leu Ala Arg Leu Asp Leu Glu Arg Lys Val
    210                 215                 220

Glu Ser Leu Gln Glu Glu Ile Ala Phe Leu Lys Lys Leu His Glu Glu
225                 230                 235                 240

Glu Ile Gln Glu Leu Gln Ala Gln Ile Gln Glu Gln His Val Gln Ile

                245                 250                 255

Asp Val Asp Val Ser Lys Pro Asp Leu Thr Ala Ala Leu Arg Asp Val
            260                 265                 270

Arg Gln Gln Tyr Glu Ser Val Ala Ala Lys Asn Leu Gln Glu Ala Glu
        275                 280                 285

Glu Trp Tyr Lys Ser Lys Phe Ala Asp Leu Ser Glu Ala Ala Asn Arg
    290                 295                 300

Asn Asn Asp Ala Leu Arg Gln Ala Lys Gln Glu Ser Thr Glu Tyr Arg
305                 310                 315                 320

Arg Gln Val Gln Ser Leu Thr Cys Glu Val Asp Ala Leu Lys Gly Thr
                325                 330                 335

Asn Glu Ser Leu Glu Arg Gln Met Arg Glu Met Glu Glu Asn Phe Ala
            340                 345                 350

Val Glu Ala Ala Asn Tyr Gln Asp Thr Ile Gly Arg Leu Gln Asp Glu
        355                 360                 365

Ile Gln Asn Met Lys Glu Glu Met Ala Arg His Leu Arg Glu Tyr Gln
    370                 375                 380

Asp Leu Leu Asn Val Lys Met Ala Leu Asp Ile Glu Ile Ala Thr Tyr
385                 390                 395                 400

Arg Lys Leu Leu Glu Gly Glu Glu Ser Arg Ile Ser Leu Pro Leu Pro
                405                 410                 415

Asn Phe Ser Ser Leu Asn Leu Arg Glu Thr Asn Leu Asp Ser Leu Pro
            420                 425                 430

Leu Val Asp Thr His Ser Lys Arg Thr Leu Leu Ile Lys Thr Val Glu
        435                 440                 445

Thr Arg Asp Gly Gln Val Ile Asn Glu Thr Ser Gln His His Asp Asp
    450                 455                 460

Leu Glu
465

<210> 15
<211> 2721
<212> DNA
<213> Homo sapiens

<400> 15
atgtgccgga tagcgggagc gctgcggacc ctgctgccgc tgctggcggc cctgcttcag 60

gcgtctgtag aggcttctgg tgaaatcgca ttatgcaaga ctggatttcc tgaagatgtt 120

tacagtgcag tcttatcgaa ggatgtgcat gaaggacagc ctcttctcaa tgtgaagttt 180

agcaactgca atggaaaaag aaaagtacaa tatgagagca gtgagcctgc agattttaag 240

gtggatgaag atggcatggt gtatgccgtg agaagctttc cactctcttc tgagcatgcc 300

aagttcctga tatatgccca agacaaagag acccaggaaa agtggcaagt ggcagtaaaa 360

ttgagcctga agccaacctt aactgaggag tcagtgaagg agtcagcaga agttgaagaa 420

atagtgttcc caagacaatt cagtaagcac agtggccacc tacaaaggca gaagagagac 480

tgggtcatcc ctccaatcaa cttgccagaa aactccaggg gaccttttcc tcaagagctt 540

gtcaggatca ggtctgatag agataaaaac ctttcactgc ggtacagtgt aactgggcca 600

ggagctgacc agcctccaac tggtatcttc attatcaacc ccatctcggg tcagctgtcg 660

gtgacaaagc ccctggatcg cgagcagata gcccggtttc atttgagggc acatgcagta 720

gatattaatg gaaatcaagt ggagaacccc attgacattg tcatcaatgt tattgacatg 780

aatgacaaca gacctgagtt cttacaccag gtttggaatg ggacagttcc tgagggatca 840

aagcctggaa catatgtgat gaccgtaaca gcaattgatg ctgacgatcc caatgccctc 900

aatgggatgt tgaggtacag aatcgtgtct caggctccaa gcaccccttc acccaacatg 960

tttacaatca acaatgagac tggtgacatc atcacagtgg cagctggact tgatcgagaa 1020

aaagtgcaac agtatacgtt aataattcaa gctacagaca tggaaggcaa tcccacatat 1080

ggcctttcaa acacagccac ggccgtcatc acagtgacag atgtcaatga caatcctcca 1140

gagtttactg ccatgacgtt ttatggtgaa gttcctgaga acagggtaga catcatagta 1200

gctaatctaa ctgtgaccga taaggatcaa ccccatacac cagcctggaa cgcagtgtac 1260

agaatcagtg gcggagatcc tactggacgg ttcgccatcc agaccgaccc aaacagcaac 1320

gacgggttag tcaccgtggt caaaccaatc gactttgaaa caaataggat gtttgtcctt 1380

actgttgctg cagaaaatca agtgccatta gccaagggaa ttcagcaccc ccctcagtca 1440

actgcaaccg tgtctgttac agttattgac gtaaatgaaa acccttattt tgcccccaat 1500

cctaagatca ttcgccaaga agaagggctt catgccggta ccatgttgac aacattcact 1560

gctcaggacc cagatcgata tatgcagcaa aatattagat acactaaatt atctgatcct 1620

gccaattggc taaaaataga tcctgtgaat ggacaaataa ctacaattgc tgttttggac 1680

cgagaatcac caaatgtgaa aaacaatata tataatgcta ctttccttgc ttctgacaat 1740

ggaattcctc ctatgagtgg aacaggaacg ctgcagatct atttacttga tattaatgac 1800

aatgcccctc aagtgttacc tcaagaggca gagacttgcg aaactccaga ccccaattca 1860

attaatatta cagcacttga ttatgacatt gatccaaatg ctggaccatt tgcttttgat 1920

cttcctttat ctccagtgac tattaagaga aattggacca tcactcggct taatggtgat 1980

tttgctcagc ttaatttaaa gataaaattt cttgaagctg gtatctatga agttcccatc 2040

ataatcacag attcgggtaa tcctcccaaa tcaaatattt ccatcctgcg tgtgaaggtt 2100

tgccagtgtg actccaacgg ggactgcaca gatgtggaca ggattgtggg tgcggggctt 2160

ggcaccggtg ccatcattgc catcctgctc tgcatcatca tcctgcttat ccttgtgctg 2220

atgtttgtgg tatggatgaa acgccgggat aaagaacgcc aggccaaaca acttttaatt 2280

gatccagaag atgatgtaag agataatatt ttaaaatatg atgaagaagg tggaggagaa 2340

gaagaccagg actatgactt gagccagctg cagcagcctg acactgtgga gcctgatgcc 2400

atcaagcctg tgggaatccg acgaatggat gaaagaccca tccacgccga gccccagtat 2460

ccggtccgat ctgcagcccc acaccctgga gacattgggg acttcattaa tgagggcctt 2520

aaagcggctg acaatgaccc cacagctcca ccatatgact ccctgttagt gtttgactat 2580

gaaggcagtg gctccactgc tgggtccttg agctccctta attcctcaag tagtggtggt 2640

gagcaggact atgattacct gaacgactgg gggccacggt tcaagaaact tgctgacatg 2700

tatggtggag gtgatgactg a 2721

<210> 16
<211> 906
<212> PRT
<213> Homo sapiens

<400> 16

<210>  17
<211>  2391
<212>  DNA
<213>  Homo sapiens

<400>  17
atgaaggaga actactgttt acaagccgcc ctggtgtgcc tgggcatgct gtgccacagc      60

catgcctttg ccccagagcg gcgggggcac ctgcggccct ccttccatgg gcaccatgag     120

aagggcaagg aggggcaggt gctacagcgc tccaagcgtg gctgggtctg gaaccagttc     180

ttcgtgatag aggagtacac cgggcctgac cccgtgcttg tgggcaggct tcattcagat     240

attgactctg gtgatgggaa cattaaatac attctctcag gggaaggagc tggaaccatt     300

tttgtgattg atgacaaatc agggaacatt catgccacca agacgttgga tcgagaagag     360

agagcccagt acacgttgat ggctcaggcg gtggacaggg acaccaatcg gccactggag     420

ccaccgtcgg aattcattgt caaggtccag gacattaatg acaaccctcc ggagttcctg     480

cacgagacct atcatgccaa cgtgcctgag aggtccaatg tgggaacgtc agtaatccag     540

gtgacagctt cagatgcaga tgaccccact tatggaaata gcgccaagtt agtgtacagt     600

atcctcgaag gacaacccta tttttcggtg gaagcacaga caggtatcat cagaacagcc     660

ctacccaaca tggacaggga ggccaaggag gagtaccacg tggtgatcca ggccaaggac     720

atgggtggac atatgggcgg actctcaggg acaaccaaag tgacgatcac actgaccgat     780

gtcaatgaca acccaccaaa gtttccgcag agcgtatacc agatgtctgt gtcagaagca     840

gccgtccctg gggaggaagt aggaagagtg aaagctaaag atccagacat tggagaaaat     900

ggcttagtca catacaatat tgttgatgga gatggtatgg aatcgtttga aatcacaacg     960

gactatgaaa cacaggaggg ggtgataaag ctgaaaaagc ctgtagattt tgaaaccaaa    1020

agagcctata gcttgaaggt agaggcagcc aacgtgcaca tcgacccgaa gtttatcagc    1080

aatggccctt tcaaggacac tgtgaccgtc aagatctcag tagaagatgc tgatgagccc    1140

cctatgttct tggccccaag ttacatccac gaagtccaag aaaatgcagc tgctggcacc    1200

gtggttggga gagtgcatgc caaagaccct gatgctgcca acagcccgat aaggtattcc    1260

atcgatcgtc acactgacct cgacagattt ttcactatta atccagagga tggttttatt 1320

aaaactacaa aacctctgga tagagaggaa acagcctggc tcaacatcac tgtctttgca 1380

gcagaaatcc acaatcggca tcaggaagcc aaagtcccag tggccattag ggtccttgat 1440

gtcaacgata atgctcccaa gtttgctgcc ccttatgaag gtttcatctg tgagagtgat 1500

cagaccaagc cactttccaa ccagccaatt gttacaatta gtgcagatga caaggatgac 1560

acggccaatg gaccaagatt tatcttcagc ctaccccctg aaatcattca caatccaaat 1620

ttcacagtca gagacaaccg agataacaca gcaggcgtgt acgcccggcg tggagggttc 1680

agtcggcaga agcaggactt gtaccttctg cccatagtga tcagcgatgg cggcatcccg 1740

cccatgagta gcaccaacac cctcaccatc aaagtctgcg ggtgcgacgt gaacggggca 1800

ctgctctcct gcaacgcaga ggcctacatt ctgaacgccg gcctgagcac aggcgccctg 1860

atcgccatcc tcgcctgcat cgtcattctc ctggtcattg tagtattgtt tgtgaccctg 1920

agaaggcaaa agaaagaacc actcattgtc tttgaggaag aagatgtccg tgagaacatc 1980

attacttatg atgatgaagg gggtggggaa gaagacacag aagcctttga tattgccacc 2040

ctccagaatc ctgatggtat caatggattt atcccccgca aagacatcaa acctgagtat 2100

cagtacatgc ctagacctgg gctccggcca gcgcccaaca gcgtggatgt cgatgacttc 2160

atcaacacga gaatacagga ggcagacaat gaccccacgg ctcctcctta tgactccatt 2220

caaatctacg gttatgaagg caggggctca gtggccgggt ccctgagctc cctagagtcg 2280

gccaccacag attcagactt ggactatgat tatctacaga actggggacc tcgttttaag 2340

aaactagcag atttgtatgg ttccaaagac acttttgatg acgattctta a 2391

<210> 18
<211> 796
<212> PRT
<213> Homo sapiens

<400> 18

Met Lys Glu Asn Tyr Cys Leu Gln Ala Ala Leu Val Cys Leu Gly Met
1               5                   10                  15

Leu Cys His Ser His Ala Phe Ala Pro Glu Arg Arg Gly His Leu Arg
            20                  25                  30

Pro Ser Phe His Gly His His Glu Lys Gly Lys Glu Gly Gln Val Leu
        35                  40                  45

Gln Arg Ser Lys Arg Gly Trp Val Trp Asn Gln Phe Phe Val Ile Glu
    50                  55                  60

Glu Tyr Thr Gly Pro Asp Pro Val Leu Val Gly Arg Leu His Ser Asp

65                  70                  75                  80

Ile Asp Ser Gly Asp Gly Asn Ile Lys Tyr Ile Leu Ser Gly Glu Gly
                85                  90                  95

Ala Gly Thr Ile Phe Val Ile Asp Asp Lys Ser Gly Asn Ile His Ala
            100                 105                 110

Thr Lys Thr Leu Asp Arg Glu Glu Arg Ala Gln Tyr Thr Leu Met Ala
        115                 120                 125

Gln Ala Val Asp Arg Asp Thr Asn Arg Pro Leu Glu Pro Pro Ser Glu
    130                 135                 140

Phe Ile Val Lys Val Gln Asp Ile Asn Asp Asn Pro Pro Glu Phe Leu
145                 150                 155                 160

His Glu Thr Tyr His Ala Asn Val Pro Glu Arg Ser Asn Val Gly Thr
                165                 170                 175

Ser Val Ile Gln Val Thr Ala Ser Asp Ala Asp Asp Pro Thr Tyr Gly
            180                 185                 190

Asn Ser Ala Lys Leu Val Tyr Ser Ile Leu Glu Gly Gln Pro Tyr Phe
        195                 200                 205

Ser Val Glu Ala Gln Thr Gly Ile Ile Arg Thr Ala Leu Pro Asn Met
    210                 215                 220

Asp Arg Glu Ala Lys Glu Glu Tyr His Val Val Ile Gln Ala Lys Asp
225                 230                 235                 240

Met Gly Gly His Met Gly Gly Leu Ser Gly Thr Thr Lys Val Thr Ile
                245                 250                 255

Thr Leu Thr Asp Val Asn Asp Asn Pro Pro Lys Phe Pro Gln Ser Val
            260                 265                 270

Tyr Gln Met Ser Val Ser Glu Ala Ala Val Pro Gly Glu Glu Val Gly
        275                 280                 285

Arg Val Lys Ala Lys Asp Pro Asp Ile Gly Glu Asn Gly Leu Val Thr
    290                 295                 300

Tyr Asn Ile Val Asp Gly Asp Gly Met Glu Ser Phe Glu Ile Thr Thr
305                 310                 315                 320

Asp Tyr Glu Thr Gln Glu Gly Val Ile Lys Leu Lys Lys Pro Val Asp
                325                 330                 335

Phe Glu Thr Lys Arg Ala Tyr Ser Leu Lys Val Glu Ala Ala Asn Val
            340                 345                 350

His Ile Asp Pro Lys Phe Ile Ser Asn Gly Pro Phe Lys Asp Thr Val
        355                 360                 365

Thr Val Lys Ile Ser Val Glu Asp Ala Asp Glu Pro Pro Met Phe Leu
    370                 375                 380

Ala Pro Ser Tyr Ile His Glu Val Gln Glu Asn Ala Ala Ala Gly Thr
385                 390                 395                 400

Val Val Gly Arg Val His Ala Lys Asp Pro Asp Ala Ala Asn Ser Pro
                405                 410                 415

Ile Arg Tyr Ser Ile Asp Arg His Thr Asp Leu Asp Arg Phe Phe Thr
            420                 425                 430

Ile Asn Pro Glu Asp Gly Phe Ile Lys Thr Thr Lys Pro Leu Asp Arg
        435                 440                 445

Glu Glu Thr Ala Trp Leu Asn Ile Thr Val Phe Ala Ala Glu Ile His
    450                 455                 460

Asn Arg His Gln Glu Ala Lys Val Pro Val Ala Ile Arg Val Leu Asp
465                 470                 475                 480

Val Asn Asp Asn Ala Pro Lys Phe Ala Ala Pro Tyr Glu Gly Phe Ile
                485                 490                 495

Cys Glu Ser Asp Gln Thr Lys Pro Leu Ser Asn Gln Pro Ile Val Thr
            500                 505                 510

Ile Ser Ala Asp Asp Lys Asp Asp Thr Ala Asn Gly Pro Arg Phe Ile
        515                 520                 525

Phe Ser Leu Pro Pro Glu Ile Ile His Asn Pro Asn Phe Thr Val Arg
    530                 535                 540

Asp Asn Arg Asp Asn Thr Ala Gly Val Tyr Ala Arg Arg Gly Gly Phe
545                 550                 555                 560

Ser Arg Gln Lys Gln Asp Leu Tyr Leu Leu Pro Ile Val Ile Ser Asp
                565                 570                 575

Gly Gly Ile Pro Pro Met Ser Ser Thr Asn Thr Leu Thr Ile Lys Val
            580                 585                 590

Cys Gly Cys Asp Val Asn Gly Ala Leu Leu Ser Cys Asn Ala Glu Ala
        595                 600                 605

Tyr Ile Leu Asn Ala Gly Leu Ser Thr Gly Ala Leu Ile Ala Ile Leu
    610                 615                 620

Ala Cys Ile Val Ile Leu Leu Val Ile Val Val Leu Phe Val Thr Leu
625                 630                 635                 640

Arg Arg Gln Lys Lys Glu Pro Leu Ile Val Phe Glu Glu Glu Asp Val
                645                 650                 655

Arg Glu Asn Ile Ile Thr Tyr Asp Asp Glu Gly Gly Gly Glu Glu Asp
            660                 665                 670

Thr Glu Ala Phe Asp Ile Ala Thr Leu Gln Asn Pro Asp Gly Ile Asn
        675                 680                 685

Gly Phe Ile Pro Arg Lys Asp Ile Lys Pro Glu Tyr Gln Tyr Met Pro
    690                 695                 700

Arg Pro Gly Leu Arg Pro Ala Pro Asn Ser Val Asp Val Asp Asp Phe
705                 710                 715                 720

Ile Asn Thr Arg Ile Gln Glu Ala Asp Asn Asp Pro Thr Ala Pro Pro
                725                 730                 735

Tyr Asp Ser Ile Gln Ile Tyr Gly Tyr Glu Gly Arg Gly Ser Val Ala
            740                 745                 750

Gly Ser Leu Ser Ser Leu Glu Ser Ala Thr Thr Asp Ser Asp Leu Asp
        755                 760                 765

Tyr Asp Tyr Leu Gln Asn Trp Gly Pro Arg Phe Lys Lys Leu Ala Asp
    770                 775                 780

Leu Tyr Gly Ser Lys Asp Thr Phe Asp Asp Asp Ser
785                 790                 795

<210>  19
<211>  2598
<212>  DNA
<213>  Homo sapiens

<400>  19

atggccctcg tactcggctc cctgttgctg ctggggctgt gcgggaactc cttttcagga 60

gggcagcctt catccacaga tgctcctaag gcttggaatt atgaattgcc tgcaacaaat 120

tatgagaccc aagactccca taaagctgga cccattggca ttctctttga actagtgcat 180

atctttctct atgtggtaca gccgcgtgat ttcccagaag atactttgag aaaattctta 240

cagaaggcat atgaatccaa aattgattat gacaagccag aaactgtaat cttaggtcta 300

aagattgtct actatgaagc agggattatt ctatgctgtg tcctggggct gctgtttatt 360

attctgatgc ctctggtggg gtatttcttt tgtatgtgtc gttgctgtaa caaatgtggt 420

ggagaaatgc accagcgaca gaaggaaaat gggcccttcc tgaggaaatg ctttgcaatc 480

tccctgttgg tgatttgtat aataataagc attggcatct tctatggttt tgtggcaaat 540

caccaggtaa gaacccggat caaaaggagt cggaaactgg cagatagcaa tttcaaggac 600

ttgcgaactc tcttgaatga aactccagag caaatcaaat atatattggc ccagtacaac 660

actaccaagg acaaggcgtt cacagatctg aacagtatca attcagtgct aggaggcgga 720

attcttgacc gactgagacc caacatcatc cctgttcttg atgagattaa gtccatggca 780

acagcgatca aggagaccaa agaggcgttg gagaacatga acagcacctt gaagagcttg 840

caccaacaaa gtacacagct tagcagcagt ctgaccagcg tgaaaactag cctgcggtca 900

tctctcaatg accctctgtg cttggtgcat ccatcaagtg aaacctgcaa cagcatcaga 960

ttgtctctaa gccagctgaa tagcaaccct gaactgaggc agcttccacc cgtggatgca 1020

gaacttgaca acgttaataa cgttcttagg acagatttgg atggcctggt ccaacagggc 1080

tatcaatccc ttaatgatat acctgacaga gtacaacgcc aaaccacgac tgtcgtagca 1140

ggtatcaaaa gggtcttgaa ttccattggt tcagatatcg acaatgtaac tcagcgtctt 1200

cctattcagg atatactctc agcattctct gtttatgtta ataacactga aagttacatc 1260

cacagaaatt tacctacatt ggaagagtat gattcatact ggtggctggg tggcctggtc 1320

atctgctctc tgctgaccct catcgtgatt ttttactacc tgggcttact gtgtggcgtg 1380

tgcggctatg acaggcatgc caccccgacc acccgaggct gtgtctccaa caccggaggc 1440

gtcttcctca tggttggagt tggattaagt ttcctctttt gctggatatt gatgatcatt 1500

gtggttctta cctttgtctt tggtgcaaat gtggaaaaac tgatctgtga accttacacg 1560

agcaaggaat tattccgggt tttggataca ccctacttac taaatgaaga ctgggaatac 1620

tatctctctg ggaagctatt taataaatca aaaatgaagc tcacttttga acaagtttac 1680

agtgactgca aaaaaaatag aggcacttac ggcactcttc acctgcagaa cagcttcaat 1740

atcagtgaac atctcaacat taatgagcat actggaagca taagcagtga attggaaagt 1800

ctgaaggtaa atcttaatat ctttctgttg ggtgcagcag gaagaaaaaa ccttcaggat 1860

tttgctgctt gtggaataga cagaatgaat tatgacagct acttggctca gactggtaaa 1920

tcccccgcag gagtgaatct tttatcattt gcatatgatc tagaagcaaa agcaaacagt 1980

ttgcccccag gaaatttgag gaactccctg aaaagagatg cacaaactat taaaacaatt 2040

caccagcaac gagtccttcc tatagaacaa tcactgagca ctctatacca aagcgtcaag 2100

atacttcaac gcacagggaa tggattgttg gagagagtaa ctaggattct agcttctctg 2160

gattttgctc agaacttcat cacaaacaat acttcctctg ttattattga ggaaactaag 2220

aagtatggga gaacaataat aggatatttt gaacattatc tgcagtggat cgagttctct 2280

atcagtgaga aagtggcatc gtgcaaacct gtggccaccg ctctagatac tgctgttgat 2340

gtctttctgt gtagctacat tatcgacccc ttgaatttgt tttggtttgg cataggaaaa 2400

gctactgtat ttttacttcc ggctctaatt tttgcggtaa aactggctaa gtactatcgt 2460

cgaatggatt cggaggacgt gtacgatgat gttgaaacta tacccatgaa aaatatggaa 2520

aatggtaata atggttatca taaagatcat gtatatggta ttcacaatcc tgttatgaca 2580

agcccatcac aacattga 2598

<210> 20
<211> 865
<212> PRT
<213> Homo sapiens

<400> 20

<210>  21
<211>  2466
<212>  DNA
<213>  Homo sapiens

<400>  21
atggtcagct ggggtcgttt catctgcctg gtcgtggtca ccatggcaac cttgtccctg      60

gcccggccct ccttcagttt agttgaggat accacattag agccagaaga gccaccaacc     120

aaataccaaa tctctcaacc agaagtgtac gtggctgcgc caggggagtc gctagaggtg     180

cgctgcctgt tgaaagatgc cgccgtgatc agttggacta aggatggggt gcacttgggg     240

cccaacaata ggacagtgct tattggggag tacttgcaga taaagggcgc cacgcctaga     300

gactccggcc tctatgcttg tactgccagt aggactgtag acagtgaaac ttggtacttc     360

atggtgaatg tcacagatgc catctcatcc ggagatgatg aggatgacac cgatggtgcg     420

gaagattttg tcagtgagaa cagtaacaac aagagagcac catactggac caacacagaa     480

aagatggaaa agcggctcca tgctgtgcct gcggccaaca ctgtcaagtt tcgctgccca     540

gccgggggga acccaatgcc aaccatgcgg tggctgaaaa acgggaagga gtttaagcag     600

gagcatcgca ttggaggcta caaggtacga aaccagcact ggagcctcat tatggaaagt     660

gtggtcccat ctgacaaggg aaattatacc tgtgtagtgg agaatgaata cgggtccatc     720

aatcacacgt accacctgga tgttgtggag cgatcgcctc accggcccat cctccaagcc     780

ggactgccgg caaatgcctc cacagtggtc ggaggagacg tagagtttgt ctgcaaggtt     840

tacagtgatg cccagcccca catccagtgg atcaagcacg tggaaaagaa cggcagtaaa     900

tacgggcccg acgggctgcc ctacctcaag gttctcaagg ccgccggtgt taacaccacg     960

gacaaagaga ttgaggttct ctatattcgg aatgtaactt ttgaggacgc tggggaatat    1020

acgtgcttgg cgggtaattc tattgggata tcctttcact ctgcatggtt gacagttctg    1080

ccagcgcctg gaagagaaaa ggagattaca gcttccccag actacctgga gatagccatt    1140

tactgcatag gggtcttctt aatcgcctgt atggtggtaa cagtcatcct gtgccgaatg    1200

aagaacacga ccaagaagcc agacttcagc agccagccgg ctgtgcacaa gctgaccaaa    1260

cgtatccccc tgcggagaca ggtaacagtt tcggctgagt ccagctcctc catgaactcc    1320

aacaccccgc tggtgaggat aacaacacgc ctctcttcaa cggcagacac ccccatgctg    1380

gcaggggtct ccgagtatga acttccagag gacccaaaat gggagtttcc aagagataag    1440

ctgacactgg gcaagcccct gggagaaggt tgctttgggc aagtggtcat ggcggaagca    1500

gtgggaattg acaaagacaa gcccaaggag gcggtcaccg tggccgtgaa gatgttgaaa    1560

gatgatgcca cagagaaaga cctttctgat ctggtgtcag agatggagat gatgaagatg    1620

attgggaaac acaagaatat cataaatctt cttggagcct gcacacagga tgggcctctc    1680

tatgtcatag ttgagtatgc ctctaaaggc aacctccgag aatacctccg agcccggagg 1740

ccacccggga tggagtactc ctatgacatt aaccgtgttc ctgaggagca gatgaccttc 1800

aaggacttgg tgtcatgcac ctaccagctg gccagaggca tggagtactt ggcttcccaa 1860

aaatgtattc atcgagattt agcagccaga aatgttttgg taacagaaaa caatgtgatg 1920

aaaatagcag actttggact cgccagagat atcaacaata tagactatta caaaaagacc 1980

accaatgggc ggcttccagt caagtggatg gctccagaag ccctgtttga tagagtatac 2040

actcatcaga gtgatgtctg gtccttcggg gtgttaatgt gggagatctt cactttaggg 2100

ggctcgccct acccagggat tcccgtggag gaacttttta agctgctgaa ggaaggacac 2160

agaatggata agccagccaa ctgcaccaac gaactgtaca tgatgatgag ggactgttgg 2220

catgcagtgc cctcccagag accaacgttc aagcagttgg tagaagactt ggatcgaatt 2280

ctcactctca caaccaatga ggaatacttg gacctcagcc aacctctcga acagtattca 2340

cctagttacc ctgacacaag aagttcttgt tcttcaggag atgattctgt tttttctcca 2400

gaccccatgc cttacgaacc atgccttcct cagtatccac acataaacgg cagtgttaaa 2460

acatga 2466

<210> 22
<211> 821
<212> PRT
<213> Homo sapiens

<400> 22

<210>  23
<211>  2649
<212>  DNA
<213>  Homo sapiens

<400>  23
atgggccctt ggagccgcag cctctcggcg ctgctgctgc tgctgcaggt ctcctcttgg        60

ctctgccagg agccggagcc ctgccaccct ggctttgacg ccgagagcta cacgttcacg 120

gtgccccggc gccacctgga gagaggccgc gtcctgggca gagtgaattt tgaagattgc 180

accggtcgac aaaggacagc ctatttttcc ctcgacaccc gattcaaagt gggcacagat 240

ggtgtgatta cagtcaaaag gcctctacgg tttcataacc cacagatcca tttcttggtc 300

tacgcctggg actccaccta cagaaagttt tccaccaaag tcacgctgaa tacagtgggg 360

caccaccacc gcccccgccc ccatcaggcc tccgtttctg gaatccaagc agaattgctc 420

acatttccca actcctctcc tggcctcaga agacagaaga gagactgggt tattcctccc 480

atcagctgcc cagaaaatga aaaaggccca tttcctaaaa acctggttca gatcaaatcc 540

aacaaagaca aagaaggcaa ggttttctac agcatcactg gccaaggagc tgacacaccc 600

cctgttggtg tctttattat tgaaagagaa acaggatggc tgaaggtgac agagcctctg 660

gatagagaac gcattgccac atacactctc ttctctcacg ctgtgtcatc caacgggaat 720

gcagttgagg atccaatgga gattttgatc acggtaaccg atcagaatga caacaagccc 780

gaattcaccc aggaggtctt taaggggtct gtcatggaag gtgctcttcc aggaacctct 840

gtgatggagg tcacagccac agacgcggac gatgatgtga acacctacaa tgccgccatc 900

gcttacacca tcctcagcca agatcctgag ctccctgaca aaaatatgtt caccattaac 960

aggaacacag gagtcatcag tgtggtcacc actgggctgg accgagagag tttccctacg 1020

tataccctgg tggttcaagc tgctgacctt caaggtgagg ggttaagcac aacagcaaca 1080

gctgtgatca cagtcactga caccaacgat aatcctccga tcttcaatcc caccacgtac 1140

aagggtcagg tgcctgagaa cgaggctaac gtcgtaatca ccacactgaa agtgactgat 1200

gctgatgccc ccaatacccc agcgtgggag gctgtataca ccatattgaa tgatgatggt 1260

ggacaatttg tcgtcaccac aaatccagtg aacaacgatg gcattttgaa aacagcaaag 1320

ggcttggatt ttgaggccaa gcagcagtac attctacacg tagcagtgac gaatgtggta 1380

ccttttgagg tctctctcac cacctccaca gccaccgtca ccgtggatgt gctggatgtg 1440

aatgaagccc ccatctttgt gcctcctgaa aagagagtgg aagtgtccga ggactttggc 1500

gtgggccagg aaatcacatc ctacactgcc caggagccag acacatttat ggaacagaaa 1560

ataacatatc ggatttggag agacactgcc aactggctgg agattaatcc ggacactggt 1620

gccatttcca ctcgggctga gctggacagg gaggattttg agcacgtgaa gaacagcacg 1680

tacacagccc taatcatagc tacagacaat ggttctccag ttgctactgg aacagggaca 1740

cttctgctga tcctgtctga tgtgaatgac aacgccccca taccagaacc tcgaactata 1800

ttcttctgtg agaggaatcc aaagcctcag gtcataaaca tcattgatgc agaccttcct 1860

cccaatacat ctcccttcac agcagaacta acacacgggg cgagtgccaa ctggaccatt 1920

cagtacaacg acccaaccca agaatctatc attttgaagc caaagatggc cttagaggtg 1980

ggtgactaca aaatcaatct caagctcatg gataaccaga ataaagacca agtgaccacc 2040

ttagaggtca gcgtgtgtga ctgtgaaggg gccgctggcg tctgtaggaa ggcacagcct 2100

gtcgaagcag gattgcaaat tcctgccatt ctggggattc ttggaggaat tcttgctttg 2160

ctaattctga ttctgctgct cttgctgttt cttcggagga gagcggtggt caaagagccc 2220

ttactgcccc cagaggatga cacccgggac aacgtttatt actatgatga agaaggaggc 2280

ggagaagagg accaggactt tgacttgagc cagctgcaca ggggcctgga cgctcggcct 2340

gaagtgactc gtaacgacgt tgcaccaacc ctcatgagtg tcccccggta tcttccccgc 2400

cctgccaatc ccgatgaaat tggaaatttt attgatgaaa atctgaaagc ggctgatact 2460

gaccccacag ccccgcctta tgattctctg ctcgtgtttg actatgaagg aagcggttcc 2520

gaagctgcta gtctgagctc cctgaactcc tcagagtcag acaaagacca ggactatgac 2580

tacttgaacg aatggggcaa tcgcttcaag aagctggctg acatgtacgg aggcggcgag 2640

gacgactag 2649

<210> 24
<211> 882
<212> PRT
<213> Homo sapiens

<400> 24